# EUROPEAN PATENT APPLICATION

(11) **EP 1 783 218 A1**
(43) Date of publication of application: **09.05.2007**
(21) Application number: 05755873.6
(22) Date of filing: 24.06.2005
(51) Int. Cl.: C12N 15/09, C07K 14/18, C07K 16/10, C12N 5/10, C12N 7/00, C12Q 1/68

(54) **HCV RNA HAVING NOVEL SEQUENCE**

(30) Priority: 25.06.2004 JP 2004188543; 28.06.2004 JP 2004190144; 24.09.2004 JP 2004277677
(71) Applicant: Advanced Life Science Institute, Inc., Wako-shi, Saitama 3510112 (JP)
(72) Inventor: YAGI, Shintaro ADVANCED LIFE SCIENCE INSTITUTE INC, Wako-shi, Saitama 3510112 (JP); YAMAGUCHI, Kenjiro, ADVANCED LIFE SCIENCE INST., Wako-shi, Saitama 3510112 (JP); ORI, Kenichi, ADVANCED LIFE SCIENCE INSTITUTE INC., Wako-shi, Saitama 3510112 (JP); TANAKA, Eiji, Nagano 3900312 (JP); KIYOSAWA, Kendo, Nagano 3900305 (JP); MAKI, Noboru, ADVANCED LIFE SCIENCE INSTITUTE, INC, Wako-shi, Saitama 3510112 (JP)
(74) Representative: Denison, Christopher Marcus
(86) International application number: PCT/JP2005/012162
(87) International publication number: WO 2006/001517

(57) **Abstract**

A truncated form hepatitis C virus gene wherein part of the gene region encoding from the core protein to the NS2 protein of hepatitis C virus has been deleted while retaining the translation frame. In particular, the gene according to claim 1 wherein said part of the gene region is present in a region encoding at least the E1 protein and the E2 protein.

## Description

### Technical Field

The present invention relates to the genome of type C chronic hepatitis virus (hereinafter referred to as "HCV").

### Background Art

HCV is a causative agent of chronic hepatitis C, and it is estimated according to WHO statistics that 170 million people are infected throughout the world. Though, unlike other viral hepatitis, HCV causes only a relatively mild symptom at the early stage of infection, the infection progresses to a chronic type at a high frequency, and after a certain asymptomatic period, develops chronic hepatitis. As the infection is prolonged, the condition becomes aggravated to cirrhosis, which leads to hepatic carcinoma at a high frequency. Hepatitis viruses are responsible for 95% of hepatic cancer, and most (80%) of it is believed to be caused by HCV.

HCV is transmitted through blood, blood components and, less frequently, body fluid components. Since testing methods for HCV have been introduced into screening at the time of blood transfusion, novel cases of posttransfusion type C hepatitis are very rare now in advanced countries. Furthermore, due to progress in medical technology, transmission due to malpractices is rare, and in Japan the appearance of new patients has been almost suppressed. Based on the epidemiological survey, however, the number of HCV carriers is estimated to be not less than 1.7 million in Japan, and most of them are 40 years of age or older and the infection tends to be prolonged. Thus, there is a grave concern for increases in the number of cases of hepatic carcinoma in the future.

The risk of hepatic carcinoma is highly associated with the state of fibrosing of the liver, and the more progressed the fibrosing is, the higher the incidence of hepatic carcinoma becomes. The state of fibrosing of the liver is usually diagnosed by combining the determination of blood concentrations of Type IV collagen and hyaluronic acid, platelet counts in the blood, diagnostic imaging (abdominal echo check) etc., and, for definite diagnosis, a liver biopsy is carried out. However, liver biopsy poses a major problem since it poses a great burden on the patients, and more patient-oriented diagnostic methods are being required.

HCV, comprising an about 9,600-base plus strand RNA genome, is a virus classified into the family Flaviviridae, the genus Flavivirus, and is thought to be transmitted through the blood and blood components and to propagate in the liver. The analysis of the gene sequence suggests the presence of at least six genotypes. After infection, the about 9,600-base genome functions as mRNA in the host cell, and a polyprotein of a stretch of about 3,000 amino acid length is synthesized, which is cleaved with a signal peptidase, signal peptidyl peptidase in the host cells, and a protease encoded by the HCV genome. As a result, 10 types of proteins such as core, E1, E2, p7, NS2, NS3, NS4A, NS4B, NS5A and NS5B are produced. In addition to this translation frame (open reading frame), there are untranslated regions (UTR) at the 5'-end and the 3'-end and these regions are responsible for the functions of translation control and genome replication control.

Among them, core, E1 and E2 are structural proteins that constitute the virus, and it is believed that the virus genome is packaged by the core protein to form a capsid, and surrounded by the E1 and E2 proteins anchored to the lipid bilayer membrane to form a virus particle (virion). The function of p7 is unknown, but is reported to be essential for the viral propagation. NS2 is a metal protease and is necessary for the cleavage of itself, but its other functions are unknown. It is believed that a complex comprising of the protein NS3 to NS5B forms a RNA replication unit together with host proteins to perform the replication of genomic RNA.

For the diagnosis of HCV infection, methods of detecting antibodies against proteins produced by HCV contained in the biological components (blood, serum, plasma etc.) of patients are widely used. However, the mere presence of antibody cannot identify whether HCV is active or not, and therefore the measurement of amount of virus in the blood is important in diagnosis. For the detection or measurement of virus in the blood, methods of detecting or determining the virus genome, or determining or detecting the core protein produced by HCV are used.

For the treatment of chronic hepatitis C, interferon has been widely used. Due to the recent improvement of the drugs and the administration regimen such as the combined therapy with ribavirin, the ratio of complete remission resulted from HCV eliminated from the body are increasing. However, the ratio of complete remission is still about 50%. Also interferon administration may cause severe side effects, and thus may not be used for elderly patients. Therefore, the development of more effective therapeutic methods or therapeutic agents is being required.

With regard to the relation of the interferon therapy and HCV, the tendency that the more the amount of the virus in the blood, the higher the resistance to the interferon therapy is, is recognized. There are also cases in which the difference of sensitivity to interferon according to genotypes is observed, and the genotype 2 in particular shows a relatively high sensitivity to interferon treatment. However, there are no definite relationship between the amount of virus in the blood and the degree of aggravation of hepatitis, or between the genotype and the grave condition of hepatitis. Thus, there are no virus markers that represent the grave condition of hepatic diseases.

HCV infects humans through the blood or blood components. In organisms other than humans, it infects anthropoids (chimpanzees), and the infection leads to the onset of hepatitis and sometimes to chronic hepatitis. In other experimental animals that are easy to keep, none are known to be infected with HCV at a high frequency.

On the other hand, it is reported that HCV could be infected to human- and monkey-derived cells in vitro and could be propagated. However, both of the infection ratio and the propagation efficiency were low. Thus, it is very difficult to infect and propagate HCV in vitro.

It was found in recent years that by transfecting the RNA containing a portion of synthesized HCV genome (subgenomic RNA) in vitro and a drug resistant marker into an established human hepatoma-derived cell, a cell in which the RNA is autonomously replicating in the cell can be isolated though at a low frequency. Since this has been reproduced in many laboratories and the cells can be maintained relatively easily, it came to be widely used in research. In these replicons the structural protein part of HCV has been artificially deleted, suggesting that the replication unit composed of proteins produced by the non-structural region has information necessary and adequate for the replication of the plus strand RNA of HCV in the cell.

The propagation of HCV in the liver has been indicated by the detection of proteins produced by HCV in the liver and the detection of HCV RNA in the liver. Also, by eliminating HCV transiently or permanently by the interferon therapy, the subsequent condition of hepatitis is alleviated, which clearly indicates that HCV is related with the development of pathology in the liver. However, much has yet to be elucidated on the mode of presence of HCV in the liver.

Thus, the mechanism of development and progress of pathology in which HCV infection leads to the onset of hepatitis, which turns into a prolonged chronic type and the disease condition aggravates, finally leading to hepatic carcinoma has not been elucidated. The extensive research proposes hypotheses on the mechanism of development and aggravation of pathological conditions by allowing each HCV protein to be recombinantly expressed in cultured cells and analyze the state of the expressing cells. Since there are no appropriate models of HCV infection and propagation, the hypotheses cannot be confirmed.

Patent document 1: Japanese Unexamined Patent Publication (Kokai) No. 2001-17187

Non-patent document 1: Science, Vol. 277, pp. 570-, 1997

Non-patent document 2: Journal of Virology, Vol. 76, pp. 4008-4021, 2002

Non-patent document 3: Science, Vol. 285, pp. 110-, 1999

Non-patent document 4: Science, Vol. 290, pp. 1972-, 2000

Non-patent document 5: Hepatology, Vol. 29, pp. 223-229, 1999

Non-patent document 6: Journal of Virology, Vol. 77, pp. 2134-2146, 2003

Non-patent document 7: Res. Virol., Vol. 144, pp. 275-279, 1993

Non-patent document 8: Journal of Virology, Vol. 75, pp. 4614-4624, 2001

Non-patent document 9: PNAS, Vol. 29, 14416-14421, 2002

Non-patent document 10: Current Opinion in Infectious Disease, Vol. 14, pp. 743-747

Non-patent document 11: Journal of Viral Hepatitis, Vol. 6, pp. 35-47, 1999

Non-patent document 12: Clinical Chemistry, Vol. 43, pp. 1507-1522, 1997

Non-patent document 13: Journal of General Virology, Vol. 81, pp. 1631-1648, 2000

### Disclosure of the Invention

Though HCV is thought to replicate itself and propagate in the liver, its mode of presence in the liver has not been analyzed. Accordingly there is very little information on how HCV is replicated in the liver and what molecules are appropriate as the target of therapeutic agents for HCV. Consequently, the therapy of HCV is carried out on a trial and error basis. Therefore, in order to develop the therapeutic agents efficiently, it is necessary to identify HCV genomic RNA that is being actively replicated in the liver of the patient. Thus, the present invention relates to HCV genomic RNA that is being replicated and propagating in the liver, and intends to provide HCV genomic RNA that serves as an appropriate target for the therapeutic agents.

Though the propagation of HCV aggravates the condition of hepatitis, there are no virus markers that indicate the condition of hepatitis. Virus markers that indicate the condition of hepatitis are being required. The present invention intends to provide a virus marker that indicates the symptom and condition of hepatitis.

Since there are no experimental animals that are easy to handle and that are able to be infected with HCV or no in vitro culture systems in which is infected with HCV at a high frequency and replicates HCV efficiently, it is difficult to carry out efficient drug screening. These represent the reasons that make the development of HCV-specific therapeutic agents difficult. For example, for the widely used interferon therapy, methods of treatment have been directly developed and improved with patients as the subject, and have posed a great burden on the patients. Thus, more patient-oriented methods for developing pharmaceutical drugs are needed. In order to resolve this problem, models of HCV infection and propagation that can be widely used are required in the development of effective therapeutic agents and therapeutic methods. One of the objectives of the present invention is to provide a model of HCV infection and propagation that can be used in the development and screening etc. of therapeutic agents and therapeutic methods.

As such a propagation model, a system that employs a subgenomic replicon has been used. Thus, by transfecting RNA having an appropriate structure encoding a HCV subgenome and an appropriate drug resistant marker into a human hepatoma-derived cell and selecting the cell retaining the RNA by drug resistance, a HCV subgenomic RNA replicon that autonomously propagates in the cell can be obtained. Using this replicon and the cell retaining the replicon, the screening of drugs for HCV is under way. However, this method has serious problems.

One of them is that the genome of subgenomic RNA replicon is composed of a structure different from the original HCV genomic RNA. Thus, the subgenomic RNA has only information derived from the nonstructural protein of the HCV genome. In order to improve this, a replicon comprising all the translated region of the HCV protein and a drug resistant marker was established. In cells that retain this replicon, all proteins encoded by the HCV genome should be produced, and thus it was expected that HCV particles should be released outside the cell. However, no HCV particles were released outside the cell. Thus, even with such an improvement, it appears to be incomplete as a propagation system of HCV.

Also, the above subgenomic replicon comprising the nonstructural region has the internal ribosome entry site (IRES) of the 5'-untranslated region and the neomycin resistant gene downstream of the gene of portion of the core, and, downstream thereto, the internal ribosome entry site (IRES) of the encephalomyocarditis virus (EMCV), a region encoding of nonstructural protein of HCV and the 3'-untranslated region. Also, the improved replicon having all of the translated region is one in which the gene region encoding the nonstructural protein of the subgenomic replicon has been replaced with a region encoding all the translated region of HCV, and the fundamental structure of these replicons is essentially the same.

Unlike the structure of the original HCV, these replicons have two IRES's (HCV IRES and EMCV IRES), and are different from the structure of HCV genome in the living body. This difference in structure is possibly one of reasons that HCV particles can not be released outside the cell. Furthermore, replicons having such a structure may be replicated in a mechanism different from the virus genome replication in the living body.

From the foregoing, it is desirable that the replicon system of HCV is a replicon system that employs RNA having the original HCV genome structure in the living body. The present invention intends to provide a replicon comprising a sequence and a structure that is actually replicated in the liver.

A further serious problem is the so-called adaptive mutation, i.e. the presence of mutation which is peculiar to the experimental system of the subgenomic replicon. When a HCV replicon which is functional in vitro was collected and analyzed, a plurality of mutations were found that were not originally present in NS3 or NS5A. This mutation is important for the effective replication and influences the efficiency of replication. However, it is known that mutation suitable for replication is undesirable for the propagation of HCV. By inoculating an in vitro-synthesized HCV genome directly into the liver of a chimpanzee, the chimpanzee is infected with HCV.

When a mutation which is important for the subgenomic replicon is introduced into the HCV RNA synthesized in vitro, the infectivity to chimpanzees retained by the original sequence disappeared. Furthermore, there are no sequences that have infectivity to chimpanzees and that are replicated and propagated in vitro in the cell. Therefore, sequences that came to be replicated in vitro have lost the function of replicating and propagating in vivo, and thus do not retain the ability of replicating RNA and propagating owned by the original HCV. They must be sequences capable of replicating and propagating both in vivo and in vitro. Thus, the replicon provided by the present invention having the HCV genomic RNA sequence can replicate both in vivo and in vitro.

The present inventors have isolated cDNA to the HCV genomic RNA in the liver of a patient and have determined the entire structure. By analyzing the sequence determined, the isolated cDNA was found to have a sequence entirely different from the structure of the HCV genomic RNA so far reported.

The genomic RNA having a new structure is characterized in that part or all of the structural protein regions of the HCV genomic RNA previously reported are deleted, and it was found that the flanking sequences of the deleted portion are connected while retaining the translation frame (reading frame) of the original HCV genome, and that this HCV genome encodes one stretch of a novel polypeptide. The one stretch of polypeptide can generates part of the structural protein and all of the nonstructural proteins of the original HCV.

Since the HCV polyprotein encoded by this genome retains a cleavage site by the intracellular signal peptidase and a cleavage sequence by its own protease, it is estimated to undergo processing. In fact, when the novel HCV genomic cDNA set forth in SEQ ID NO: 1 was expressed in a mammalian cell and the product was analyzed, the core protein was normally processed, the E1 and NS2 proteins were expressed as a fusion protein and processed with NS3, and NS3 was processed to a molecule of the original size.

This HCV genomic RNA is called a truncated form (TF). In contrast, the previously reported HCV RNA containing all of the structural genes is called a full-length form (FLF). Biopsy samples and serums from a plurality of patients with chronic hepatitis C were analyzed. From a plurality of patients, a TF HCV genome having the common characteristics was detected. The characteristic is that part or all of sequence of the structural protein has been deleted, but the latter half of the NS2 sequence and after are retained, and that the remaining sequence may be expressed in such a form as to retain the translation frame that is inferred from FLF.

Thus, the present invention provides:
(1) A truncated form hepatitis C virus gene wherein part of the structural protein-coding region has been retained and, while retaining one translation frame, part of a region encoding from the core protein to the NS2 protein has been deleted in the hepatitis C virus gene;
(2) The truncated form hepatitis C virus gene according to (1) wherein part of the structural protein-coding region has been retained and, while retaining one translation frame, at least a region encoding the amino acid sequence at the junction the E1 protein and the E2 protein has been deleted in the hepatitis C virus gene;
(3) A truncated form hepatitis C virus gene wherein part of the E1 protein-coding region, the E2 protein-coding region, the P7 protein-coding region and part of the NS2 protein-coding region have been deleted while retaining the translation frame in the hepatitis C virus gene;
(4) The gene according to any of the above (1) to (3), said gene having 5'UTR and 3'UTR; and
(5) The gene according to any one of the above (1) to (3), said gene having 5'UTR, a protein-coding region downstream from NS3, and 3'UTR.

Though hepatitis C virus genome is RNA, the hepatitis C virus gene of the present invention may be used whether it is RNA or DNA.

In other words, the present invention provides (a) a truncated form hepatitis C virus gene wherein part of the E1 protein-coding region, the E2 protein-coding region, the P7 protein-coding region and part of the NS2 protein-coding region have been deleted while retaining the translation frame in the hepatitis C virus gene.

The present invention also provides the truncated form hepatitis C virus gene according to the above (a), said gene having all or part of a region encoding from the 5'-untranslated region to the core protein which is a structural protein and all or part of a region encoding from a region encoding two transmembrane domains at the latter part of NS2 which is a nonstructural protein to the 3'-untranslated region.

Furthermore, the present invention provides the truncated form hepatitis C virus gene according to the above (a), said gene having all or part of No. 1 to No. 914 and all or part of No. 3001 and after of the nucleic acid sequence of the hepatitis C virus gene.

On the other hand, the analysis of the subgenomic replicon demonstrated that information necessary for RNA replication is encoded in the region of the nonstructural protein at NS3 and after. Thus, the TF genome has all the information of the nonstructural protein required for RNA replication. Furthermore, since the TF genome is preferentially detected in some liver biopsy samples, it can be seen that the TF genome is autonomously replicating in the liver. In addition, when TF-containing RNA is transfected in vitro into the cell, it is replicated in the cell. Thus, the TF HCV genomic RNA functions as a replicon. These results revealed that the HCV genomic RNA provided by the present invention can be replicated both in the liver and in vitro.

Thus, the present invention also provides:
(6) A replicon gene of a truncated form hepatitis C virus gene wherein part of region of the gene encoding from the core protein to the NS2 protein of the hepatitis C virus that autonomously replicates in the cell has been deleted while retaining the translation frame, or a truncated form hepatitis C virus gene wherein the deleted region is present at a region encoding at least the E1 protein and the E2 protein.

The present invention also provides:
(7) A replicon gene in which a selection marker gene has been connected to the replicon gene according to the above (3).

Alternatively, it provides:
(8) a cell in which the above replicon gene is replicated.

TF propagates more advantageously than FLF in the liver, and the patients exhibit severe symptoms of hepatitis. The appearance of TF is deeply associated with symptoms of hepatitis, and thus the suppression, inhibition and elimination of the propagation of TF provide an effective therapeutic method. Thus, drugs that effectively suppress or alleviate the symptoms of chronic hepatitis C are preferably developed with TF as the target. Thus, it is obvious that the screening of pharmaceuticals with a replicon using TF is suitable for the development of pharmaceuticals for suppressing, alleviating or eliminating the progress of hepatitis to severe forms.

Thus, the present invention further provides:
(9) A method of screening drugs using a cell in which a truncated form gene is replicated, a method of evaluating the efficacy of drugs, and a method of producing drugs by evaluating the efficacy;
(10) A cell in which a vector having integrated a truncated form gene therein is retained and in which the protein is being expressed; and
(11) A method of diagnosing HCV using a cell in which a replicon containing a truncated form gene is being replicated or a protein produced by the cell.

The TF genome can also be detected in the blood. Thus, this HCV genomic RNA is replicated in the liver and exits as VLP in the blood. Therefore, for the detection of the TF genome, blood or blood components can be used as the sample.

In patients in whom TF is detected, there can be observed cases in which TF is the dominant form among the liver RNAs. This indicates that TF is more suitable for RNA replication in the liver than FLF. Analysis of liver biopsy and serum from many patients revealed that patients in which TF is the dominant form develop severe hepatitis in many cases, and prognosis of the interferon therapy is bad in many of them. This indicates that when TF is the dominant form in the liver, hepatitis is at a severe stage and the effect of the interferon therapy is limited. Thus, the detection and determination of TF provides an index for the state of hepatitis. This means that a method of detecting and determining TF can be applied as a method for diagnosing the state of hepatitis and can be used as a novel virus marker.

Thus, the present invention also provides:
(12) A method of detecting a truncated form gene in the sample. This method also includes methods of detecting the truncated form gene of hepatitis C virus using all methods for detecting the deletion of genes.

As another example, the present invention provides a method of detecting or determining the truncated form gene by amplifying the truncated form gene by PCR with primers designed from sequences from nucleic acids No. 1 to No. 914 and No. 3001 and after of the hepatitis C virus gene.
(13) It also provides a method of determining the quantitative ratio of the truncated form hepatitis C virus gene and the full-length form hepatitis C virus gene in a sample in which they are mixed.
(14) It also provides a method of determining the quantitative ratio by quantitating a gene at the common region of the truncated form gene and the full-length form gene, and quantitating a gene at the region where is deleted in the truncated form gene.

The TF genome in the blood is thought to exist as hepatitis C virus particles or hepatitis C virus-like particles. This is because, unless a certain structure is formed, the TF genome which is RNA is rapidly digested by RNase in the blood and cannot be detected.

Thus the present invention provides:
(15) Hepatitis C virus particles or hepatitis C virus-like particles retaining the truncated form gene.

In the TF genome, a region encoding the structural protein is deleted. Therefore, a novel HCV polyprotein in which a peptide that was encoded in the deleted region is deleted can be produced. As described above, when the HCV genomic cDNA set forth in SEQ ID NO: 1 was expressed in a mammalian cell, a fusion protein of E1 and NS2 was produced.

Thus, the present invention also provides:
(16) A polyprotein of the hepatitis C virus produced from the truncated form gene and a protein processed from the polyprotein. It also provides:
(17) An antibody that specifically recognizes this protein.

By using a novel HCV genomic RNA (TF), a RNA replicon replicating in vivo can be replicated in vitro. By using this replicon, a model of infected cells which are preferentially replicated in the patient's liver and which aggravate hepatitis into a severe form can be constructed. By using this model, the development and screening of pharmaceuticals that suppress and/or inhibit the progress of hepatitis into a severe form can be carried out.

The detection or quantitation of TF is effective for grasping the state of hepatitis, and is useful as a virus marker that indicates the state of hepatitis. Also by a diagnostic method using this marker, the state of hepatitis in patients, monitoring of the effect of drugs, and resistance to drugs can be diagnosed.

### Brief Explanation of the Drawings

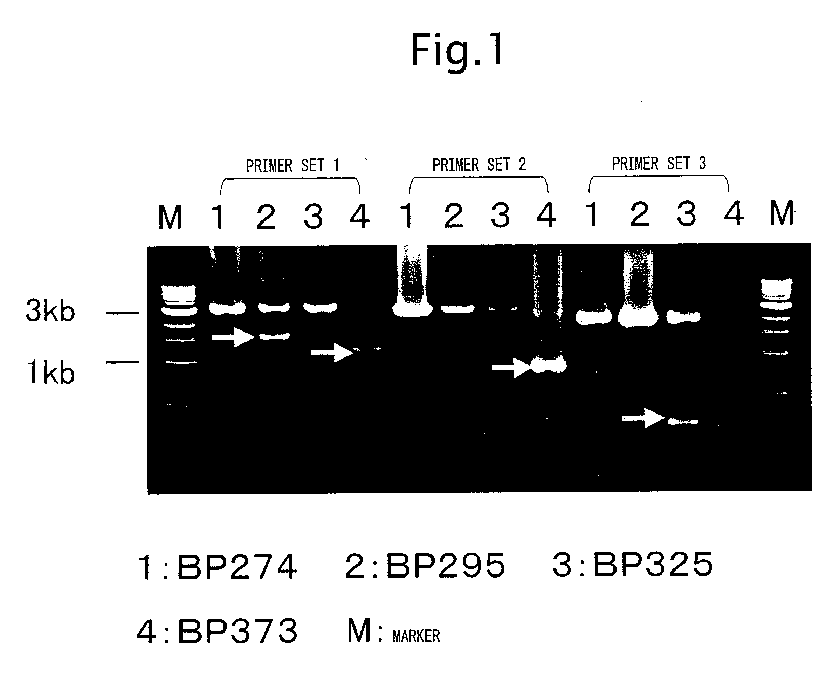

Fig. 1 shows a representative image of electrophoresis of a TF genome detected by RT-PCR from the HCV genomic RNA of the liver tissue. In primer set 1, the cDNA synthetic primer is 3481R, the 1st PCR primer is HClongAl and 3481R, and the 2nd PCR primer is 85F and 3279R; in primer set 2, the cDNA synthetic primer is 3945R, the 1st PCR primer is 831S and 3945R, and the 2nd PCR primer is 841S and 3759R; in primer set 3, the cDNA synthetic primer is 3945R, the 1st PCR primer is 813S and 3174AS, and the 2nd PCR primer is 841S and 3111AS. The arrow indicates the PCR product of the TF genome.

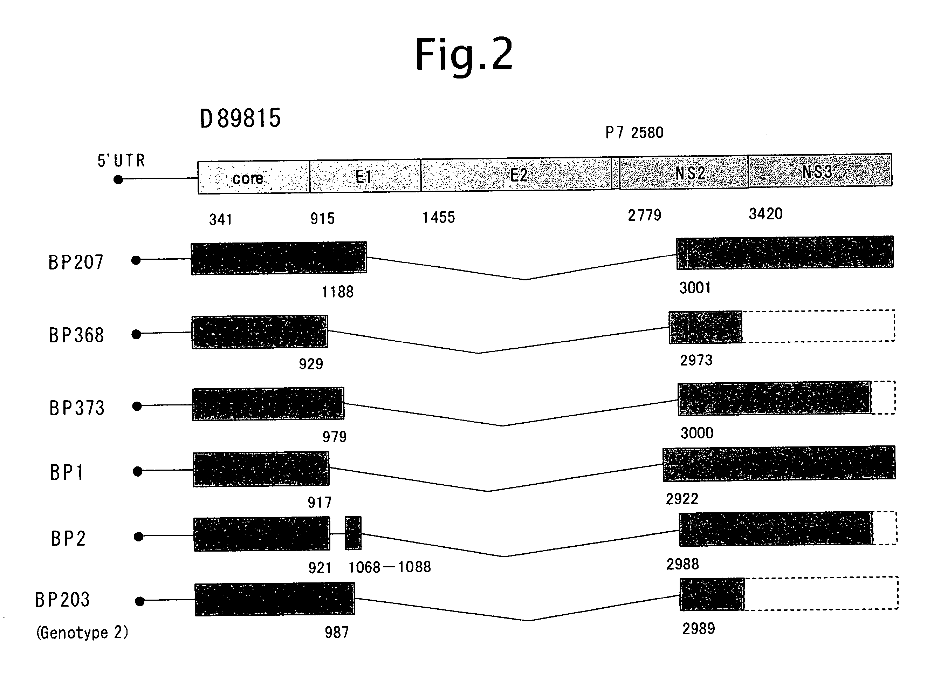

Fig. 2 shows difference in the structure of the TF genome in a sample in which the TF genome was only detected and D89815 genome. In a nucleic acid sequence encoding each protein of D89815, the core is present at 341-914, E1 at 915-1454, E2 at 1455-2579, P7 at 2580-2778, and NS2 at 2779-3419. For the TF genome obtained from each sample, 5'UTR is represented by a solid line and the protein-coding region is represented by a gray bar. The deleted region is represented by connecting the bars by a line. The number under the bar of each sample represents the position of a nucleic acid corresponding to D89815.

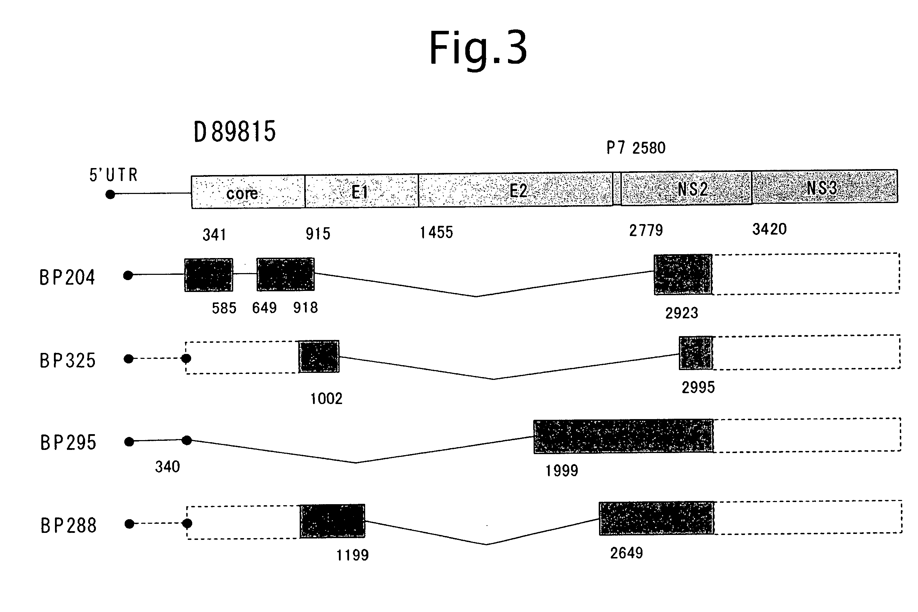

Fig. 3 shows difference in the structure of the TF genome in a sample in which both the TF genome and the FLF genome were detected and D89815 genome. As in Fig. 2, for the TF genome obtained from each sample, 5'UTR is represented by a solid line and the protein-coding region is represented by a gray bar. The deleted region is represented by connecting the bars by a line. The number under the bar of each sample represents the position of a nucleic acid sequence corresponding to D89815.

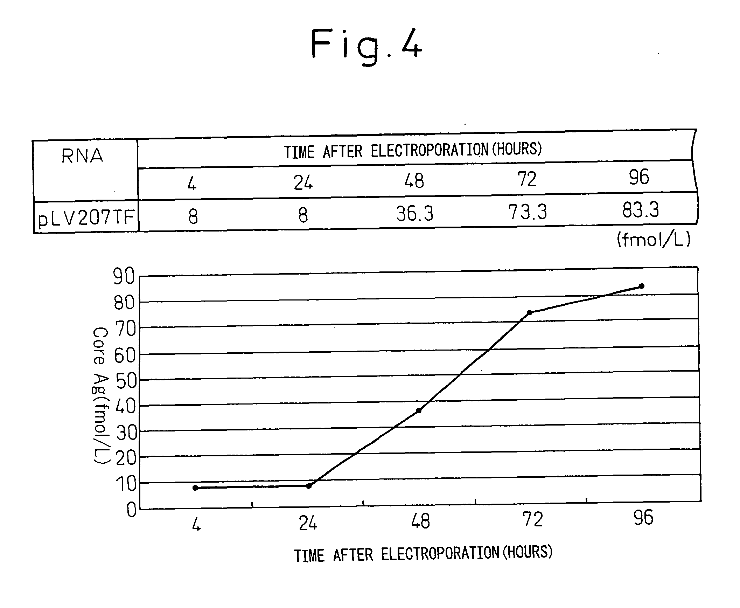

Fig. 4 is a graph showing time course in the amount of core antigen in Example 6.

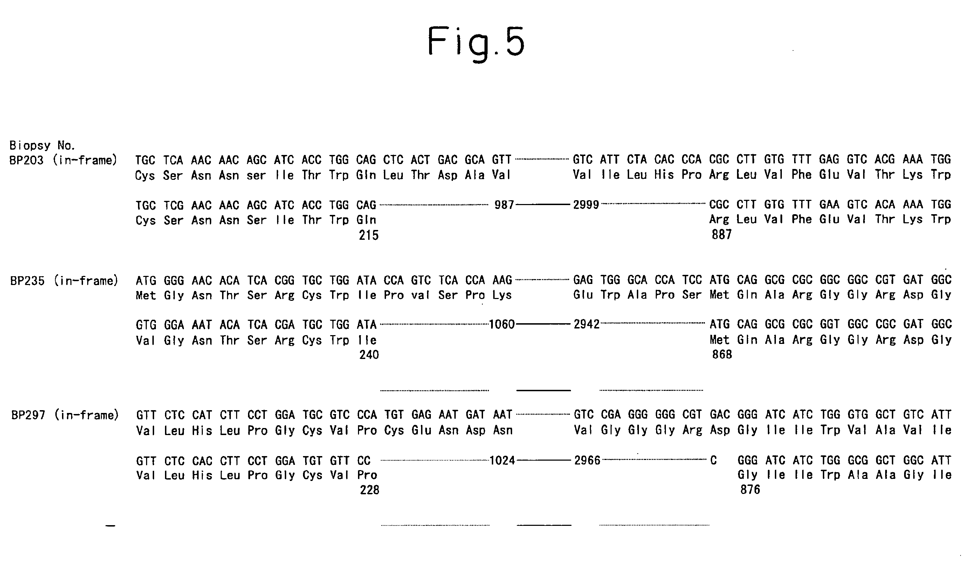

Fig. 5 shows flanking sequences of a gene having the deletion obtained in Example 7.

### Best Mode for Carrying out the Invention

The best mode of the present invention is described below, but it should be noted that the present invention is not limited to it in any way.

The present invention relates to the gene of a novel structure of hepatitis C virus. The normal FLF gene of hepatitis C virus comprises 5'UTR followed by regions encoding the structural proteins of the virus such as the core protein, the E1 protein, the E2 protein and the P7 protein, and the nonstructural proteins such as the NS2 protein, the NS3 protein, the NS4A protein, the NS4B protein, the NS5A protein and the NS5B protein, and 3'UTR.

The hepatitis C virus gene of the present invention is a TF genome in which genes encoding all or part of the structural proteins such as the core protein, the E1 protein, the E2 protein and the P7 protein, and/or part of the NS2 protein, a nonstructural protein, have been deleted. In this TF genome, there is in-frame deletion while retaining the translation frame of the FLF genome, and in the region other than the deleted regions, the normal HCV polyprotein can be produced. Thus, the present invention relates to a hepatitis C virus gene in which part of the region of the gene encoding the polyprotein of hepatitis C virus has been deleted in-frame, and is characterized by the structure of the TF genome.

The TF genome of HCV retains 5'UTR, and it is believed that in many cases at least the gene at No. 3001 and after of the FLF genome of HCV has been retained. In the gene at No. 3001 and after, two transmembrane domains at the C terminal of the NS2 region are present, and proteins of HCV at NS3 and after can be normally produced in the cell from this TF genome (a number indicating the position of the nucleic acid sequence describes the number of the position corresponding to the HCV sequence in GeneBank accession No. D89815 which is a full-length form sequence).

This TF genome is characterized in that a region encoding part or all of the core protein, the E1 protein and the E2 protein has been deleted. Specifically, as described in Fig. 2 and Fig. 3, it is characterized in that it does not retain the gene encoding the E1 and the E2 region in its complete form. In other words, it is a gene characterized in that the gene encoding part or all of the E1 protein and the E2 protein has been deleted. In the TF genome obtained at present, no TF genome that retains from position 1200 encoding E1 to position 1998 encoding E2 has been confirmed. The contiguous deletion of each TF gene is at least 63 bases, and at most 2043 bases. Also the total of deletions of one TF gene is 1449-2067 bases.

The most typical structure of the TF genome is a structure in which an about 2kb gene encoding the E1 protein to the NS2 protein has been deleted (Fig. 2).

Also desirable are a gene having the above deletion and retaining 5'UTR, a gene encoding proteins downstream from NS3 and a gene retaining 3'UTR.

The TF gene is characterized by the structure of its deletion region. Thus, all nucleic acids are included in the present invention as long as they are the gene sequence of HCV. In addition to the genotype 1, the present invention was confirmed to have the TF gene of the genotype 2, and the TF genes with a sequence of genotypes 3 to 6 are also included.

The TF genome is autonomously replicating in the liver of patients with chronic active hepatitis C. There are also samples in the tissue of the patient in which the TF genome alone is amplified by PCR. This suggests that the TF genome is being preferentially replicated in the liver tissue instead of the FLF genome.

Thus, this TF genome can be used as a replicable replicon in the liver cells or the liver-derived cells. As the TF per se has an autonomous replicating ability, the structure as it is functions as a replicon. Also, in order to select a replicon, a drug resistance gene can be connected thereto and be selected with the drug. The drug resistance gene, such as neomycin etc. can be used. The TF genome that can be used as a replicon contains all of the above TF genome in which the structural region is deleted. Also, in addition, any structure of a TF genome having in-frame deletion of the HCV gene replicating in the liver can be used.

The most preferred replicon of the present invention is a replicon that has the IRES of the 5'-untranslated region, the core region, part of the E1 region, and a region encoding downstream from part of NS2 to the nonstructural protein, and 3'UTR of HCV, and a replicon that has the same structure as the TF genome replicating in vivo. Furthermore, those in which part of the replicon such as one having integrated therein the neomycin-resistant gene in the core region can also be used as the replicon.

Furthermore, the present invention provides a cell in which this replicon is replicated. HCV RNA having the structure of the TF genome is actually being replicated in the liver cell, and the replication system of the TF genome replicon using this structure is thought to reflect a virus replication system in the liver. The cell in which the replicon is replicated may be a subcultured cell derived from the liver or a primary hepatic cell. Cells not derived from the liver can also be used as long as the replicon is replicated therein. Cells in which the replicon is replicated include cells in which replicon is permanently replicated. Also, cells in which replicon is transiently replicated are included.

The replication system of the TF genome replicon is useful in screening drugs against HCV infection. This is because the TF genome is actually being replicated in the liver tissue. Using the replicon replication system in which this replicon is actually replicating, drugs that suppress HCV propagation can be screened. Also included are methods of evaluating drug efficacy using a replicon-replicating cell. Since this screening system targets the replicon of the TF genome being actually replicated in the liver, screening of more effective drugs can be expected. It is also useful as a method for evaluating the effect of screened drugs. In this case, it is important to carry out the evaluation of drugs with this method. If it is essential for the control of drugs, it can also be used as a method for producing drugs.

Furthermore, the present invention provides a method of detecting the TF genome. The TF genome has a deleted region. Any method that detects a gene having this deletion can be used. For example, a primer is designed outside to the 3'-end of the deleted region, and cDNA is synthesized from RNA. Primers are designed outside to the 5' and 3' so as to sandwich the deleted region of the cDNA, and by performing PCR, genes having deletion shorter than the FLF genome can be detected. After performing PCR, it is also possible to detect shorter TF genomes by Northern blotting.

This method of detecting the TF genome includes all the methods of detecting gene deletions in addition to the above-mentioned methods.

Also, by subtracting the amount of the FLF genome from the amount of the full-length FLF genome and the TF genome, it is also possible to determine the amount of the TF genome.

Primers are designed at the common region in the FLF genome and the TF genome having no deletions, and the amount of both genes is determined by RT-PCR. Then, by designing a PCR primer in the deleted region of the TF genome and determining the amount of the gene, the amount of the FLF genome alone can be determined. By comparing the amount of both the FLF genome and the TF genome and the amount of the FLF genome alone, the amount of the TF genome can be determined.

A primers for determining the amount of the FLF genome and the TF genome may be designed at any region as long as the FLF genome and the TF genome are overlapping therein. For example, a primer can be designed at 5'UTR or a region encoding nonstructural proteins NS3 and after, or at 3'UTR. Also, a primer for determining the FLF genome alone can be designed at any deleted region in the TF genome, for example, a region encoding structural proteins such as the gene region of core, E1 and E2, or a region of P7 or NS2 in which the deletion is observed. As a typical TF genome has a deletion of a nucleic acid sequence No. 1189 to 2922, the FLF genome alone can be detected by designing a primer at this region. More preferably, as all TF genomes obtained in Examples of the present invention have a deletion of 1200 to 1998, the FLF genome alone can be detected by designing a primer at this region.

The quantitation of HCV RNA gene can be carried out by the RT-PCR method. For example, competitive RT-PCR, the realtime PCR method or the like can be used.

The present invention provides an invention that relates to a polyprotein produced by the TF genome. The TF genome has an in-frame deletion and encodes a polyprotein. This polyprotein is one that does not have a polypeptide encoded in the deleted region compared to the polyprotein encoded in FLF genome. Also, it is a novel protein different from the normal polyprotein produced from the FLF genome, in which a protein at the N terminal end upstream from the deleted region and a protein at the C terminal end downstream from the deleted region are fused.

The present invention also provides a protein that was processed from the above polyprotein. The above polyprotein is cleaved with a peptidase, and processing yields novel proteins such as a fusion protein of E1 and NS2, a fusion protein of E1 and E2 and a fusion protein of core and E2. The above polyprotein or processed protein is not produced from the FLF genome, and thus by detecting this protein, an effect similar to the detection of the TF genome can be obtained.

The present invention also provides a method of diagnosing the conditions of hepatitis by detecting or quantitating the TF genome or a polyprotein produced from the TF genome.

The present invention also provides an antibody specific to a polyprotein or a fusion protein produced from the TF genome. These antibodies are useful for detecting a polyprotein or a fusion protein.

The present invention also provides particles having a virus-like structure containing the TF genome. The TF genome is detected from the blood other than the liver cells. In the blood, the TF genome exists in association with the core protein. The virus particles or virus-like particles can also be used for diagnosis, treatment or the like.

### Examples

### Example 1. Isolation and analysis of a truncated genome sequence

A patient liver section BP207 (0.5 mm × 1 mm) was disrupted in 100 µl of the RIPA buffer (20 mM Tris-HC1 [pH 7.5], 150 mM NaCl, 1% NP40, 0.1% deoxycholate, complete protease inhibitor cocktail [Roche Diagnostics Corporation]), and after centrifuging at 10 krpm for 5 minutes, the supernatant was collected. From this extract, using the High Pure Viral Nucleic Acid Kit (Roche Diagnostics Corporation), a nucleic acid was purified according to the manufacturer's instructions. To the nucleic acid purified, the HC9405R-1b primer was added, and using the MMLV reverse transcriptase (Invitrogen) a reverse transcription reaction was carried out at 42°C for 1 hour according to the manufacturer's instructions to obtain cDNA.

To this reaction mixture, RNaseH (Invitrogen) was added, and reacted at 37°C for 30 minutes to digest RNA. Using a portion of this reaction mixture, and using KlenTaq LA DNA polymerase (Clontech, BD Bioscience) in the presence of the HC-Long A1 primer and the T7-HC9313R primer, a polymerase chain reaction (PCR) was carried out at a thermal cycle reaction for 30 cycles with each cycle comprising 94°C for 20 seconds and 68°C for 9 minutes to amplify cDNA of HCV genomic RNA (HCV cDNA). Using a portion of this reaction mixture, PCR was further carried out in the presence of HC85F and HC9302R primers to amplify HCV cDNA.

The amplified fragment was separated on a 0.7% agarose gel electrophoresis, and using the QIAquick gel purification kit (QIAGEN), a DNA fragment was purified from the agarose gel according to the manufacturer's instructions. The purified HCV cDNA fragment was cloned into the pGEM-T easy vector (Promega) according to the manufacturer's instructions to transform the DH5α strain. A transformant that is ampicillin-resistant and that forms a white colony on an agar medium to which IPTG and x-gal had been added in a plate culture was selected, and cultured in a 2YT medium to which ampicillin had been added to 100 µg/ml. From the cultured cells, plasmid was purified using the Wizard Plus SV Miniprep DNA Purification system.

The sequence of HCV cDNA that has been cloned into the purified plasmid was analyzed using primers prepared as appropriate so as to be consistent with the vector and HCV cDNA by carrying out a reaction with the CEQ DTCS Quick Start Kit (Beckman Coulter) according to the manufacturer's instructions and analyzing with the CEQ2000 XL DNA analysis system (Software version 4.0.0, Beckman Coulter). Based on the data obtained, the sequence data was assembled and analyzed using the Sequencer (Version 4.1.2, Gene Codes Corporation) to determine the base sequence of HCV cDNA. The sequences of three types of HCV cDNA of clones LV207-0193-1, LV207-0193-6 and LV207-0193-15 were determined.

By comparing with the published sequence (D89851) of HCV cDNA, the isolated sequences were found to contain a sequence of positions 85 to 9302 which is a primer region used for PCR, but all clones lacked a sequence corresponding to positions 1189 to 3000. The predicted amino acid sequence was found that the sequence of LV207-0193-1 encodes a stretch of an amino acid length but lacks a sequence from the middle of E1 to the middle of NS2 without deviation from (in-frame with) the translation frame. Based on the determined sequence, a common sequence and a consensus sequence were determined using the MacVector (version 7), and by combining the suitable fragments of HCV cDNA clones obtained, HCV cDNA fragments having the consensus sequence were constructed.

Specifically, to the fragment of clone 1, a ClaI site at position 709 of SEQ ID NO: 1 was mutation-introduced using Cla_s and Cla_as primers and the Quick Mutagenesis Kit (Stratagene) according to the manufacturer's instructions. From LV207-0193-1, a fragment having from the ClaI site at position 709 to the AfeI site at position 1063, a fragment from the HpaI site at 4169 to the SacI site at 5569, and a fragment having from the SfiI site at 6687 to the BglII site at 7123 were isolated and used for construction. And from LV207-0193-6, a fragment having from the AfeI site at 1063 to the BsiWI site at 1265 was isolated and used.

From LV207-0193-15, a fragment having from the BsiWI site at position 1265 to the HpaI site at position 4169, a fragment having from the SacI site at 5569 to the SfiI site at 6687 , and a fragment having from the BglII site at 7123 to the HindIII site at 7386 were isolated and used for construction. By combining these fragments, and further combining a fragment of HindIII at 7383 to XbaI at 7786 containing 3'UTR isolated from a patient tissue, they were inserted into the ClaI site to the XbaI site of a cloning vector pBluescript SKII(-) (Stratagene) to construct pLVC_ClaXba7.2K having a sequence from positions 709 to 7786 of SEQ ID NO: 1. The restriction enzymes and T4 DNA ligase were purchased from New England Biolabs, Takara Shuzo, Toyobo and NIPPON GENE Co., Ltd..

On the other hand, the end of HCV cDNA was isolated as follows. The cDNA reaction mixture treated with the above RNaseH was subjected to PCR comprising 35 thermal cycles of 94°C for 20 seconds, 55°C for 30 seconds and 72°C for 1 minute using HCLongH1 and HC705R and the JumpStart RedTaq DNA polymerase (Sigma) to amplify a fragment corresponding to positions 1 to 709 of the previously reported HCV cDNA. The cloning and analysis of fragments were carried out according to the standard methods. As a result, HCV cDNA, pLV207-0007, containing positions 1 to 709 of SEQ ID NO: 1 was obtained. By PCR using this as the template and the T7-HIV2 primer and the CoreCla_as primer, an about 0.7 kb fragment was amplified, and by cloning to pGEM-T Easy, pT7_LV207_0007 was obtained.

An about 0.7 kb fragment obtained by cleaving pT7_LV207_0007 with NotI and ClaI and an about 7.2 kb fragment obtained by cleaving pLVC_ClaXba7.2K with ClaI and XbaI were inserted in between the NotI site and the XbaI site of pcDNA3.1(+) (Invitrogen) to obtain a plasmid pcD-LV207TF having inserted therein HCV cDNA having a sequence from positions 1 to 7786 of SEQ ID NO: 1.

### Separation of the 3'UTR gene from the patient tissue

In a method similar to the above, RNA was recovered from the tissue of a patient with chronic hepatitis. To 2.5 µl of RNA, 5 pmole (0.5 µl) of primer 8913F was added, incubated at 70°C for 3 minutes, and quickly cooled on ice. To this, 2 µl of 5× First-Strand Buffer, 1 µl of 0.1 M DTT, 0.5 µl of 20 mM dNTP, 20 units of RNase Inhibitor (TAKARA), and 0.5 µl of the MMLV reverse transcriptase were added, and a diethyl pyrocarbonate-treated sterile water was added thereto to a total volume of 10 µl. The mixture was reacted at 42°C for 60 minutes. In order to disrupt RNA, 12 U of RNaseH (TAKARA, 60 U/µl) was added, incubated at 37°C for 30 minutes, and then incubated at 72°C for 3 minutes for the inactivation of RNaseH, and cDNA was used.

Using primers 8913F and RP2, 2 µl of this cDNA was subjected to PCR in a method similar to the above. The PCR product was subjected to the second PCR with primers 8939F and R1 to obtain a PCR product of about 600 bases. The PCR product was cloned into the pGEM-T Easy vector, and the nucleic acid sequence was determined in a method described above.

The following is part of primer sequences used in cloning and the construction of genes:
1b160Bam: 5'-cgcggatcct tagtcctcca gaacccggac ac-3' (SEQ ID NO: 49)
chiba-as: 5'-tgcacggtct acgagacct-3' (SEQ ID NO: 50)
chiba-s: 5'-tagtggtctg cggaaccggt-3' (SEQ ID NO: 51)
core_cla_as: 5'-gccgcatgta agggtatcga tgacc-3' (SEQ ID NO: 52)
core_cla_s: 5'-ggtcatcgat acccttacat gcggc-3' (SEQ ID NO: 53)
eco_npt_as: 5'-gcgaattctt atcagaagaa ctcgtcaaga ag-3' (SEQ ID NO: 54)
HC1b9405R: 5'-gcctattggc ctggagtgtt tagctc-3' (SEQ ID NO: 55)
HC85F: 5'-atggcgttag tatgagtgtc gtgcagcct-3' (SEQ ID NO: 56)
HC705R: 5'-agccgcatgt aagggtatcg atgac-3' (SEQ ID NO: 57)
HC1986S: 5'-tggttcggct gyacatggat gaa-3' (SEQ ID NO: 58)
HC2199AS: 5'-ggrtagtgcc aragcctgta tgggta-3' (SEQ ID NO: 59)
HC9302R: 5'-tcgggcacga gacaggctgt gatatatgtc t-3' (SEQ ID NO: 60)
HClongA1: 5'-atcgtcttca cgcagaaagc gtctagccat-3' (SEQ ID NO: 61)
HClongH1: 5'-gccagccccc tgatgggggc gacactccac c-3' (SEQ ID NO: 62)
Nde_core9_as: 5'-aatcatatgt ctttgaggtt taggatttgt-3' (SEQ ID NO: 63)
Nde_npt_s: 5'-gacatatgat tgaacaagat ggattgcac-3' (SEQ ID NO: 64)
SbfH1: 5'-gtcctgcagg ccagccccct gatgggggcg aca-3' (SEQ ID NO: 65)
SbfNpt-R: 5'-gacctgcagg ttatcagaag aactcgtcaa gaag-3' (SEQ ID NO: 66)
T7_H1V2: 5'-gccttaatta atacgactca ctataggcca gccccctgatgggggcgaca-3' (SEQ ID NO: 67)
T7_HclongH1: 5'-tctagtcgac ggccagtgaa ttgtaatacg actcactata gggcggccag ccccctgatgggggcgacac tccacc-3' (SEQ ID NO: 68)
T7_HC9313b: 5'-tctagtcgac ggccagtgaa ttgtaatacg actcactcta gggcggcggg gtcgggcwcg ngacabgctg tga-3' (SEQ ID NO: 83)

### Example 2. Isolation and analysis of cDNA to TF HCV genomic RNA from a patient

Subsequently, the detection of TF HCV-RNA from 23 samples of liver biopsy from the liver of chronic active hepatitis and 3 samples of BP1, BP2 and BP3 that are obtained from surgically removed liver tissues from patients with hepatic carcinoma.
Extraction of RNA was attempted.

### RNA extraction

According to a protocol of "Isolation of RNA from trace samples" of the extraction reagent for RNA, ISOGEN (NIPPON GENE Co., Ltd.), RNA was extracted. To a liver section of about 0.5 mm × 1 mm in dimension from a patient, 0.8 ml of ISOGEN was added, the tissue section was loosened with a 1 ml pipette tip, and disrupted by pipetting. After standing at room temperature for 5 minutes, 0.2 ml of chloroform was added, and after vigorous shaking for 30 seconds, it was allowed to stand at 4°C for 5 minutes. After centrifuging at 12,000g, 4°C for 15 minutes in a refrigerated micro centrifuge, the aqueous phase was collected.

About 5 µg of yeast tRNA was added, 0.8 ml of isopropanol was added, and the mixture was allowed to stand at 4°C for 30 minutes or overnight. After centrifuging at 12,000g, 4°C for 15 minutes, the supernatant was discarded, and the pelleted RNA was collected. To the pellet was added 1 ml of 70% ethanol, which was vigorously shaken and then centrifuged at 12,000g, 4°C for 15 minutes. The supernatant was discarded, and the same procedure was repeated twice to wash the pellet. After the last washing, the pellet was air-dried for 10 minutes, dissolved in 50 µl of a diethyl pyrocarbonate-treated sterile water to prepare a RNA sample. The sample was stored at -80°C until use.

### Synthesis of cDNA

The deleted region of the TF genome obtained from the tissue of BP207 for which cDNA was synthesized from the RNA sample extracted from the liver biopsy tissue was nucleic acids Nos. 1189 to 3000 of the HCV gene, several primers for cDNA synthesis were designed at the 3'-untranslated region side from 3000 and cDNA was synthesized. The reverse transcriptase used was the MMLV reverse transcriptase from GIBCO-BRL, and synthesis was carried out according to the attached protocol.

To 2.5 µl of RNA, 5 pmole (0.5 µl) of primers was added, incubated at 70°C for 3 minutes, and quickly cooled on ice. To this, 2 µl of 5x First-Strand Buffer, 1 µl of 0.1 M DTT, 0.5 µl of 20 mM dNTP, 20 units of RNase Inhibitor (TAKARA), and 0.5 µl of the MMLV reverse transcriptase were added, and a diethyl pyrocarbonate-treated sterile water was added thereto to a total volume of 10 µl. The mixture was reacted at 42°C for 60 minutes. In order to disrupt RNA, 12 U of RNaseH (TAKARA, 60 U/µl) was added, incubated at 37°C for 30 minutes, and then incubated at 72°C for 3 minutes for inactivation of RNaseH, and cDNA was used.

Primers used for cDNA synthesis were any of the following:
5035R: 5'-AGGCCTGTGA AGACGCTCTC CCAGAACT-3' (SEQ ID NO: 69)
HC3297R: 5'-GGTGATGAC CTTGGTCTCC AT-3' (SEQ ID NO: 70)
HC3481R: 5'-GCTTAGAGGC TAGTGATGAT GCAACCAAGT AC-3' (SEQ ID NO: 71)
HC3945R: 5'-GGCGACCGCA TAGTAGTTTC CATA-3' (SEQ ID NO: 72).
The primers used are described in Table 1.

### Amplification by polymerase chain reaction (PCR)

Using cDNA, PCR was carried out with a combination of several primers from 5'UTR to the region encoding NS3. For PCR, TaKaRa LA Taq (TAKARA) was used and reacted according to the attached protocol. Two µl of cDNA, 2.5 µl of 10× LA PCR buffer II (Mg²⁺ free), 2.5 µl of 25 mM MgCl₂, 2.5 µl of 2.5 mM dNTP, 10 pmole of a sense primer, 10 pmole of an antisense primer, 0.25 µl (5 units/µl) of TaKaRa LA Taq were added, to which a sterile water was added to 25 µl, and PCR was carried out. For the reaction, the master cycler gradient (Eppendorf Iatron) was used. The reaction profile comprised, after heating at 94°C for 2 minutes, 10 cycles of denaturation at 94°C for 20 seconds, annealing at 62°C for 30 seconds and extension at 68°C for 3 minutes, and then 25 cycles of denaturation at 94°C for 20 seconds, annealing at 58°C for 30 seconds and extension at 68°C for 3 minutes.

Using 2.5 µl of the product from the PCR (1st PCR), the second-round PCR (2nd PCR) was carried out. 2.5 µl of 1st PCR product, 2.5 µl of 10× LA PCR buffer II (Mg²⁺ free), 2.5 µl of 25 mM MgCl₂, 2.5 µl of 2.5 mM dNTP, 10 pmole of a sense primer, 10 pmole of an antisense primer, and 0.25 µl (5 units/µl) of TaKaRa LA Taq were added, to which a sterile water was added to 25 µl, and PCR was carried out in a reaction profile similar to the first PCR.

Primers used for cDNA synthesis were combinations of any of the following:
HC85F: 5'-ATGGCGTTAG TATGAGTGTC GTGCAGCCT-3' (SEQ ID NO: 56)
HC813S: 5'-CTGGAGGACG GCGTGAACTA TGCAACAGGG AA-3' (SEQ ID NO: 73)
HC841S: 5'-GGAACTTGCC CGGTTGCTCT TTCTCTATCT TC-3' (SEQ ID NO: 74)
HC3206R: 5'-TGGGGCAAGA TGGTTATAAA C-3' (SEQ ID NO: 75)
HC3174AS: 5'-GGGGTAAGAT GGTTATAAAC GTACGTACCT G-3' (SEQ ID NO: 76)
HC3111AS: 5'-ATAATGACCC CCGGCGACTT TCCGCACTAA C-3' (SEQ ID NO: 77)
HC3297R: 5'-GGTGATGAC CTTGGTCTCC AT-3' (SEQ ID NO: 70)
HC3481R: 5'-GCTTAGAGGC TAGTGATGAT GCAACCAAGT AC-3' (SEQ ID NO: 71)
HC3759R: 5'-TGACATCAGC ATGTCTCGTG ACCA-3' (SEQ ID NO: 78)
HCLONGA1: 5'-ATCGTCTTCA CGCAGAAAGC GTCTAGCCAT-3' (SEQ ID NO: 61)
HC3945R: 5'-GGCGACCGCA TAGTAGTTTC CATA-3' (SEQ ID NO: 72)
The combinations of 1st and 2nd primers used are described in Table 1.

### Analysis of PCR products

PCR products after the completion of reaction were analyzed by electrophoresis. Using 1% agarose gel, samples were subjected to submerged electrophoresis in 1xTAE buffer (Tris-HCl 40 mM, glacial acetic acid 40 mM, EDTA 1 mM). A gel loading buffer was added to the PCR product, and after electrophoresis, stained with ethidium bromide and the PCR product was observed under UV.

The image of electrophoresis of the HCV gene amplified with three sets of primers from 4 samples of RNA is shown in Fig. 1. With the combination of HC85F and HC3297R, 841S and 3759R, and 841S and 3111AS as the primers of the 2nd PCR, FLF HCV-RNA of an about 3.1 kb, 2.9 kb, and 2.2 kb should be detected, respectively. For the sample (BP274) of number 1, a PCR product of the FLF was only found with any combination of primers. For the sample (BP295) of number 2, with the combination of HC85F and HC3297R, about 3.1 kb FLF and 2 kb TF, with the combination of 841S and 3759R, an about 2.9 kb FLF, and with the combination of 841S and 3111AS, an about 2.2 kb FLF were detected.

For the sample (BP325) of number 3, with the combination of HC85F and HC3297R, an about 3.1 kb FLF, with the combination of 841S and 3759R, an about 2.9 kb FLF, and with the combination of 841S and 3111AS, an about 2.2 kb FLF and 300 bp TF were detected. For the sample (BP373) of number 4, with the combination of HC85F and HC3297R, an about 1.2 kb TF, with the combination of 841S and 3759R, an about 1 kb TF were detected, and with the combination of 841S and 3111AS, no PCR product was detected. There were samples in which FLF was only detected, samples in which FLF and TF were detected, and samples in which TF was only detected, though this varies with the combination of primers. For 26 samples, PCR products obtained with each combination of cDNA synthesis primer, the 1st PCR and the 2nd PCR primers are shown in Table 1.

**Table 1**

| | | cDNA synthesis primer | 3945R | 3481R | 3945R | 3945R | 3297R | 3174R | 5035R | | Amount of HCV-RNA |
|---|---|---|---|---|---|---|---|---|---|---|---|
| | | 1st PCR primer | HClongAl -3945R | HClongAl -3481R | 813S-3945R | 813S-3174AS | HClongA -3297R | 813S-3174AS | HClongAl -5035R | | (copis/ul RNA) |
| | Sample No. | 2nd PCR primer | 85F-3297R | 85F-3297R | 841S-3759R | 841S-3111AS | 85F-3174AS | 841-3111AS | 85F-3945R | Genotype | |
| 1 | BP171 | | - | 1.5k | 3k | 2.3k+0.5k | 1.8k | NT | NT | 1 | 424.2 |
| 2 | BP178 | | - | - | - | - | - | NT | NT | 2 | 28.9 |
| 3 | BP193 | | 3k | 3k | 3k+1k | 2.3k | 3k+0.5k | NT | NT | 1 | 1853.0 |
| 4 | BP201 | | - | - | - | - | - | NT | NT | ? | 35.0 |
| 5 | BP203 | | - | **1.2k(TF)** | - | - | **1.1k(TF)** | NT | NT | 2 | 467.4 |
| 6 | BP204 | | **3k(TF^{#}) *** | 1k | NT | NT | NT | NT | NT | 1 | NT |
| 7 | BP235 | | - | - | 3k | - | - | NT | NT | 2 | 1009.0 |
| 8 | BP245 | | 1.2k | - | NT | NT | NT | NT | NT | 1 | |
| 9 | BP248 | | - | - | - | - | 3k | NT | NT | 2 | 131.1 |
| 10 | BP257 | | - | - | - | 2.3k | - | NT | NT | 1 | 115.9 |
| 11 | BP274 | | 3k | 3k | 3k | 2.3k | 1k | NT | NT | 1 | 1375.0 |
| 12 | BP288 | | 3k | 3k | 3k | **2.3k+0.8k(TF)** | 3k+1.5k | NT | NT | 1 | 388.6 |
| 13 | BP295 | | 3k | **3k+1.5k(TF)** | 3k | 2.3k | 3k | NT | NT | 1 | 65.6 |
| 14 | BP297 | | - | - | - | - | - | NT | NT | 2 | 330.6 |
| 15 | BP298 | | - | 3k+1.7k+0.5k | - | - | - | NT | NT | ? | 1582.0 |
| 16 | BP299 | | - | - | 3k | 2.3k | 0.3k | NT | NT | 1 | 151.8 |
| 17 | BP305 | | - | 3k+1.7k+0.5k | - | - | - | NT | NT | 2 | 203.0 |
| 18 | BP325 | | 3k | 3k | 3k | **2.3k+0.3k(TF)** | 0.8+0.5k | NT | NT | 1 | 931.1 |
| 19 | BP331 | | 3k | 1.5k(TF)+0.5k | 3k | 2.3k+1.2k | 1.3k | NT | NT | 1 | 627.0 |
| 20 | BP357 | | - | - | - | - | - | NT | NT | 2 | 710.4 |
| 21 | BP368 | | - | **1k(TF)** | NT | NT | - | NT | NT | 1 | NT |
| 22 | BP372 | | 3k | 3k | 3k | 2.3k | 2.5k | NT | NT | 1 | 916.9 |
| 23 | BP373 | | **1.3k(TF)** | - | **1k(TF)** | - | 1.2k | NT | NT | - | 7979.0 |
| 24 | BP1 | | **1.3k(TF)** | NT | **1k(TF)** | NT | NT | **0.3k(TF)** | **1.9k(TF)** | NT | 442.0 |
| 25 | BP2 | | **1.3k(TF)** | NT | **1k(TF)** | NT | NT | NT | NT | NT | 88.8 |
| 26 | BP3 | | - | NT | - | NT | NT | NT | NT | NT | 46.7 |

| | | | | | | | | | | | |
|---|---|---|---|---|---|---|---|---|---|---|---|
| * Bold letters show the TF genome of which nucleic acid sequence has been determined and described in the sequence list. # TF means that deletion was confirmed. NT: not tested | | | | | | | | | | | |

### Sequence determination

The obtained PCR products thought to be FLF and TF were cloned into plasmids and the sequences were determined.

A PCR product was excised from the electrophoresed agarose gel, purified by the QIAquick PCR Purification Kit (QIAGEN), and extracted into 40 µl of sterile water. To 5 µl of DNA and 0.5 µl of pGEM-T Easy Vector (Promega), 1 µl of 10× T4 ligase buffer, 1 µl of T4 DNA ligase and 2.5 µl of sterile water were added, and reacted at 16°C for 1 hour to ligate the DNA and the vector. To competent cells of E. coli DH5α prepared by the method of Inoue et al. (Gene, vol. 96, 1990, pp. 23-28), DNA was added, and transformed according to a standard method.

The colonies that developed were cultured for one night and day in a 2xYT medium, miniprepped by the Wizard Plus SV Minipreps DNA Purification System (Promega), and plasmid DNA was collected. The plasmid DNA in which a PCR product has been inserted was analyzed and sequenced by the CEQ 2000XL DNA analysis system (BECKMAN COULTER). It was reacted using the CEQ2000 Dye Terminator Cycle Sequencing with Quick Start Kit according to the attached protocol and analyzed. As the sequence primers, primers of pGEM-T Easy and those suitable for the HCV sequence were selected as appropriate. The sequence was analyzed by the MacVector (Accelrys) and Sequencher (Gene Codes Corporation).

The nucleic acid sequence of the detected TF genome and the amino acid sequences predicted therefrom are shown in the sequence list. Sample BP203 are shown in SEQ ID NOs: 9-12, BP204 in SEQ ID NOs: 13 and 14, BP208 in SEQ ID NOs: 15 and 16, BP295 in SEQ ID NOs: 17 and 18, BP325 in SEQ ID NOs: 19 and 20, BP368 in SEQ ID NOs: 21 and 22, BP373 in SEQ ID NOs: 23 to 28, BP1 in SEQ ID NOs: 29 to 34, and BP2 in SEQ ID NOs: 35 to 38. Those in which FLF was detected and those in which TF was detected with any combination of primers are summarized in Table 2.

**Table 2**

| | TF | FLF | Genotype |
|---|---|---|---|
| BP207 | ○ | | 1 |
| BP203 | ○ | | 2 |
| BP368 | ○ | | 1 |
| BP373 | ○ | | 1 |
| BP1 | ○ | | 1 |
| BP2 | ○ | | 1 |
| BP204 | ○ | ○ | 1 |
| BP288 | ○ | ○ | 1 |
| BP295 | ○ | ○ | 1 |
| BP325 | ○ | ○ | 1 |
| BP331 | ○ | ○ | 1 |
| BP171 | | ○ | 1 |
| BP193 | | ○ | 1 |
| BP257 | | ○ | 1 |
| BP274 | | ○ | 1 |
| BP299 | | ○ | 1 |
| BP372 | | ○ | 1 |

In 6 samples TF alone was detected, in 5 samples both FLF and TF were detected, in 6 samples FLF alone was detected, and in the rest of samples neither was detected. Most of the samples in which neither was detected were genotype 2. Since the cDNA synthesis primer, and 1st PCR and the 2nd PCR primers were synthesized based on the sequence of genotype 1, the sequences may not correspond to the sequence of HCV-RNA of genotype 2, and this may be a reason that PCR products were not obtained in many samples.

### Analysis of the TF genome

The sequence of TF HCV-RNA was compared to the sequence of HCV-RNA of No. D89815. Fig. 2 shows the structures of BP207, BP368, BP373, BP1, BP2 and BP203 in which TF alone was detected. Though the deleted region was about 2 kb, none had the deletion of the same region as BP207, and regions deleted in each patient were different. BP203, the only sample among the samples of genotype 2 in which TF was detected, had a similar deletion, and the deleted region was present at nucleic acid Nos. 988 to 2988.

Common in these HCV-RNAs is the occurrence of in-frame deletion as in BP207, and it is thought that HCV polyprotein is being synthesized at regions excluding the deleted region. In particular, the gene encoding the core protein is retained in the entire form and the core protein per se is normally synthesized, and none has the deletion of transmembrane domains at two sites at the C terminal end of NS2, and thus it is suggested that proteins at NS3 and after can be normally expressed. Furthermore, since the regions encoding the E1 and NS2 proteins are connected in-frame, it is thought that a fusion protein of E1 and NS2 is being produced.

Then, for the samples in which both types of TF and FLF were detected, the structures of the TF sequences were compared (Fig. 3). The sequences of BP204, BP325, BP295 and BP288 were confirmed. In BP204 and BP295 in which the gene corresponding to the region encoding the core protein has been obtained, unlike the sample in which TF alone was detected, part or all of the core region was deleted. Also, in some samples, NS2 was retained at the part subsequent to the deleted region and part of the E2 region was deleted.

However, HCV-RNA in these samples also had in-frame deletion as in BP207, and thus excluding the deleted region, it is thought that HCV polyprotein is being synthesized.

### Study on the HCV-RNA sequence of TF and FLF

For sample of patients with chronic active hepatitis in which HCV-RNAs of TF and FLF were obtained, the nucleic acid sequences of the overlapping fragment and the predicted amino acid sequences were compared. A PCR product of the length of TF was cloned into a vector in a similar manner, and the nucleic acid sequence was determined. The sequences of FLF of BP204, BP325 and BP208 are shown in SEQ ID NOs: 39 and 40, 41 and 42, and 43 and 44, respectively. Comparison of the nucleic acid sequences and amino acid sequences revealed that it is 96.7% for nucleic acid and 97.5% for amino acid in BP325, and 97.3% for nucleic acid and 97.7% for amino acid in BP288, suggesting that they are viruses belonging to the same quasispecies. On the other hand, in BP204, sequences of TF and FLF were different with nucleic acid 82.6% and amino acid 83.6%.

**Table 3**

| | Nucleic acid | Amino acid |
|---|---|---|
| BP204 | 82.6% | 83.6% |
| | (298n.t.) | (98a.a.) |
| BP325 | 96.70% | 97.50% |
| | (245n.t.) | (81a.a.) |
| BP288 | 97.3% | 97.7% |
| | (786n.t.) | (264a.a.) |

### Separation of TF-HCV-RNA from the serum

For samples in which TF-HCV-RNA was detected from the liver tissue, separation of TF-HCV-RNA from the serum was attempted. It was extracted using the High Pure Viral RNA Kit (Roche) from the serum collected simultaneously with the liver biopsy. From 200 µl of the serum, it was extracted into 50 µl of the elution buffer according to the attached protocol. As used herein, serum corresponding to BP368, which is a sample for liver biopsy, is designated as S368.

Using 2.5 µl of RNA, cDNA was synthesized from RNA, and PCR products were obtained in a method similar to that in liver biopsy. When the 3297R primer was used in cDNA synthesis, HCLONGA1 and 3297R were used in the 1st PCR and 85F and 3174AS were used in the 2nd PCR for S368, a 1.2 kb possible PCR product of TF was obtained (Table 4) .

**Table 4**

| | | | cDNA synthesis primer | 3945R | 3481R | 3297R | Amount of HCV-RNA |
|---|---|---|---|---|---|---|---|
| | | | 1st PCR primer | Al-3945R | Al-3481R | Al-3297R | (copis/ul RNA) |
| | | Genotype | 2nd PCR primer | 85F-3297R | 85F-3297R | 85F-3174AS | |
| 1 | S171 | 1 | | - | - | - | 12.3 |
| 3 | S193 | 1 | | - | - | - | 36.7 |
| 5 | S203 | 2 | | - | - | - | - |
| 6 | S204 | 1 | | - | - | - | 140.9 |
| 7 | S207 | 1 | | - | - | - | 713 |
| 12 | S288 | 1 | | - | - | - | - |
| 15 | S298 | ? | | - | - | - | 1.2 |
| 17 | S305 | 2 | | - | - | - | 4.2 |
| 18 | S325 | 1 | | - | - | - | 9.9 |
| 19 | S331 | 1 | | - | - | - | 3.1 |
| 21 | S368 | 1 | | - | - | **1.2kbp(TF)** | 297.6 |
| 23 | S373 | 1 | | - | - | - | 6.2 |

| | | | | | | | |
|---|---|---|---|---|---|---|---|
| * Bold letters show the TF genome of which nucleic acid sequence has been determined and described in the sequence list. | | | | | | | |

Then, for S204, S207 and S368 which had a relatively large amount of RNA in the quantitation of HCV-RNA at the 5'UTR region, cDNA synthesis to PCR were carried out with different combinations of primers. As a result, a PCR product with the length of TF was obtained for S207 and S368 and a PCR product with the length of FLF was obtained for S204 (Table 5). The PCR products were similarly cloned into the pGEM-T Easy vector and the sequences were determined. The sequences determined for S207 and S368 are shown in SEQ ID NOs: 45 and 46 and SEQ ID NOs: 47 and 48, respectively. When the sequence homology of the nucleic acid sequence and the amino acid sequence for the overlapping region of BP207 and S207, and BP368 and S368 are compared, they were 99.4% and 99.3% for BP207 and S207, and 98.8% and 97.3% for P368 and S368, suggesting that they are viruses belonging to the same quasispecies (Table 6). This indicates that TF-HCV-RNA replicated in the liver is being released into the serum by some system.

**Table 5**

| | cDNA synthesis | 3945R | 3481R | 3297R | 3565AS | 3519AS | 3447AS |
|---|---|---|---|---|---|---|---|
| | 1st PCR | 813S-3297R | 813S-3297R | 813S-3297R | 813S-3297R | 813S-3297R | 813S-3297R |
| Sample No. | 2nd PCR | 841S-3174AS | 841S-3174AS | 841S-3174AS | 841S-3174AS | 841S-3174AS | 841S-3174AS |
| S204 | | - | - | - | - | 2.2kbp (FLF) | - |
| S207 | | - | - | **0.5kbp (TF)** | - | - | 0.5kbp |
| S368 | | 0.5kbp (TF) | 0.5kbp (TF) | 0.5kbp (TF) | 0.5kbp (TF) | 0.5kbp (TF) | 0.5kbp |

| | | | | | | | |
|---|---|---|---|---|---|---|---|
| * Bold letters show the TF genome of which nucleic acid sequence has been determined and described in the sequence list. | | | | | | | |

**Table 6**

| | Nucleic acid | Amino acid |
|---|---|---|
| BP207 | 99.4% | 99.3% |
| vs S207 | (490n.t.) | (163a.a.) |
| BP368 | 98.8% | 97.3% |
| vs S368 | (1018n.t.) | (263a.a.) |

### Example 3. Construction of HCV RNA replicon

By inserting a linker fragment obtained by annealing the XhoX-Xba-s oligomer and the XhoX-Xba-as oligomer in between the XhoI site and the XbaI site of pBluescript IISK(+), pBSIISK(+) ΔXX was constructed. Also, by subjecting pLV207-0007 to PCR using the Sbf_H1 primer and the Cla_as primer, an about 0.7 kb fragment was amplified, which was cloned into pGEM-T Easy to obtain pLVC-0007Sbf. By linking and inserting an about 0.7 kb fragment obtained by cleaving pLVC-0007Sbf with NotI and ClaI and an about 7.2 kb fragment obtained by cleaving pLVC_ClaXba7.2K with ClaI and XbaI in between the NotI site and the XbaI site of pBSIISK(+)ΔXX, pSbf-LV207TF was obtained.

On the other hand, by adding the T7-HC9313b primer to RNA purified from HCV antibody-positive serum G14, cDNA was synthesized using the SuperscriptII reverse transcriptase (Invitrogen) according to the manufacturer's instructions. From this cDNA reaction mixture, HCV cDNA was amplified by PCR (35 cycles with one cycle comprising 95°C for 30 seconds, 55°C for 1 minute, 74°C for 1 minute) using EX-Taq DNA polymerase (Takara Shuzo) in the presence of the T7-HClongH1 primer and the 1b160Bam primer. After separating fragments amplified by PCR on agarose gel electrophoresis, DNA was purified from the agarose gel using the QIAquick gel kit. The purified fragment was cloned into the pT7-blue T (Novagen), and the sequence was determined using the Applied Biosystems DNA sequencer 377A using reagents and conditions recommended by the manufacturer.

Then, HCV cDNA was amplified by PCR using the T7-H1V2 primer and the nde_core9_as primer. On the other hand, PCR was carried out with nde_npt_S and the EcoNpt_as primer as the template using pcDNA3.1(+), and a neomycin-resistant gene fragment was amplified. By linking these fragments with the restriction site of NdeI and then cloning this into pBluescriptIISK(-), a fusion fragment of 5'UTR, the first 9 amino acids of core of HCV cDNA and the neomycin-resistant gene (neomycin phosphotransferase, NPT-II) was constructed. A plasmid having this fragment was further subjected to PCR using the T7-HlV2 primer and the Sbf_Npt_R primer to prepare a fragment having a PacI site at the 5'-end and a SbfI site at the 3'-end. By cloning this into pGEM-T Easy, pG14UTRcNEO was obtained.

By inserting an about 1.2 kb fragment obtained by cleaving pG14UTRcNEO with NotI and SbfI into between the NotI site and the SbfI site of pSbf-LV207TF, pLV207TFRepG14 was obtained. Using this plasmid DNA cleaved with XbaI and NotI as the template, RNA was synthesized using the Megascript T7 kit (Ambion). RNA was purified according to the manufacturer's instructions.

Human hepatoma cells (Huh7, JCRB0403) were cultured at 5% carbon dioxide at 37°C in the Dulbecco's Modified Eagle's Medium (D-MEM, IWAKI) supplemented with 10% fetal bovine serum (FBS), penicillin and streptomycin to 50 U/ml and 50 ug/ml, respectively. The subconfluent cells were treated with trypsin and EDTA to detach them from the culture plate, and resuspended into a serum-added medium to inactivate trypsin. After washing twice with PBS, they were suspended in Cytomix (120 mM potassium chloride, 10 mM potassium phosphate, 5 mM magnesium chloride, 25 mM HEPES, 0.15 mM calcium chloride, 2 mM EGTA, pH 7.6) supplemented 1.25% DMSO, it was then transferred to an electroporation cuvette with a gap of 0.4 cm.

After adding a suitable amount of RNA to cells, they are fully cooled on ice for 5 minutes. Cells were pulsed at 270 V and 960 µF using the electroporator (Bio-Rad). They are immediately resuspended into 8 ml of a medium, and are transferred into culture plates. After culturing for a certain period, the cells are detached with 0.1% EDTA, PBS, precipitated by centrifugation, and collected. From the cells collected, RNA was isolated using Isogen (NIPPON GENE) according to the manufacturer's instructions. The amount of HCV RNA contained in the isolated RNA was analyzed by the quantitative RT-PCR method.

### Method of determining the minus strand

The presence of HCV RNA replication was investigated by whether the minus strand in the 5'UTR regin of HCV RNA can be detected in the cells. A specific determination method for the minus strand was carried out similarly to the specific detection method for the minus strand described in the Japanese Unexamined Patent Publication (Kokai) No. 08-187097.

A significant amount of a minus strand RNA could be detected in the cells in which RNA in vitro-synthesized with pLV207TFRepG14 as the template was transfected by the electroporation, and therefore the replication of HCV RNA was confirmed.

### Example 4. Correlation of the quantitative ratio of truncated RNA to the total RNA with HCV-related diseases

The quantitation of HCV RNA isolated from patient samples was determined as follows. When RNA is determined using 5'UTR as the target, Chiba-S and Chiba-AS primers were used. Using the QauntiTect SYBR Green RT-PCR Kit (QIAGEN), a reaction mixture was prepared under the condition recommended by the manufacturer, transferred to the LightCycler Capillary (Roche Diagnostics), set to the LightCycler (Roche Diagnostics), reacted and PCR products were monitored with time. Appropriately diluted known concentrations of RNA containing in vitro-synthesized HCV 5'UTR were used as the standards. LightCycler software (V3.5.3) was used in analysis.

When RNA was determined with the E2 region as the target, the HC1986S primer and the HC2199-as primer were used. Using the OneStep RT-PCR kit (QIAGEN), a reverse transcription reaction was carried out under the condition recommended by the manufacturer. This reaction mixture was added to a reaction mixture prepared by the LightCycler-FastStart DNA Master SYBR Green I kit (Roche Diagnostics) containing equal amounts of primers, transferred to the LightCycler Capillary (Roche Diagnostics), and set to the LightCycler (Roche Diagnostics). This was subjected to a PCR reaction, and PCR products were monitored with time. Appropriately diluted known concentrations of RNA containing in vitro-synthesized HCV 5'UTR were used as the standards, and using the LightCycler software, the quantitative values were determined.

### Example 5. Expression of cDNA encoding a truncated sequence in a mammalian cell and analysis of HCV protein

By inserting an about 8.5 kb fragment obtained by cleaving pLV207TFRepGl4 with NotI and XbaI into NotI and XbaI of pcDNA3.1, pcD-LVTRG was obtained. This plasmid DNA was transfected into human kidney-derived cultured cells 293TRex using the Lipofectamine 2000 (Invitrogen). At four hours after DNA transfection, the medium was changed, and the cells were further cultured for 18 hours. Cells were detached from the plate, precipitated by centrifugation, and collected. The cells were disrupted by pipetting in the RIPA buffer, and the supernatant was collected by centrifugation. To the collected supernatant, a 1/3 amount of 3x SDS sample buffer (187.5 mM Tris-HCl [pH 6.8], 6% SDS, 125 mM DTT, 30% glycerol) was added, and heat-treated at 95°C for 5 minutes.

It was applied on a polyacrylamide gel (Daiichi Kagaku Yakuhin), and was subjected to electrophoresis according to the manufacturer's instructions. After electrophoresis, the proteins were transferred to a PVDF membrane (Millipore) using the Semi-Dry blotter (Sartorius) according to a standard method. After washing the transferred membrane in TTBS (20 mM Tris-HCl [pH 7.5], 150 mM NaCl, 0.1% Tween 20), it was reacted in a 10-fold diluted blocking agent (Milk Diluent/Blocking Solution, Kirkegaad & Perry Laboratories) at room temperature for 2 hours. A primary antibody diluted to a final concentration of 0.3 µg/ml was added thereto, and reacted under shaking at room temperature for 1.5 hour. The reaction mixture was discarded, washed three times in TTBS, and then a 40,000-fold diluted HRP-labelled antibody was added thereto, and reacted under shaking at room temperature for 1 hour.

The reaction mixture was discarded, washed three times in TTBS, and then reacted using the SuperSignal West Pico Chemiluminescent Substrate (Pierce) at room temperature for 5 minutes. The resulting chemiluminescence was detected using the LAS1000 (Fuji Film) or, when the signal was inadequate, exposed to the BioMax Film (Kodak) for detection. As predicted from the sequences of HCV cDNA of pLV207TRG, the core antigen was detected at a position corresponding to the molecular weight of the matured core antigen and the NS3 antigen was detected at a position corresponding to the molecular weight of the matured NS3 antigen using an anti-core monoclonal antibody and an anti-NS3 rabbit antiserum, respectively.

Furthermore, when reacted with an anti-E1 monoclonal antibody, the proteins having a molecular weight close to the normal molecular weight of about 35 kd can be detected, whereas after EndoH-treatment, it changed to a molecular weight of 24 kd. This molecular weight almost agrees with the molecular weight of an amino acid sequence predicted from HCV cDNA of LV207, said molecular weight being calculated from the amino acid sequence of a fusion protein of E1 and NS2. This revealed that E1 and NS2 occur as a fusion protein, and undergo sugar-chain modification. These have shown that the HCV polyprotein encoded by HCV cDNA of LV207 has been cleaved at the same cleavage site as the polyprotein of the FLF type.

### Example 6. Construction of HCV RNA replicon and replication in the cell

A replicon of the TF type having no neomycin-resistant gene, a drug resistant marker, was constructed. The pSbf-LV207TF plasmid constructed in Example 3 was cleaved with NotI and ClaI to obtain an about 0.7 kb fragment. By inserting this fragment in between the NotI site and the ClaI site of plasmid pLV207TFRepG14 constructed in Example 3, a plasmid pLV207TF was constructed.

With this plasmid cleaved with NotI and XbaI as the template, RNA was synthesized using the Megascript T7 Kit (Ambion). This RNA was purified according to the manufacturer's instructions, and used for transfection into cells.

The purified RNA was transfected by electroporation into the human hepatoma cells Huh7 that had been cultured for 2 days. The cells were detached by treating with trypsin and EDTA, resuspended into a serum-added medium, and trypsin was inactivated. After washing twice in PBS, they were suspended in Cytomix (120 mM potassium chloride, 10 mM potassium phosphate, 5 mM magnesium chloride, 25 mM HEPES, 0.15 mM calcium chloride, 2 mM EGTA, pH 7.6) supplemented 1.25% DMSO, and then about 4x10⁶ cells were transferred to an electroporation cuvette with a gap of 0.4 cm.

After adding 10 µg of RNA into the cuvette, the cells are fully cooled on ice for 5 minutes. Cells were pulsed at 270 V 960 µF using the electroporator (Bio-Rad). They are immediately resuspended into 8 ml of a medium, and seeded in a 12-well plate (22.1 mm in diameter). At 4 hours, 24 hours, 48 hours, 72 hours and 96 hours, the cells were detached with 0.1% EDTA-PBS, and collected by centrifugation. The cell pellet was dissolved in 50 µl of the RIPA buffer (20 mM Tris-HCl [pH 7.5], 150 mM NaCl, 1 mM EDTA, 1% NP40, 0.1% deoxycholate, 0.1% SDS, a complete protease inhibitor cocktail [Roche Diagnostics Corporation]), and After centrifuging at 10 krpm for 5 minutes, the supernatant was collected. The amounts of HCV core antigen in 10 µl of the supernatant was determined using a kit (Fujirebio, Lumipulse) for HCV core antigen.

As shown in Fig. 4, the measured value for the core antigen is below the detection limit until 24 hours, it started to increase from 48 hours and was increasing after 96 hours. This indicates that the replicon of TF type of the present invention is replicated in the cells and is replicating the core protein. It demonstrates that the TF genome having the same structure as that replicating in the liver can be replicated in vitro.

### Example 7. Preparation of the truncated form gene from HCV samples of the genotype 2

In Example 2, a truncated form gene was detected from a biopsy sample of a patient with chronic hepatitis by the RT-PCR method, whereas no truncated form gene was detected from samples of the genotype 2 except the sample of BP203. The reason for this was possibly that the sequences of the primers used for detection were designed based on the sequence of the genotype 1.

Thus, based on the sequence of the genotype 2, primers were designed, and with the primers, the detection of the truncated form gene from biopsy samples of patients with chronic hepatitis C of the genotype 2 was attempted.

cDNA synthesis, PCR, cloning of PCR fragments and determination of base sequences were carried out for samples of the getnotype 2 shown in Table 1 according to the method of Example 2, except the primers used. Combinations of cDNA synthesis and PCR primers were the following two sets. In primer set A, the primers are 2a_HC3293R for cDNA synthesis, 2a_807S and 2a_3216R for 1st PCR, and 2a_HC835S and 2a_HC3203R for 2nd PCR, and in primer set B, 2a_HC3156R for cDNA synthesis, 2a_807S and 2a_3144R for 1st PCR, and 2a_HC835S and 2a_HC3108R for 2nd PCR. The sequences of the primers are shown below. 2a_HC3293R: TCTCCATTGGGCTGAACACCACAGGCTCCAC (SEQ ID NO: 84)
2a_HC3216R: GGGGAGAGGTGGTCATAGATGTAAGTGCCGG (SEQ ID NO: 85)
2a_HC3203R: CATAGATGTAAGTGCCGGTCCACCTGCCTA (SEQ ID NO: 86)
2a_HC3144R: CTCCTGCGAGGTGTCTCACCAGGGTACACA (SEQ ID NO: 87)
2a_HC3108R: AGCAGAGCGTGAGCTCTGACGAAGTATGG (SEQ ID NO: 88)
2a_HC835S: GGAATCTACCCGGTTGCTCTTTTTCTATCTTC (SEQ ID NO: 89)
2a_HC807S: CTGGAAGACGGGATAAATTATGCAACAGGGAA (SEQ ID NO: 90)

As shown in Table 7, in 6 of 9 samples for primer set A and in 2 of 9 samples for primer set B, genes having deletion were detected. Sequence of these genes indicated that, BP203, BP235 and BP297 had a deletion of about 2 kb in 987-2999nt, 1060-2945 nt and 1024-2966 nt, respectively, and the typical truncated form genes connected in-frame were observed. The sequence of the deleted region is shown in Fig. 5.

**Table 7**

| | primer | set A | primer | set B |
|---|---|---|---|---|
| | FL | TF | FL | TF |
| 178 | - | ND | - | ND |
| 201 | - | ND | - | ND |
| 203 | ND | - | ND | ND |
| 235 | ND | - | - | ND |
| 248 | - | - | - | ND |
| 297 | - | - | - | - |
| 298 | - | - | - | - |
| 305 | - | - | - | ND |
| 357 | - | ND | - | ND |

| | | | | |
|---|---|---|---|---|
| ND: not detected Primer set A cDNA: 2a_HC3293R 1st PCR: 2a_HC807S×2a_HC3216R 2nd PCR: 2a _HC835S×2a_HC3203R Primer set B cDNA: 2a_HC3156R 1st PCR: 2a_HC807S×2a_HC3144R 2nd PCR: 2a_HC835S×2a_HC3108R | | | | |

### Example 8. The detection rate of the truncated form gene from the liver tissue of patients with chronic active hepatitis and patients with hepatic carcinoma

Summarizing the results of Example 2 and Example 7 revealed that in 6 samples of BP207, BP203, BP325, BP297, BP368 and BP373 among the liver tissues of 24 tissue samples including BP207 from patients with chronic active hepatitis, typical truncated form genes in which an about 2 kb gene from E1 to NS2 is deleted in-frame were detected. On the other hand, from 3 hepatoma tissues, a similar typical truncated form gene was detected in BP1 and BP2. The detection rates of truncated form gene for them were 25% (6/24) in patients with chronic hepatitis and 66.6% (2/3) in patients with hepatic carcinoma, and the detection rate increased with the progress from chronic hepatitis to hepatic carcinoma.

Furthermore, the detection of the truncated form gene in 20 plasma samples from asymptomatic carriers was attempted, but no typical truncated form gene was detected. The result indicates that the progress of pathological conditions from chronic hepatitis to cirrhosis to hepatic carcinoma by the infection of hepatitis C virus is related to the presence of the truncated form gene in some way. Thus, the detection of the truncated form gene is considered to be useful as a predictive factor for the progress of pathological conditions.

### Example 9. Preparation of the truncated form gene from patients with fulminant hepatitis

Preparation of the truncated form gene from serums of patients with fulminant hepatitis was attempted. From the patient serum, RNA was extracted using ISOGEN-LS (NIPPON GENE CO., LTD.) according to the attached instruction.

In a procedure similar to that used in obtaining genes from BP207 in Example 1, the HCV gene from positions 85 to 9302 was obtained from this RNA. Sequences of 11 clones that were cloned into the pGEM-T Easy vector indicated that they are typical truncated form gene in which positions 922 to 1062, positions 1096 to 1131 and positions 1209 to 2997 were deleted in 10 clones. Though in the rest of one clone, there was no deletion from positions 1096 to 1131, but the clone was typical truncated form gene in which positions 922 to 1062 and positions 1209 to 2997 were deleted.

Furthermore, cDNA in the 5'-untranslated region and the 3'-untranslated region was obtained from the RNA of this patient in the method described in Example 1.

Then, in order to determine the sequence of the end of the 5'-untranslated region, obtainment of the end sequence by the 5' RACE method was attempted. From patient RNA, using the kit of the 5' RACE System for Rapid Amplification of cDNA Ends, Version 2.0 (Invitrogen Corporation) according to the attached instruction, the end of the 5'-untranslated region of HCV was obtained. As the antisense primer for cDNA synthesis, Chiba-as was used. cDNA was synthesized by the SuperScript II Reverse Transcriptase, purified by the S.N.A.P. column, and then purified cDNA was subjected to TdT-tailing reaction to add dCTP. The cDNA was subjected to the 1st PCR using the 5' RACE Abridged Anchor primer, KY78 primer attached to the kit: 5'-CTCGCAAGCACCCTATCAGCCAGT-3' (SEQ ID NO: 91) and LA Taq (TAKARA). With a portion of the PCR product as the template, the 2nd PCR was carried out using the UAP primer, the KM2 primer attached to the kit: 5'-AGGCATTGAGCGGGTTTATC-3' (SEQ ID NO: 92) and LA Taq (TAKARA) to obtain a PCR product. This PCR product was cloned into the pGEM-T Easy vector, and the sequence was determined. As a result, this truncated form gene had from the sequence at position 1 to the 5'-untranslated region similarly to the standard full-length HCV gene.

Furthermore, in order to determine the sequence of the end of the 3'-untranslated region, obtaining the end sequence by the 3' RACE method was attempted. First, using the Poly(A) Tailing Kit (Ambion, Inc.) according to the attached instruction, Poly(A) was added to the RNA from the patient. From the Poly(A)-added RNA, using dT-Adp primer: 5'-CTAGACTCGAGTCGACATCGTTTTTTTTTTTTTTTTTT-3' (SEQ ID NO: 93), cDNA was synthesized in a manner similar to the procedure for cDNA synthesis described in Example 1. With this cDNA as the template, the 1st PCR was carried out using 3UTR-1F primer: 5'-ATCTTAGCCCTAGTCACGGC-3' (SEQ ID NO: 94) and the Adp primer: 5'-CTAGACTCGAGTCGACATCG-3' (SEQ ID NO: 95), and the 2nd PCR was carried out using XR58F: 5'-CTAGCTGTGAAAGGTCCGTGAGCCGCATGA-3' (SEQ ID NO: 96) and the Adp primer (SEQ ID NO: 95) using LA Taq (TAKARA). This PCR product was cloned into the pGEM-T Easy vector, and the sequence was determined. As a result, the 3'-end of this truncated form gene had a 3'-end similar to that in the standard full-length HCV gene.

### Industrial Applicability

As an application example of the present invention, the replicon replication system can be used in drug screening for the development of therapeutic agents for hepatitis C virus. This system can also be used in the efficacy evaluation and the production of therapeutic agents. Also, the detection system of the TF genome can be used as a pathological marker for hepatitis C and is useful as a diagnostic reagent.

## Claims

1. A truncated form hepatitis C virus gene wherein part of the E1 protein-coding region, the E2 protein-coding region, the P7 protein-coding region and part of the NS2 protein-coding region have been deleted while retaining the translation frame in the hepatitis C virus gene.

2. The truncated form hepatitis C virus gene according to claim 1, said gene having all or part of a region encoding from the 5'-untranslated region to the core protein which is a structural protein and all or part of a region encoding from a region encoding two transmembrane domains at the latter part of NS2 which is a nonstructural protein to the 3'-untranslated region.

3. The truncated form hepatitis C virus gene according to claim 1, said gene having all or part of No. 1 to No. 914 and all or part of No. 3001 and after of the nucleic acid sequence of the hepatitis C virus gene.

4. The replicon gene according to any one of claims 1 to 3 that autonomously replicates itself in the cell.

5. The replicon gene according to claim 4 to which a selection marker gene has been connected.

6. The cell in which the above replicon according to claim 4 or 5 is replicated.

7. A method of screening or evaluating the efficacy of drugs using the cell according to claim 6.

8. A cell retaining a vector having integrated the gene according to any one of claims 1 to 3 and that is expressing the protein.

9. A method of diagnosing HCV using the cell according to claim 6 or 8 in which the replicon is being replicated or the protein produced by the cell.

10. A method of detecting the truncated form gene of hepatitis C virus using a method that detects the deletion of a gene.

11. A method of detecting or quantitating the truncated form gene by amplifying the truncated form gene by PCR with primers designed from sequences of nucleic acids 1-914 and 3001 and after of the hepatitis C virus gene.

12. A method of determining the quantitative ratio by quantitating the gene at the common region of the truncated form gene and the full-length form gene, and quantitating the gene at the deleted region of the truncated form gene.

13. Hepatitis C virus particles or hepatitis C virus-like particles retaining the gene described in claims 1 to 3.

14. A polyprotein of the hepatitis C virus produced by the gene according to any one of claims 1 to 3 and a protein processed from the polyprotein.

15. An antibody that specifically recognizes the protein according to claim 14.


SEQUENCE LISTING

<110> Advanced Life Science Institute, Inc.

<120> HCV truncated genome RNA

<130> R774

<160> 96

<170> PatentIn version 3.1

<210> 1
<211> 7785
<212> DNA
<213> Hepatitis C virus

<220>
<221> 5'UTR
<222> (1)..(341)
<223>

<220>
<221> gene
<222> (342)..(7559)
<223>

<220>
<221> CDS
<222> (342)..(950)
<223> core protein

<220>
<221> CDS
<222> (951)..(1607)

<223> truncated E1-NS2 fusion protein

<220>
<221> CDS
<222> (1608)..(3500)
<223> NS3 protein

<220>
<221> CDS
<222> (3501)..(3662)
<223> NS4A protein

<220>
<221> CDS
<222> (3663)..(4442)
<223> NS4B protein

<220>
<221> CDS
<222> (4443)..(5786)
<223> NS5A protein

<220>
<221> CDS
<222> (5787)..(7562)
<223> NS5B protein

<220>
<221> 3' UTR
<222> (7560)..(7775)

<223>

<400> 1
gccagccccc tgatgggggc gacactccac catagatcac tcccctgtga ggaactactg 60

tcttcacgca gaaagcgtct agccatggcg ttagtatgag tgtcgtgcag cctccaggac 120

cccccctccc gggagagcca tagtggtctg cggaaccggt gagtacaccg gaattgccag 180

gacgaccggg tcctttcttg gatcaacccg ctcaatgcct ggagatttgg gcgtgccccc 240

gcgagactgc tagccgagta gtgttgggtc gcgaaaggcc ttgtggtact gcctgatagg 300

gtgcttgcga gtgccccggg aggtctcgta gaccgtgcac c atg agc acg aat cct 356
Met Ser Thr Asn Pro
1               5

aaa cct caa aga aaa acc aaa cct aac acc aac cgc cgc cca cag gac 404
Lys Pro Gln Arg Lys Thr Lys Pro Asn Thr Asn Arg Arg Pro Gln Asp
                10                  15                  20

gtc aag ttc ccg ggc ggt ggt cag atc gtt ggt gga gtt tac ctg ttg 452
Val Lys Phe Pro Gly Gly Gly Gln Ile Val Gly Gly Val Tyr Leu Leu
            25                  30                  35

ccg cgc agg ggc ccc cgg ttg ggt gtg cgc gcg act agg aag act tcc 500
Pro Arg Arg Gly Pro Arg Leu Gly Val Arg Ala Thr Arg Lys Thr Ser
        40                  45                  50

gag cgg tcg caa cct cgt gga agg cga caa cct atc ccc aag gct cgc 548
Glu Arg Ser Gln Pro Arg Gly Arg Arg Gln Pro Ile Pro Lys Ala Arg
    55                  60                  65

cgg ccc gag ggc agg gcc tgg gct cag ccc ggg tac ccc tgg ccc ctc 596
Arg Pro Glu Gly Arg Ala Trp Ala Gln Pro Gly Tyr Pro Trp Pro Leu
70                  75                  80                  85

tat ggc aat gag ggc tta ggg tgg gca gga tgg ctc ctg tca ccc cgc 644
Tyr Gly Asn Glu Gly Leu Gly Trp Ala Gly Trp Leu Leu Ser Pro Arg
                90                  95                  100

ggc tct cgg cct agt tgg ggc ccc acg gac ccc cgg cgt agg tcg cgt 692
Gly Ser Arg Pro Ser Trp Gly Pro Thr Asp Pro Arg Arg Arg Ser Arg
            105                 110                 115

aac ttg ggt aag gtc atc gat acc ctc aca tgc ggc ttc gcc gac ctc 740
Asn Leu Gly Lys Val Ile Asp Thr Leu Thr Cys Gly Phe Ala Asp Leu
        120                 125                 130

atg ggg tac att ccg ctc gtc ggt gcc ccc cta ggg ggc gct gcc agg 788
Met Gly Tyr Ile Pro Leu Val Gly Ala Pro Leu Gly Gly Ala Ala Arg
    135                 140                 145

gcc cta gca cat ggt gtc cgg gtt ctg gag gac ggc gtg aac tac gca 836
Ala Leu Ala His Gly Val Arg Val Leu Glu Asp Gly Val Asn Tyr Ala
150                 155                 160                 165

aca ggg aat ttg ccc ggt tgc tct ttc tct atc ttc ctc ttg gct ctg 884
Thr Gly Asn Leu Pro Gly Cys Ser Phe Ser Ile Phe Leu Leu Ala Leu
                170                 175                 180

ctg tcc tgt ctg acc atc cca gct tcc gct tat gaa gtg cgc aac gtg 932
Leu Ser Cys Leu Thr Ile Pro Ala Ser Ala Tyr Glu Val Arg Asn Val
            185                 190                 195

tcc gga ata tac cat gtc acg aac gac tgc tcc aac tca agc att gtg 980
Ser Gly Ile Tyr His Val Thr Asn Asp Cys Ser Asn Ser Ser Ile Val
        200                 205                 210

tat gag gca gcg gac gtg atc atg cat acc ccc ggg tgc gtg ccc tgt 1028
Tyr Glu Ala Ala Asp Val Ile Met His Thr Pro Gly Cys Val Pro Cys
    215                 220                 225

gtt cgg gag ggt aac gcc tcc cgc tgt tgg gca gcg ctc act ccc acg 1076
Val Arg Glu Gly Asn Ala Ser Arg Cys Trp Ala Ala Leu Thr Pro Thr
230                 235                 240                 245

ctc gcg gtc ggg aat gcc agc gtc ccc act aag gca ata cgg cgc cac 1124
Leu Ala Val Gly Asn Ala Ser Val Pro Thr Lys Ala Ile Arg Arg His
                250                 255                 260

gtc gat ctg ctt gtt ggg acg gct gct ttc tgc tcc gcc atg tac gtg 1172
Val Asp Leu Leu Val Gly Thr Ala Ala Phe Cys Ser Ala Met Tyr Val
            265                 270                 275

ggg gat ctc tgc gga tac atc acc aaa ctc ctg ctc gcc aca ctc ggt 1220
Gly Asp Leu Cys Gly Tyr Ile Thr Lys Leu Leu Leu Ala Thr Leu Gly
        280                 285                 290

ctg ctc atg gtg ctc cag gct gcc ata gct agg gtg ccg tac ttc gta 1268
Leu Leu Met Val Leu Gln Ala Ala Ile Ala Arg Val Pro Tyr Phe Val
    295                 300                 305

cgc act cag ggg ctc att cgt gtg tgt atg tta gtg cgg aaa gtc gcc 1316
Arg Thr Gln Gly Leu Ile Arg Val Cys Met Leu Val Arg Lys Val Ala
310                 315                 320                 325

ggg ggt cac tat gcc cag atg gcc ttc atc aag ctg gcc gca ctg aca 1364
Gly Gly His Tyr Ala Gln Met Ala Phe Ile Lys Leu Ala Ala Leu Thr
                330                 335                 340

ggt aca tac gtt tat gac cat ctt act cca ctg cga gat tgg gcc cat 1412
Gly Thr Tyr Val Tyr Asp His Leu Thr Pro Leu Arg Asp Trp Ala His
            345                 350                 355

gcg ggc ctg cga gac ctt gcg gtg gca gtg gag ccc gtc atc ttc tct 1460
Ala Gly Leu Arg Asp Leu Ala Val Ala Val Glu Pro Val Ile Phe Ser
        360                 365                 370

gac atg gag acc aag atc atc acc tgg gga gca gac acc gcg gcg tgt 1508
Asp Met Glu Thr Lys Ile Ile Thr Trp Gly Ala Asp Thr Ala Ala Cys
    375                 380                 385

ggg gat att att ttg ggt ctg ccc gtc tcc gcc cga agg ggg agg gag 1556
Gly Asp Ile Ile Leu Gly Leu Pro Val Ser Ala Arg Arg Gly Arg Glu
390                 395                 400                 405

ata ctt ctg ggg ccg gcc gat agt ctt gag ggg cgg ggg tgg cga ctc 1604
Ile Leu Leu Gly Pro Ala Asp Ser Leu Glu Gly Arg Gly Trp Arg Leu
                410                 415                 420

ctt gcg ccc atc acg gct tat tct caa cag acg cgg ggt tta ctc ggc 1652
Leu Ala Pro Ile Thr Ala Tyr Ser Gln Gln Thr Arg Gly Leu Leu Gly
            425                 430                 435

tgc atc atc act agt ctc acg ggc cgg gac aag aac cag gtc gag ggg 1700
Cys Ile Ile Thr Ser Leu Thr Gly Arg Asp Lys Asn Gln Val Glu Gly
        440                 445                 450

gag gtt caa gtg gtt tcg acc gcg aca caa tcc ttc ctg gcg acc tgt 1748
Glu Val Gln Val Val Ser Thr Ala Thr Gln Ser Phe Leu Ala Thr Cys
    455                 460                 465

gtc aac ggc gtg tgt tgg act gtc tat cat ggt gcc ggc tca aaa acc 1796
Val Asn Gly Val Cys Trp Thr Val Tyr His Gly Ala Gly Ser Lys Thr
470                 475                 480                 485

cta gcc ggc cca aaa ggg ccg att atc caa atg tat acc aat gta gac 1844
Leu Ala Gly Pro Lys Gly Pro Ile Ile Gln Met Tyr Thr Asn Val Asp
                490                 495                 500

cag gac ctt gtt ggc tgg caa gcg ccc ccc ggg gcg cgt tcc ttg aca 1892
Gln Asp Leu Val Gly Trp Gln Ala Pro Pro Gly Ala Arg Ser Leu Thr
            505                 510                 515

cca tgc acc tgc ggc agc tcg gac ctt tac ctg gtt acg aga cat gct 1940
Pro Cys Thr Cys Gly Ser Ser Asp Leu Tyr Leu Val Thr Arg His Ala
        520                 525                 530

gac gtc att ccg gtg cgc cgg cga ggt gac ggt agg ggg agc cta ctc 1988
Asp Val Ile Pro Val Arg Arg Arg Gly Asp Gly Arg Gly Ser Leu Leu
    535                 540                 545

tcc ccc aaa ccc atc tcc tac ttg aaa ggc tct tcg ggt ggt ccg ctg 2036
Ser Pro Lys Pro Ile Ser Tyr Leu Lys Gly Ser Ser Gly Gly Pro Leu
550                 555                 560                 565

ctc tgc cct tcg ggg cac gct gtg ggc atc ttt cgg gct gct gtg tgc 2084
Leu Cys Pro Ser Gly His Ala Val Gly Ile Phe Arg Ala Ala Val Cys
                570                 575                 580

acc cgg ggg att gcg aag gct gtg gac ttt gta ccc gtt gag tgt atg 2132
Thr Arg Gly Ile Ala Lys Ala Val Asp Phe Val Pro Val Glu Cys Met
            585                 590                 595

gaa act act atg cgg tct ccg gtc ttc aca gac aac tcg tcc ccc ccg 2180
Glu Thr Thr Met Arg Ser Pro Val Phe Thr Asp Asn Ser Ser Pro Pro
        600                 605                 610

acc gta ccg cag aca ttc caa gtg gcc cat cta cac gct ccc act ggc 2228
Thr Val Pro Gln Thr Phe Gln Val Ala His Leu His Ala Pro Thr Gly
    615                 620                 625

agc ggc aaa agc acc aaa gta ccg gct gca tat gcg gcc caa ggg tat 2276
Ser Gly Lys Ser Thr Lys Val Pro Ala Ala Tyr Ala Ala Gln Gly Tyr
630                 635                 640                 645

aag gta ctc gtc ctg aac ccg tcc gtt gcc gcc acc ctg agt ttt ggg 2324
Lys Val Leu Val Leu Asn Pro Ser Val Ala Ala Thr Leu Ser Phe Gly
                650                 655                 660

gcg tat atg tcc aag gca cat ggt gtc gac cct aac atc aga act ggg 2372
Ala Tyr Met Ser Lys Ala His Gly Val Asp Pro Asn Ile Arg Thr Gly
            665                 670                 675

atg agg acc atc acc aca ggc gct ccc atc acg tac tcc acc tat ggc 2420
Met Arg Thr Ile Thr Thr Gly Ala Pro Ile Thr Tyr Ser Thr Tyr Gly
        680                 685                 690

aag ttc ctt gcc gac ggt ggt tgt tcc ggg ggc gcc tat gac atc ata 2468
Lys Phe Leu Ala Asp Gly Gly Cys Ser Gly Gly Ala Tyr Asp Ile Ile
    695                 700                 705

tta tgt gat gag tgc cac tca act gac tca act act gtt tta ggc atc 2516
Leu Cys Asp Glu Cys His Ser Thr Asp Ser Thr Thr Val Leu Gly Ile
710                 715                 720                 725

ggc aca gtt ctg gac caa gcg gag acg gct gga gcg cga ctc gtc gtg 2564
Gly Thr Val Leu Asp Gln Ala Glu Thr Ala Gly Ala Arg Leu Val Val
                730                 735                 740

ctc gcc acc gct acg cct cca gga tcg gtc acc gtg cca cac ccc aat 2612
Leu Ala Thr Ala Thr Pro Pro Gly Ser Val Thr Val Pro His Pro Asn
            745                 750                 755

atc gag gaa gtg gct ctg tcc aac act gga gag atc ccc ttc tat ggc 2660
Ile Glu Glu Val Ala Leu Ser Asn Thr Gly Glu Ile Pro Phe Tyr Gly
        760                 765                 770

aaa gcc atc cct atc gag gtc atc aag ggg gga agg cat ctc att ttc 2708
Lys Ala Ile Pro Ile Glu Val Ile Lys Gly Gly Arg His Leu Ile Phe
    775                 780                 785

tgt cat tcc aag aag aaa tgc gac gag ctt gct gca aag ttg tca ggt 2756
Cys His Ser Lys Lys Lys Cys Asp Glu Leu Ala Ala Lys Leu Ser Gly
790                 795                 800                 805

ctc gga ctc aat gct gta gtg tat tac cgg ggc ctt gac gtg tcc gtc 2804
Leu Gly Leu Asn Ala Val Val Tyr Tyr Arg Gly Leu Asp Val Ser Val
                810                 815                 820

ata cct acc agc gga gac gtc gtt gtc gtg gca aca gac gct cta atg 2852
Ile Pro Thr Ser Gly Asp Val Val Val Val Ala Thr Asp Ala Leu Met
            825                 830                 835

acg ggc tat acc ggt gac ttt gac tca gtg atc gac tgt aat aca tgt 2900
Thr Gly Tyr Thr Gly Asp Phe Asp Ser Val Ile Asp Cys Asn Thr Cys
        840                 845                 850

gtc act cag aca gtc gac ttc agc ttg gat cct acc ttc acc att gac 2948
Val Thr Gln Thr Val Asp Phe Ser Leu Asp Pro Thr Phe Thr Ile Asp
    855                 860                 865

acg acg acc gta ccc caa gac gcg gta tca cgc tcg cag cgg cga ggt 2996
Thr Thr Thr Val Pro Gln Asp Ala Val Ser Arg Ser Gln Arg Arg Gly
870                 875                 880                 885

agg act ggc agg ggt agg gga ggc atc tac agg ttt gtg act cca gga 3044
Arg Thr Gly Arg Gly Arg Gly Gly Ile Tyr Arg Phe Val Thr Pro Gly
                890                 895                 900

gaa cgg ccc tcg ggc atg ttc gat tct tcg gtc ttg tgt gag tgt tat 3092
Glu Arg Pro Ser Gly Met Phe Asp Ser Ser Val Leu Cys Glu Cys Tyr
            905                 910                 915

gac gcg ggc tgt gct tgg tat gag ctc acg ccc gcc gaa acc acg gtt 3140
Asp Ala Gly Cys Ala Trp Tyr Glu Leu Thr Pro Ala Glu Thr Thr Val
        920                 925                 930

agg ttg cgg gct tac ctt aat aca cca ggg ttg ccc gtc tgt cag gac 3188
Arg Leu Arg Ala Tyr Leu Asn Thr Pro Gly Leu Pro Val Cys Gln Asp
    935                 940                 945

cac ctg gag ttc tgg gag ggt gtc ttc aca ggc ctc acc cac ata gaa 3236
His Leu Glu Phe Trp Glu Gly Val Phe Thr Gly Leu Thr His Ile Glu
950                 955                 960                 965

gct cat ctc ttg tcc cag act aag gat gca gga gac aat tac ccc tac 3284
Ala His Leu Leu Ser Gln Thr Lys Asp Ala Gly Asp Asn Tyr Pro Tyr
                970                 975                 980

ctg gta gcg tac caa gcc acg gtg tgc gcc agg gct cag gcc cca cct 3332
Leu Val Ala Tyr Gln Ala Thr Val Cys Ala Arg Ala Gln Ala Pro Pro
            985                 990                 995

ccg tct tgg gat caa atg tgg aag tgt ctc atg cgg ctt aaa cct 3377
Pro Ser Trp Asp Gln Met Trp Lys Cys Leu Met Arg Leu Lys Pro
        1000                1005                1010

acg ctg cac ggg cca aca ccc ctg ctg tat agg cta gga gcc gtc 3422
Thr Leu His Gly Pro Thr Pro Leu Leu Tyr Arg Leu Gly Ala Val
        1015                1020                1025

cag aat gag gtc acc ctt aca cac ccc ata acc aaa tac atc atc 3467
Gln Asn Glu Val Thr Leu Thr His Pro Ile Thr Lys Tyr Ile Ile
        1030                1035                1040

aca tgc atg tca gct gac ctg gag gtt gtc act agc acc tgg gtg 3512
Thr Cys Met Ser Ala Asp Leu Glu Val Val Thr Ser Thr Trp Val
        1045                1050                1055

cta gta ggc gga gtc ctt gca gct ttg gcc gca tac tgc ctg aca 3557
Leu Val Gly Gly Val Leu Ala Ala Leu Ala Ala Tyr Cys Leu Thr
        1060                1065                1070

aca ggc agt gtg gtc att gtg ggc agg atc atc ttg tcc ggg aag 3602
Thr Gly Ser Val Val Ile Val Gly Arg Ile Ile Leu Ser Gly Lys
        1075                1080                1085

ccg gct gtc atc ccc gac agg gaa gtc ctc tac cag gcg ttc gat 3647
Pro Ala Val Ile Pro Asp Arg Glu Val Leu Tyr Gln Ala Phe Asp
        1090                1095                1100

gaa atg gag gag tgt gcc tca cac ctc cct tac atc gaa cag gga 3692
Glu Met Glu Glu Cys Ala Ser His Leu Pro Tyr Ile Glu Gln Gly
        1105                1110                1115

atg cag ctc gcc gag caa ttc aag cag aaa gcg ctc ggg ctg cta 3737
Met Gln Leu Ala Glu Gln Phe Lys Gln Lys Ala Leu Gly Leu Leu
        1120                1125                1130

caa acg gcc act aag caa gcg gag gct gct gct ccc atg gtg gag 3782
Gln Thr Ala Thr Lys Gln Ala Glu Ala Ala Ala Pro Met Val Glu
        1135                1140                1145

tcc aaa tgg cac gcc ctt gag gct ttc tgg gcg aag cac atg tgg 3827
Ser Lys Trp His Ala Leu Glu Ala Phe Trp Ala Lys His Met Trp
        1150                1155                1160

aac ttc atc agc ggg ata cag tac tta gca ggc ttg tcc act ctg 3872
Asn Phe Ile Ser Gly Ile Gln Tyr Leu Ala Gly Leu Ser Thr Leu
        1165                1170                1175

cct ggg aac ccc gca ata gca tca ctg atg gca ttc aca gcc tct 3917
Pro Gly Asn Pro Ala Ile Ala Ser Leu Met Ala Phe Thr Ala Ser
        1180                1185                1190

gtc acc agc ccg ctt acc acc cag agc acc ctc ttg ttt aac atc 3962
Val Thr Ser Pro Leu Thr Thr Gln Ser Thr Leu Leu Phe Asn Ile
        1195                1200                1205

ttg ggg gga tgg gtg gct gcc caa ctc gct ccc ccc ggt gct gct 4007
Leu Gly Gly Trp Val Ala Ala Gln Leu Ala Pro Pro Gly Ala Ala
        1210                1215                1220

tcg gct ttt gtg ggc gcc gga att gcc ggc gcg gcc gta ggc agc 4052
Ser Ala Phe Val Gly Ala Gly Ile Ala Gly Ala Ala Val Gly Ser
        1225                1230                1235

ata ggc ctt ggg aag gtg ctt gtg gac att ctg gct gga tat ggg 4097
Ile Gly Leu Gly Lys Val Leu Val Asp Ile Leu Ala Gly Tyr Gly
        1240                1245                1250

gca ggg gtg gca ggc gca ctc gtg gct ttt aag atc atg agc ggc 4142
Ala Gly Val Ala Gly Ala Leu Val Ala Phe Lys Ile Met Ser Gly
        1255                1260                1265

gat atg ccc tcc acc gag gac ctg gtt aac ttg ctt cct gcc atc 4187
Asp Met Pro Ser Thr Glu Asp Leu Val Asn Leu Leu Pro Ala Ile
        1270                1275                1280

ctc tct cct ggt gcc ctg gtc gtc ggg gtc gtg tgc gca gca ata 4232
Leu Ser Pro Gly Ala Leu Val Val Gly Val Val Cys Ala Ala Ile
        1285                1290                1295

ctg cgt cgg cac gtg ggc ccg gga gag ggg gct gtg cag tgg atg 4277
Leu Arg Arg His Val Gly Pro Gly Glu Gly Ala Val Gln Trp Met
        1300                1305                1310

aac cgg ctg ata gcg ttc gct tcc cgg ggt aac cac atc tcc ccc 4322
Asn Arg Leu Ile Ala Phe Ala Ser Arg Gly Asn His Ile Ser Pro
        1315                1320                1325

acg cac tat gtg cct gag agc gac gcc gca gcg cgt gtt acc cag 4367
Thr His Tyr Val Pro Glu Ser Asp Ala Ala Ala Arg Val Thr Gln
        1330                1335                1340

att ctt tcc aac ctt acc atc act cag ctg ctg aag agg ctt cac 4412
Ile Leu Ser Asn Leu Thr Ile Thr Gln Leu Leu Lys Arg Leu His
        1345                1350                1355

caa tgg atc aat gag gac tgc tcc acg cca tgc tcc ggc tcg tgg 4457
Gln Trp Ile Asn Glu Asp Cys Ser Thr Pro Cys Ser Gly Ser Trp
        1360                1365                1370

ctt agg gat gtt tgg gac tgg ata tgc acg gtg ttg gct gac ttc 4502
Leu Arg Asp Val Trp Asp Trp Ile Cys Thr Val Leu Ala Asp Phe
        1375                1380                1385

aag acc tgg ctc cag tcc aag ctc ctg ccg cgg ttg ccg gga gtc 4547
Lys Thr Trp Leu Gln Ser Lys Leu Leu Pro Arg Leu Pro Gly Val
        1390                1395                1400

cct ttc ttc tca tgc caa cgc ggg tac aag gga gtt tgg cgg ggg 4592
Pro Phe Phe Ser Cys Gln Arg Gly Tyr Lys Gly Val Trp Arg Gly
        1405                1410                1415

gat ggc atg atg cat acc acc tgc cca tgt gga gca caa atc acc 4637
Asp Gly Met Met His Thr Thr Cys Pro Cys Gly Ala Gln Ile Thr
        1420                1425                1430

gga cat gtc aaa aat ggt tcc atg agg atc gct ggg cct aga acc 4682
Gly His Val Lys Asn Gly Ser Met Arg Ile Ala Gly Pro Arg Thr
        1435                1440                1445

tgc agc aac acg tgg cat ggg acg ttc ccc atc aac gca tac acc 4727
Cys Ser Asn Thr Trp His Gly Thr Phe Pro Ile Asn Ala Tyr Thr
        1450                1455                1460

acg ggc ccc tgc aca ccc tcc ccg gcg ccc aac tat tcc aag gcg 4772
Thr Gly Pro Cys Thr Pro Ser Pro Ala Pro Asn Tyr Ser Lys Ala
        1465                1470                1475

cta tgg cgg gtg gct gct gag gag tac gtg gaa gtt acg cga gtg 4817
Leu Trp Arg Val Ala Ala Glu Glu Tyr Val Glu Val Thr Arg Val
        1480                1485                1490

gga gac ttc cac tac gtg acg ggc atg acc act gac aac ata aaa 4862
Gly Asp Phe His Tyr Val Thr Gly Met Thr Thr Asp Asn Ile Lys
        1495                1500                1505

tgc cca tgc cag gtt ccg gcc ccc gaa ttc ttc aca gaa ctg gat 4907
Cys Pro Cys Gln Val Pro Ala Pro Glu Phe Phe Thr Glu Leu Asp
        1510                1515                1520

gga gtg cgg ttg cac agg tac gct ccg gtg tgc aaa ccc ctc cta 4952
Gly Val Arg Leu His Arg Tyr Ala Pro Val Cys Lys Pro Leu Leu
        1525                1530                1535

cgg gag gag gtt tta ttc cag gtt ggg tgc aac caa tac ctg gtc 4997
Arg Glu Glu Val Leu Phe Gln Val Gly Cys Asn Gln Tyr Leu Val
        1540                1545                1550

ggg tca cag ctt cca tgc gag ccc gaa ccg gac gta gca gtg ctc 5042
Gly Ser Gln Leu Pro Cys Glu Pro Glu Pro Asp Val Ala Val Leu
        1555                1560                1565

act tcc atg ctt gcc gac ccc tcc cac att aca gca gag aca gct 5087
Thr Ser Met Leu Ala Asp Pro Ser His Ile Thr Ala Glu Thr Ala
        1570                1575                1580

aag cgt agg ttg gcc agg ggg tct ccc ccc tcc ttg gcc agc tcg 5132
Lys Arg Arg Leu Ala Arg Gly Ser Pro Pro Ser Leu Ala Ser Ser
        1585                1590                1595

tca gct agc cag ttg tct gca cct tct ttg aag gcg aca tgc aat 5177
Ser Ala Ser Gln Leu Ser Ala Pro Ser Leu Lys Ala Thr Cys Asn
        1600                1605                1610

acc cat cac cgc tcc ccg gac ctt gac ctc atc gag gcc aac ctc 5222
Thr His His Arg Ser Pro Asp Leu Asp Leu Ile Glu Ala Asn Leu
        1615                1620                1625

ctg tgg tgg cag gag aag ggt gga aac atc acc cgt gtg gag tca 5267
Leu Trp Trp Gln Glu Lys Gly Gly Asn Ile Thr Arg Val Glu Ser
        1630                1635                1640

gag aac aag gtg ata atc atg gac tct ttc gat ccg ctt cga gcg 5312
Glu Asn Lys Val Ile Ile Met Asp Ser Phe Asp Pro Leu Arg Ala
        1645                1650                1655

gag gag gat gag agg gaa ata tct gtt gcg gcg gag atc ctg cgg 5357
Glu Glu Asp Glu Arg Glu Ile Ser Val Ala Ala Glu Ile Leu Arg
        1660                1665                1670

caa tcc agg aaa ttc ccc cca gcg ttg ccc gta tgg gca cgc ccg 5402
Gln Ser Arg Lys Phe Pro Pro Ala Leu Pro Val Trp Ala Arg Pro
        1675                1680                1685

gat tat aac cct cca cta cta gag ccc tgg aag gac ccg gac tat 5447
Asp Tyr Asn Pro Pro Leu Leu Glu Pro Trp Lys Asp Pro Asp Tyr
        1690                1695                1700

gtc cct ccg gtg gta cat ggg tgc ccg ctg ccg cct gcc aag act 5492
Val Pro Pro Val Val His Gly Cys Pro Leu Pro Pro Ala Lys Thr
        1705                1710                1715

cct cca ata cca cct cca cgg agg aaa agg acg gtt gtc ctg aca 5537
Pro Pro Ile Pro Pro Pro Arg Arg Lys Arg Thr Val Val Leu Thr
        1720                1725                1730

gag tcc acc gtg tct tct gtt ctg gcg gag ctc act act aag acc 5582
Glu Ser Thr Val Ser Ser Val Leu Ala Glu Leu Thr Thr Lys Thr
        1735                1740                1745

ttc ggc agc tcc gaa tcg tcg gcc gct gat agc ggc atg gcg acc 5627
Phe Gly Ser Ser Glu Ser Ser Ala Ala Asp Ser Gly Met Ala Thr
        1750                1755                1760

gcc cct cct gac cag gcc tcc ggc gac ggc gac aaa gag tcc gac 5672
Ala Pro Pro Asp Gln Ala Ser Gly Asp Gly Asp Lys Glu Ser Asp
        1765                1770                1775

gtt gag tcg tac tcc tcc atg ccc ccc ctt gag gga gag ccg ggg 5717
Val Glu Ser Tyr Ser Ser Met Pro Pro Leu Glu Gly Glu Pro Gly
        1780                1785                1790

gac ccc gat ctc agc gac ggg tct tgg tct acc gtg agc gag gag 5762
Asp Pro Asp Leu Ser Asp Gly Ser Trp Ser Thr Val Ser Glu Glu
        1795                1800                1805

gct ggt gag gac gtc gtc tgc tgt tca atg tcc tat aca tgg aca 5807
Ala Gly Glu Asp Val Val Cys Cys Ser Met Ser Tyr Thr Trp Thr
        1810                1815                1820

ggc gcc ttg atc aca cca tgc gct gcg gag gaa agc aag ctg ccc 5852
Gly Ala Leu Ile Thr Pro Cys Ala Ala Glu Glu Ser Lys Leu Pro
        1825                1830                1835

atc aac gcg ttg agc aac tct ttg ctg cgt cat cac aac atg gtc 5897
Ile Asn Ala Leu Ser Asn Ser Leu Leu Arg His His Asn Met Val
        1840                1845                1850

tat gcc aca aca tct cgc agc gca agc cag cgg cag aag aag gtc 5942
Tyr Ala Thr Thr Ser Arg Ser Ala Ser Gln Arg Gln Lys Lys Val
        1855                1860                1865

acc ttt gac aga ctg cag gtc ctg gat gat cac tac cgg gac gtg 5987
Thr Phe Asp Arg Leu Gln Val Leu Asp Asp His Tyr Arg Asp Val
        1870                1875                1880

ctt aag gag atg aag gcg aag gcg tcc aca gtt aag gct aaa ctt 6032
Leu Lys Glu Met Lys Ala Lys Ala Ser Thr Val Lys Ala Lys Leu
        1885                1890                1895

ctc tct gta gaa gaa gcc tgc aag ctg acg ccc cca cat tcg gcc 6077
Leu Ser Val Glu Glu Ala Cys Lys Leu Thr Pro Pro His Ser Ala
        1900                1905                1910

aaa tct aag ttt ggt tat ggg gca aag gac gtc cgg aac cta tcc 6122
Lys Ser Lys Phe Gly Tyr Gly Ala Lys Asp Val Arg Asn Leu Ser
        1915                1920                1925

agc agg gcc gtt aac cac att cgc tcc gtg tgg aag gac ttg ctg 6167
Ser Arg Ala Val Asn His Ile Arg Ser Val Trp Lys Asp Leu Leu
        1930                1935                1940

gaa gac act gaa aca cca att gac acc acc atc atg gca aaa agt 6212
Glu Asp Thr Glu Thr Pro Ile Asp Thr Thr Ile Met Ala Lys Ser
        1945                1950                1955

gag gtt ttc tgc atc caa cca gag aaa gga ggc cgc aag cca gct 6257
Glu Val Phe Cys Ile Gln Pro Glu Lys Gly Gly Arg Lys Pro Ala
        1960                1965                1970

cgc ctt atc gtg ttc cca gac ctg gga gtc cgt gta tgc gag aaa 6302
Arg Leu Ile Val Phe Pro Asp Leu Gly Val Arg Val Cys Glu Lys
        1975                1980                1985

atg gcc ctc tac gac gtg gtc tcc acc ctt cct cag gcc gtg atg 6347
Met Ala Leu Tyr Asp Val Val Ser Thr Leu Pro Gln Ala Val Met
        1990                1995                2000

ggc tcc tca tat gga ttc caa tac tct cct ggg cag cga gtc gag 6392
Gly Ser Ser Tyr Gly Phe Gln Tyr Ser Pro Gly Gln Arg Val Glu
        2005                2010                2015

ttc ctg gta aat gcc tgg aaa tca aag aaa aac ccc atg ggc ttc 6437
Phe Leu Val Asn Ala Trp Lys Ser Lys Lys Asn Pro Met Gly Phe
        2020                2025                2030

tca tat gac act cgc tgt ttc gac tca acg gtc act gag agt gac 6482
Ser Tyr Asp Thr Arg Cys Phe Asp Ser Thr Val Thr Glu Ser Asp
        2035                2040                2045

atc cgc gtt gag gag tca atc tac caa tgt tgt gac ttg gcc ccc 6527
Ile Arg Val Glu Glu Ser Ile Tyr Gln Cys Cys Asp Leu Ala Pro
        2050                2055                2060

gaa gcc aga cag gcc ata aag tcg ctc aca gag cgg ctc tat atc 6572
Glu Ala Arg Gln Ala Ile Lys Ser Leu Thr Glu Arg Leu Tyr Ile
        2065                2070                2075

ggg ggt ccc ctg act aat tca aaa ggg caa aac tgc ggt tat cgc 6617
Gly Gly Pro Leu Thr Asn Ser Lys Gly Gln Asn Cys Gly Tyr Arg
        2080                2085                2090

cgg tgt cgc gcc agc ggc gtg ctg acg act agc tgc ggt aat acc 6662
Arg Cys Arg Ala Ser Gly Val Leu Thr Thr Ser Cys Gly Asn Thr
        2095                2100                2105

ctc aca tgt tac ttg aag gcc gct gcg gcc tgt cga gct gcg aag 6707
Leu Thr Cys Tyr Leu Lys Ala Ala Ala Ala Cys Arg Ala Ala Lys
        2110                2115                2120

ctc cag gac tgc acg atg ctc gtg aac gga gac gac cta gtc gtt 6752
Leu Gln Asp Cys Thr Met Leu Val Asn Gly Asp Asp Leu Val Val
        2125                2130                2135

atc tgt gag agt gcg gga acc caa gag gat gcg gcg aac cta cga 6797
Ile Cys Glu Ser Ala Gly Thr Gln Glu Asp Ala Ala Asn Leu Arg
        2140                2145                2150

gtc ttc acg gag gct atg act agg tac tct gct ccc cca ggg gac 6842
Val Phe Thr Glu Ala Met Thr Arg Tyr Ser Ala Pro Pro Gly Asp
        2155                2160                2165

tcg cct caa  cca gaa tac gac ttg  gag ttg ata aca tct  tgc tcc  6887
Ser Pro Gln  Pro Glu Tyr Asp Leu  Glu Leu Ile Thr Ser  Cys Ser
        2170                 2175                 2180

tcc aat gtg  tcg gtc gcg cac gat  gcg tct ggc aag agg  gtg tac  6932
Ser Asn Val  Ser Val Ala His Asp  Ala Ser Gly Lys Arg  Val Tyr
        2185                 2190                 2195

tac ctc act  cgt gac ccc acc acc  ccc ctt gca cgg gct  gcg tgg  6977
Tyr Leu Thr  Arg Asp Pro Thr Thr  Pro Leu Ala Arg Ala  Ala Trp
        2200                 2205                 2210

gag aca gct  aga cac act cca gtc  aac tcc tgg cta ggc  aat atc  7022
Glu Thr Ala  Arg His Thr Pro Val  Asn Ser Trp Leu Gly  Asn Ile
        2215                 2220                 2225

atc atg tat  gcg ccc acc tta tgg  gca agg atg att ctg  atg acc  7067
Ile Met Tyr  Ala Pro Thr Leu Trp  Ala Arg Met Ile Leu  Met Thr
        2230                 2235                 2240

cac ttc ttc  tcc atc ctt cta gct  cag gaa caa ctt gga  aaa gcc  7112
His Phe Phe  Ser Ile Leu Leu Ala  Gln Glu Gln Leu Gly  Lys Ala
        2245                 2250                 2255

ctg gat tgc  cag atc tat ggg gcc  tgt tac tcc att gag  cca ctt  7157
Leu Asp Cys  Gln Ile Tyr Gly Ala  Cys Tyr Ser Ile Glu  Pro Leu
        2260                 2265                 2270

gat cta cct  cag atc att gaa cga  ctc cac ggt ctt agc  gca ttt  7202
Asp Leu Pro  Gln Ile Ile Glu Arg  Leu His Gly Leu Ser  Ala Phe
        2275                 2280                 2285

tca ctc cat  agt tac tct cca ggt  gag atc aat agg gtg  gct tca  7247
Ser Leu His  Ser Tyr Ser Pro Gly  Glu Ile Asn Arg Val  Ala Ser
        2290                 2295                 2300

tgc ctc agg aaa ctt ggg gta cca ccc ttg cga gtc tgg aga cat 7292
Cys Leu Arg Lys Leu Gly Val Pro Pro Leu Arg Val Trp Arg His
        2305                2310                2315

cgg gcc aga agt gtc cgc gct aag cta ctg tcc cag ggg ggg agg 7337
Arg Ala Arg Ser Val Arg Ala Lys Leu Leu Ser Gln Gly Gly Arg
        2320                2325                2330

gcc gcc act tgt ggc aag tac ctc ttc aac tgg gca gta aag acc 7382
Ala Ala Thr Cys Gly Lys Tyr Leu Phe Asn Trp Ala Val Lys Thr
        2335                2340                2345

aag ctt aaa ctc act cca atc ccg gct gcg tcc cag ttg gat tta 7427
Lys Leu Lys Leu Thr Pro Ile Pro Ala Ala Ser Gln Leu Asp Leu
        2350                2355                2360

tcc agc tgg ttc gtt gct ggt tac agc ggg gga gac ata tat cac 7472
Ser Ser Trp Phe Val Ala Gly Tyr Ser Gly Gly Asp Ile Tyr His
        2365                2370                2375

agc ctg tct cgt gcc cga ccc cgc tgg ttc atg tgg tgc cta ctc 7517
Ser Leu Ser Arg Ala Arg Pro Arg Trp Phe Met Trp Cys Leu Leu
        2380                2385                2390

cta ctt tct gta ggg gta ggc atc tat cta ctc ccc aac cga tga 7562
Leu Leu Ser Val Gly Val Gly Ile Tyr Leu Leu Pro Asn Arg
        2395                2400                2405

acggggagct aaacactcca ggccaatagg ccatcctgtt ttttttttct tttttttttt 7622

tccttttttt tttttttttt ttttttttcc tttttttttt ttttcttttt tccttttctt 7682

tcctttggtg gctccatctt agccctagtc acggctagct gtgaaaggtc cgtgagccgc 7742

ttgactgcag agagtgctga tactggcctc tctgcagatc atg 7785

<210> 2
<211> 203
<212> PRT
<213> Hepatitis C virus

<400> 2

Met Ser Thr Asn Pro Lys Pro Gln Arg Lys Thr Lys Pro Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Ala Arg Arg Pro Glu Gly Arg Ala Trp Ala Gln Pro Gly
65                  70                  75                  80

Tyr Pro Trp Pro Leu Tyr Gly Asn Glu Gly Leu Gly Trp Ala Gly Trp
                85                  90                  95

Leu Leu Ser Pro Arg Gly Ser Arg Pro Ser Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Ala Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Leu Thr Ile Pro Ala Ser Ala Tyr
            180                 185                 190

Glu Val Arg Asn Val Ser Gly Ile Tyr His Val
        195                 200

<210>  3
<211>  219
<212>  PRT
<213>  Hepatitis C virus

<400>  3

Thr Asn Asp Cys Ser Asn Ser Ser Ile Val Tyr Glu Ala Ala Asp Val
1               5                   10                  15

Ile Met His Thr Pro Gly Cys Val Pro Cys Val Arg Glu Gly Asn Ala
            20                  25                  30

Ser Arg Cys Trp Ala Ala Leu Thr Pro Thr Leu Ala Val Gly Asn Ala
        35                  40                  45

Ser Val Pro Thr Lys Ala Ile Arg Arg His Val Asp Leu Leu Val Gly
    50                  55                  60

Thr Ala Ala Phe Cys Ser Ala Met Tyr Val Gly Asp Leu Cys Gly Tyr
65                  70                  75                  80

Ile Thr Lys Leu Leu Leu Ala Thr Leu Gly Leu Leu Met Val Leu Gln
                85                  90                  95

Ala Ala Ile Ala Arg Val Pro Tyr Phe Val Arg Thr Gln Gly Leu Ile
            100                 105                 110

Arg Val Cys Met Leu Val Arg Lys Val Ala Gly Gly His Tyr Ala Gln
        115                 120                 125

Met Ala Phe Ile Lys Leu Ala Ala Leu Thr Gly Thr Tyr Val Tyr Asp
    130                 135                 140

His Leu Thr Pro Leu Arg Asp Trp Ala His Ala Gly Leu Arg Asp Leu
145                 150                 155                 160

Ala Val Ala Val Glu Pro Val Ile Phe Ser Asp Met Glu Thr Lys Ile
                165                 170                 175

Ile Thr Trp Gly Ala Asp Thr Ala Ala Cys Gly Asp Ile Ile Leu Gly
            180                 185                 190

Leu Pro Val Ser Ala Arg Arg Gly Arg Glu Ile Leu Leu Gly Pro Ala
        195                 200                 205

Asp Ser Leu Glu Gly Arg Gly Trp Arg Leu Leu
    210                 215

<210> 4
<211> 631
<212> PRT
<213> Hepatitis C virus

<400> 4

Ala Pro Ile Thr Ala Tyr Ser Gln Gln Thr Arg Gly Leu Leu Gly Cys
1               5                   10                  15

Ile Ile Thr Ser Leu Thr Gly Arg Asp Lys Asn Gln Val Glu Gly Glu
            20                  25                  30

Val Gln Val Val Ser Thr Ala Thr Gln Ser Phe Leu Ala Thr Cys Val
        35                  40                  45

Asn Gly Val Cys Trp Thr Val Tyr His Gly Ala Gly Ser Lys Thr Leu

50                  55                  60

Ala Gly Pro Lys Gly Pro Ile Ile Gln Met Tyr Thr Asn Val Asp Gln
65                  70                  75                  80

Asp Leu Val Gly Trp Gln Ala Pro Pro Gly Ala Arg Ser Leu Thr Pro
                85                  90                  95

Cys Thr Cys Gly Ser Ser Asp Leu Tyr Leu Val Thr Arg His Ala Asp
            100                 105                 110

Val Ile Pro Val Arg Arg Arg Gly Asp Gly Arg Gly Ser Leu Leu Ser
        115                 120                 125

Pro Lys Pro Ile Ser Tyr Leu Lys Gly Ser Ser Gly Gly Pro Leu Leu
    130                 135                 140

Cys Pro Ser Gly His Ala Val Gly Ile Phe Arg Ala Ala Val Cys Thr
145                 150                 155                 160

Arg Gly Ile Ala Lys Ala Val Asp Phe Val Pro Val Glu Cys Met Glu
                165                 170                 175

Thr Thr Met Arg Ser Pro Val Phe Thr Asp Asn Ser Ser Pro Pro Thr
            180                 185                 190

Val Pro Gln Thr Phe Gln Val Ala His Leu His Ala Pro Thr Gly Ser

195                 200                 205

Gly Lys Ser Thr Lys Val Pro Ala Ala Tyr Ala Ala Gln Gly Tyr Lys
    210                 215                 220

Val Leu Val Leu Asn Pro Ser Val Ala Ala Thr Leu Ser Phe Gly Ala
225                 230                 235                 240

Tyr Met Ser Lys Ala His Gly Val Asp Pro Asn Ile Arg Thr Gly Met
                245                 250                 255

Arg Thr Ile Thr Thr Gly Ala Pro Ile Thr Tyr Ser Thr Tyr Gly Lys
            260                 265                 270

Phe Leu Ala Asp Gly Gly Cys Ser Gly Gly Ala Tyr Asp Ile Ile Leu
        275                 280                 285

Cys Asp Glu Cys His Ser Thr Asp Ser Thr Thr Val Leu Gly Ile Gly
    290                 295                 300

Thr Val Leu Asp Gln Ala Glu Thr Ala Gly Ala Arg Leu Val Val Leu
305                 310                 315                 320

Ala Thr Ala Thr Pro Pro Gly Ser Val Thr Val Pro His Pro Asn Ile
                325                 330                 335

Glu Glu Val Ala Leu Ser Asn Thr Gly Glu Ile Pro Phe Tyr Gly Lys

            340                 345                 350

Ala Ile Pro Ile Glu Val Ile Lys Gly Gly Arg His Leu Ile Phe Cys
        355                 360                 365

His Ser Lys Lys Lys Cys Asp Glu Leu Ala Ala Lys Leu Ser Gly Leu
    370                 375                 380

Gly Leu Asn Ala Val Val Tyr Tyr Arg Gly Leu Asp Val Ser Val Ile
385                 390                 395                 400

Pro Thr Ser Gly Asp Val Val Val Val Ala Thr Asp Ala Leu Met Thr
                405                 410                 415

Gly Tyr Thr Gly Asp Phe Asp Ser Val Ile Asp Cys Asn Thr Cys Val
            420                 425                 430

Thr Gln Thr Val Asp Phe Ser Leu Asp Pro Thr Phe Thr Ile Asp Thr
        435                 440                 445

Thr Thr Val Pro Gln Asp Ala Val Ser Arg Ser Gln Arg Arg Gly Arg
    450                 455                 460

Thr Gly Arg Gly Arg Gly Gly Ile Tyr Arg Phe Val Thr Pro Gly Glu
465                 470                 475                 480

Arg Pro Ser Gly Met Phe Asp Ser Ser Val Leu Cys Glu Cys Tyr Asp

                485                 490                 495

Ala Gly Cys Ala Trp Tyr Glu Leu Thr Pro Ala Glu Thr Thr Val Arg
            500                 505                 510

Leu Arg Ala Tyr Leu Asn Thr Pro Gly Leu Pro Val Cys Gln Asp His
        515                 520                 525

Leu Glu Phe Trp Glu Gly Val Phe Thr Gly Leu Thr His Ile Glu Ala
    530                 535                 540

His Leu Leu Ser Gln Thr Lys Asp Ala Gly Asp Asn Tyr Pro Tyr Leu
545                 550                 555                 560

Val Ala Tyr Gln Ala Thr Val Cys Ala Arg Ala Gln Ala Pro Pro Pro
                565                 570                 575

Ser Trp Asp Gln Met Trp Lys Cys Leu Met Arg Leu Lys Pro Thr Leu
            580                 585                 590

His Gly Pro Thr Pro Leu Leu Tyr Arg Leu Gly Ala Val Gln Asn Glu
        595                 600                 605

Val Thr Leu Thr His Pro Ile Thr Lys Tyr Ile Ile Thr Cys Met Ser
    610                 615                 620

Ala Asp Leu Glu Val Val Thr

625                 630

<210>  5
<211>  54
<212>  PRT
<213>  Hepatitis C virus

<400>  5

Ser Thr Trp Val Leu Val Gly Gly Val Leu Ala Ala Leu Ala Ala Tyr
1               5                   10                  15

Cys Leu Thr Thr Gly Ser Val Val Ile Val Gly Arg Ile Ile Leu Ser
            20                  25                  30

Gly Lys Pro Ala Val Ile Pro Asp Arg Glu Val Leu Tyr Gln Ala Phe
        35                  40                  45

Asp Glu Met Glu Glu Cys
    50

<210>  6
<211>  260
<212>  PRT
<213>  Hepatitis C virus

<400>  6

Ala Ser His Leu Pro Tyr Ile Glu Gln Gly Met Gln Leu Ala Glu Gln
1               5                   10                  15

Phe Lys Gln Lys Ala Leu Gly Leu Leu Gln Thr Ala Thr Lys Gln Ala
            20                  25                  30

Glu Ala Ala Ala Pro Met Val Glu Ser Lys Trp His Ala Leu Glu Ala
        35                  40                  45

Phe Trp Ala Lys His Met Trp Asn Phe Ile Ser Gly Ile Gln Tyr Leu
    50                  55                  60

Ala Gly Leu Ser Thr Leu Pro Gly Asn Pro Ala Ile Ala Ser Leu Met
65                  70                  75                  80

Ala Phe Thr Ala Ser Val Thr Ser Pro Leu Thr Thr Gln Ser Thr Leu
                85                  90                  95

Leu Phe Asn Ile Leu Gly Gly Trp Val Ala Ala Gln Leu Ala Pro Pro
            100                 105                 110

Gly Ala Ala Ser Ala Phe Val Gly Ala Gly Ile Ala Gly Ala Ala Val
        115                 120                 125

Gly Ser Ile Gly Leu Gly Lys Val Leu Val Asp Ile Leu Ala Gly Tyr
    130                 135                 140

Gly Ala Gly Val Ala Gly Ala Leu Val Ala Phe Lys Ile Met Ser Gly
145                 150                 155                 160

Asp Met Pro Ser Thr Glu Asp Leu Val Asn Leu Leu Pro Ala Ile Leu
                165                 170                 175

Ser Pro Gly Ala Leu Val Val Gly Val Val Cys Ala Ala Ile Leu Arg
            180                 185                 190

Arg His Val Gly Pro Gly Glu Gly Ala Val Gln Trp Met Asn Arg Leu
        195                 200                 205

Ile Ala Phe Ala Ser Arg Gly Asn His Ile Ser Pro Thr His Tyr Val
    210                 215                 220

Pro Glu Ser Asp Ala Ala Ala Arg Val Thr Gln Ile Leu Ser Asn Leu
225                 230                 235                 240

Thr Ile Thr Gln Leu Leu Lys Arg Leu His Gln Trp Ile Asn Glu Asp
                245                 250                 255

Cys Ser Thr Pro
            260

<210>  7
<211>  448
<212>  PRT
<213>  Hepatitis C virus

<400>  7

Cys Ser Gly Ser Trp Leu Arg Asp Val Trp Asp Trp Ile Cys Thr Val
1               5                   10                  15

Leu Ala Asp Phe Lys Thr Trp Leu Gln Ser Lys Leu Leu Pro Arg Leu
            20                  25                  30

Pro Gly Val Pro Phe Phe Ser Cys Gln Arg Gly Tyr Lys Gly Val Trp
        35                  40                  45

Arg Gly Asp Gly Met Met His Thr Thr Cys Pro Cys Gly Ala Gln Ile
    50                  55                  60

Thr Gly His Val Lys Asn Gly Ser Met Arg Ile Ala Gly Pro Arg Thr
65                  70                  75                  80

Cys Ser Asn Thr Trp His Gly Thr Phe Pro Ile Asn Ala Tyr Thr Thr
                85                  90                  95

Gly Pro Cys Thr Pro Ser Pro Ala Pro Asn Tyr Ser Lys Ala Leu Trp
            100                 105                 110

Arg Val Ala Ala Glu Glu Tyr Val Glu Val Thr Arg Val Gly Asp Phe
        115                 120                 125

His Tyr Val Thr Gly Met Thr Thr Asp Asn Ile Lys Cys Pro Cys Gln
    130                 135                 140

Val Pro Ala Pro Glu Phe Phe Thr Glu Leu Asp Gly Val Arg Leu His
145                 150                 155                 160

Arg Tyr Ala Pro Val Cys Lys Pro Leu Leu Arg Glu Glu Val Leu Phe
                165                 170                 175

Gln Val Gly Cys Asn Gln Tyr Leu Val Gly Ser Gln Leu Pro Cys Glu
            180                 185                 190

Pro Glu Pro Asp Val Ala Val Leu Thr Ser Met Leu Ala Asp Pro Ser
        195                 200                 205

His Ile Thr Ala Glu Thr Ala Lys Arg Arg Leu Ala Arg Gly Ser Pro
    210                 215                 220

Pro Ser Leu Ala Ser Ser Ser Ala Ser Gln Leu Ser Ala Pro Ser Leu
225                 230                 235                 240

Lys Ala Thr Cys Asn Thr His His Arg Ser Pro Asp Leu Asp Leu Ile
                245                 250                 255

Glu Ala Asn Leu Leu Trp Trp Gln Glu Lys Gly Gly Asn Ile Thr Arg
            260                 265                 270

Val Glu Ser Glu Asn Lys Val Ile Ile Met Asp Ser Phe Asp Pro Leu
        275                 280                 285

Arg Ala Glu Glu Asp Glu Arg Glu Ile Ser Val Ala Ala Glu Ile Leu
    290                 295                 300

Arg Gln Ser Arg Lys Phe Pro Pro Ala Leu Pro Val Trp Ala Arg Pro
305                 310                 315                 320

Asp Tyr Asn Pro Pro Leu Leu Glu Pro Trp Lys Asp Pro Asp Tyr Val
                325                 330                 335

Pro Pro Val Val His Gly Cys Pro Leu Pro Pro Ala Lys Thr Pro Pro
            340                 345                 350

Ile Pro Pro Pro Arg Arg Lys Arg Thr Val Val Leu Thr Glu Ser Thr
        355                 360                 365

Val Ser Ser Val Leu Ala Glu Leu Thr Thr Lys Thr Phe Gly Ser Ser
    370                 375                 380

Glu Ser Ser Ala Ala Asp Ser Gly Met Ala Thr Ala Pro Pro Asp Gln
385                 390                 395                 400

Ala Ser Gly Asp Gly Asp Lys Glu Ser Asp Val Glu Ser Tyr Ser Ser
                405                 410                 415

Met Pro Pro Leu Glu Gly Glu Pro Gly Asp Pro Asp Leu Ser Asp Gly
            420                 425                 430

Ser Trp Ser Thr Val Ser Glu Glu Ala Gly Glu Asp Val Val Cys Cys
        435                 440                 445

<210> 8
<211> 591
<212> PRT
<213> Hepatitis C virus

<400> 8

Ser Met Ser Tyr Thr Trp Thr Gly Ala Leu Ile Thr Pro Cys Ala Ala
1               5                   10                  15

Glu Glu Ser Lys Leu Pro Ile Asn Ala Leu Ser Asn Ser Leu Leu Arg
            20                  25                  30

His His Asn Met Val Tyr Ala Thr Thr Ser Arg Ser Ala Ser Gln Arg
        35                  40                  45

Gln Lys Lys Val Thr Phe Asp Arg Leu Gln Val Leu Asp Asp His Tyr
    50                  55                  60

Arg Asp Val Leu Lys Glu Met Lys Ala Lys Ala Ser Thr Val Lys Ala
65                  70                  75                  80

Lys Leu Leu Ser Val Glu Glu Ala Cys Lys Leu Thr Pro Pro His Ser
                85                  90                  95

Ala Lys Ser Lys Phe Gly Tyr Gly Ala Lys Asp Val Arg Asn Leu Ser

            100                 105                 110

Ser Arg Ala Val Asn His Ile Arg Ser Val Trp Lys Asp Leu Leu Glu
        115                 120                 125

Asp Thr Glu Thr Pro Ile Asp Thr Thr Ile Met Ala Lys Ser Glu Val
    130                 135                 140

Phe Cys Ile Gln Pro Glu Lys Gly Gly Arg Lys Pro Ala Arg Leu Ile
145                 150                 155                 160

Val Phe Pro Asp Leu Gly Val Arg Val Cys Glu Lys Met Ala Leu Tyr
                165                 170                 175

Asp Val Val Ser Thr Leu Pro Gln Ala Val Met Gly Ser Ser Tyr Gly
            180                 185                 190

Phe Gln Tyr Ser Pro Gly Gln Arg Val Glu Phe Leu Val Asn Ala Trp
        195                 200                 205

Lys Ser Lys Lys Asn Pro Met Gly Phe Ser Tyr Asp Thr Arg Cys Phe
    210                 215                 220

Asp Ser Thr Val Thr Glu Ser Asp Ile Arg Val Glu Glu Ser Ile Tyr
225                 230                 235                 240

Gln Cys Cys Asp Leu Ala Pro Glu Ala Arg Gln Ala Ile Lys Ser Leu

                245                 250                 255

Thr Glu Arg Leu Tyr Ile Gly Gly Pro Leu Thr Asn Ser Lys Gly Gln
            260                 265                 270

Asn Cys Gly Tyr Arg Arg Cys Arg Ala Ser Gly Val Leu Thr Thr Ser
        275                 280                 285

Cys Gly Asn Thr Leu Thr Cys Tyr Leu Lys Ala Ala Ala Ala Cys Arg
    290                 295                 300

Ala Ala Lys Leu Gln Asp Cys Thr Met Leu Val Asn Gly Asp Asp Leu
305                 310                 315                 320

Val Val Ile Cys Glu Ser Ala Gly Thr Gln Glu Asp Ala Ala Asn Leu
                325                 330                 335

Arg Val Phe Thr Glu Ala Met Thr Arg Tyr Ser Ala Pro Pro Gly Asp
            340                 345                 350

Ser Pro Gln Pro Glu Tyr Asp Leu Glu Leu Ile Thr Ser Cys Ser Ser
        355                 360                 365

Asn Val Ser Val Ala His Asp Ala Ser Gly Lys Arg Val Tyr Tyr Leu
    370                 375                 380

Thr Arg Asp Pro Thr Thr Pro Leu Ala Arg Ala Ala Trp Glu Thr Ala

385                 390                 395                 400

Arg His Thr Pro Val Asn Ser Trp Leu Gly Asn Ile Ile Met Tyr Ala
                405                 410                 415

Pro Thr Leu Trp Ala Arg Met Ile Leu Met Thr His Phe Phe Ser Ile
            420                 425                 430

Leu Leu Ala Gln Glu Gln Leu Gly Lys Ala Leu Asp Cys Gln Ile Tyr
        435                 440                 445

Gly Ala Cys Tyr Ser Ile Glu Pro Leu Asp Leu Pro Gln Ile Ile Glu
    450                 455                 460

Arg Leu His Gly Leu Ser Ala Phe Ser Leu His Ser Tyr Ser Pro Gly
465                 470                 475                 480

Glu Ile Asn Arg Val Ala Ser Cys Leu Arg Lys Leu Gly Val Pro Pro
                485                 490                 495

Leu Arg Val Trp Arg His Arg Ala Arg Ser Val Arg Ala Lys Leu Leu
            500                 505                 510

Ser Gln Gly Gly Arg Ala Ala Thr Cys Gly Lys Tyr Leu Phe Asn Trp
        515                 520                 525

Ala Val Lys Thr Lys Leu Lys Leu Thr Pro Ile Pro Ala Ala Ser Gln

530                 535                 540

Leu Asp Leu Ser Ser Trp Phe Val Ala Gly Tyr Ser Gly Gly Asp Ile
545                 550                 555                 560

Tyr His Ser Leu Ser Arg Ala Arg Pro Arg Trp Phe Met Trp Cys Leu
                565                 570                 575

Leu Leu Leu Ser Val Gly Val Gly Ile Tyr Leu Leu Pro Asn Arg
            580                 585                 590

<210>  9
<211>  1060
<212>  DNA
<213>  Hepatitis C virus

<220>
<221>  CDS
<222>  (229)..(1059)
<223>

<400>  9
ccaggccccc ccctcccggg agagccatag tggtctgcgg aaccggtgag tacaccggaa      60

ttaccggaaa gactgggtcc tttcttggat aaacccactc tatgtccggt catttgggcg     120

tgcccccgca agactgctag ccgagtagcg ttggggtgcg aagggccttg tggtactgcc     180

tgatagggtg cttgcgagtg ccccgggagg tctcgtagac cgtgcatc atg agc aca      237
                                                     Met Ser Thr
                                                     1

aat cct aaa cct caa aga aaa acc aaa aga agc aca aac cgc cgc cca 285
Asn Pro Lys Pro Gln Arg Lys Thr Lys Arg Ser Thr Asn Arg Arg Pro
    5                   10                  15

cag gac gtc aag ttc ccg ggt ggc ggt cag atc gtt ggc gga gtt tac 333
Gln Asp Val Lys Phe Pro Gly Gly Gly Gln Ile Val Gly Gly Val Tyr
20                  25                  30                  35

ttg ctg ccg cgc agg ggc ccc agg ttg ggt gtg cgc gcg aca agg aag 381
Leu Leu Pro Arg Arg Gly Pro Arg Leu Gly Val Arg Ala Thr Arg Lys
                40                  45                  50

act tcc gag cga tcc cag ccg cgt ggg aga cgc cag ccc atc ccg aaa 429
Thr Ser Glu Arg Ser Gln Pro Arg Gly Arg Arg Gln Pro Ile Pro Lys
            55                  60                  65

gat cgg cgc tcc acc ggc aag tcc tgg gga aag cca gga tat cct tgg 477
Asp Arg Arg Ser Thr Gly Lys Ser Trp Gly Lys Pro Gly Tyr Pro Trp
        70                  75                  80

ccc ctg tat gga aac gag ggt tgc ggc tgg gca ggt tgg ctc ctg tcc 525
Pro Leu Tyr Gly Asn Glu Gly Cys Gly Trp Ala Gly Trp Leu Leu Ser
    85                  90                  95

ccc cgc ggg tct cgt cct act tgg ggc ccc acc gac ccc cgg cac aga 573
Pro Arg Gly Ser Arg Pro Thr Trp Gly Pro Thr Asp Pro Arg His Arg
100                 105                 110                 115

tca cgc aat tgg ggt aaa gtc atc gat acc ctt acg tgt ggt ttt gcc 621
Ser Arg Asn Trp Gly Lys Val Ile Asp Thr Leu Thr Cys Gly Phe Ala
                120                 125                 130

gac ctc atg ggg tac atc cct gtc att ggc gcc ccg gtc gga ggc gtt 669
Asp Leu Met Gly Tyr Ile Pro Val Ile Gly Ala Pro Val Gly Gly Val
            135                 140                 145

gcc aga gcc cta gcg cac ggt gtt agg gtc ctg gaa gac ggg gtg aat 717
Ala Arg Ala Leu Ala His Gly Val Arg Val Leu Glu Asp Gly Val Asn
        150                 155                 160

tac gca aca ggg aat cta ccc ggt tgc tct ttt tct atc ttc ttg ctt 765
Tyr Ala Thr Gly Asn Leu Pro Gly Cys Ser Phe Ser Ile Phe Leu Leu
    165                 170                 175

gcc ctt ctg tcg tgc gtc aca gtg cca gtg tct gca gtg gag gtc agg 813
Ala Leu Leu Ser Cys Val Thr Val Pro Val Ser Ala Val Glu Val Arg
180                 185                 190                 195

aac att agt tct agc tac tat gcc act aac gat tgc tcg gac aac agc 861
Asn Ile Ser Ser Ser Tyr Tyr Ala Thr Asn Asp Cys Ser Asp Asn Ser
                200                 205                 210

atc acc tgg cag cgc ctt gtg ttt gaa gtc aca aaa tgg ttg tta gca 909
Ile Thr Trp Gln Arg Leu Val Phe Glu Val Thr Lys Trp Leu Leu Ala
            215                 220                 225

atc ctg ggg tct gcc cac ctc ctt aaa gcg tcc ctg cta cgg gtg cca 957
Ile Leu Gly Ser Ala His Leu Leu Lys Ala Ser Leu Leu Arg Val Pro
        230                 235                 240

tac ttt gtg agg gct cac gct ctg cta cgg gtg tgt acc ctg gtg agg 1005
Tyr Phe Val Arg Ala His Ala Leu Leu Arg Val Cys Thr Leu Val Arg
    245                 250                 255

cac ctt gca gga gct aag tac atc cag atg ctg ttg atc act gtg ggc 1053
His Leu Ala Gly Ala Lys Tyr Ile Gln Met Leu Leu Ile Thr Val Gly
260                 265                 270                 275

agg cgg a 1060
Arg Arg

<210> 10
<211> 277
<212> PRT
<213> Hepatitis C virus

<400> 10

Met Ser Thr Asn Pro Lys Pro Gln Arg Lys Thr Lys Arg Ser Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Asp Arg Arg Ser Thr Gly Lys Ser Trp Gly Lys Pro Gly
65                  70                  75                  80

Tyr Pro Trp Pro Leu Tyr Gly Asn Glu Gly Cys Gly Trp Ala Gly Trp
                85                  90                  95

Leu Leu Ser Pro Arg Gly Ser Arg Pro Thr Trp Gly Pro Thr Asp Pro
            100                 105                 110

Arg His Arg Ser Arg Asn Trp Gly Lys Val Ile Asp Thr Leu Thr Cys
        115                 120                 125

Gly Phe Ala Asp Leu Met Gly Tyr Ile Pro Val Ile Gly Ala Pro Val
    130                 135                 140

Gly Gly Val Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Val Thr Val Pro Val Ser Ala Val
            180                 185                 190

Glu Val Arg Asn Ile Ser Ser Ser Tyr Tyr Ala Thr Asn Asp Cys Ser
        195                 200                 205

Asp Asn Ser Ile Thr Trp Gln Arg Leu Val Phe Glu Val Thr Lys Trp
    210                 215                 220

Leu Leu Ala Ile Leu Gly Ser Ala His Leu Leu Lys Ala Ser Leu Leu
225                 230                 235                 240

Arg Val Pro Tyr Phe Val Arg Ala His Ala Leu Leu Arg Val Cys Thr
                245                 250                 255

Leu Val Arg His Leu Ala Gly Ala Lys Tyr Ile Gln Met Leu Leu Ile
            260                 265                 270

Thr Val Gly Arg Arg
        275

<210>  11
<211>  1161
<212>  DNA
<213>  Hepatitis C virus

<220>
<221>  CDS
<222>  (229)..(1161)
<223>

<400>  11
ccaggccccc ccctcccggg agagccatag tggtctgcgg aaccggtgag tacaccggaa      60

ttaccggaaa gtctgggtcc tttcttggat aaacccactc tatgtccggt catttgggcg     120

tgcccccgca agactgctag ccgagtagcg ttgggttgcg aaaggccttg tggtactgcc     180

tgatagggtg cttgcgagtg ccccgggagg tctcgtagac cgtgcatc atg agc aca      237
                                                     Met Ser Thr
                                                     1

aat cct aaa cct caa aga aaa acc aaa aga aac aca aac cgc cgc cca      285
Asn Pro Lys Pro Gln Arg Lys Thr Lys Arg Asn Thr Asn Arg Arg Pro
    5                   10                  15

cag gac gtc aag ttc ccg ggt ggc ggt cag atc gtt ggc gga gtt tac      333
Gln Asp Val Lys Phe Pro Gly Gly Gly Gln Ile Val Gly Gly Val Tyr
20                  25                  30                  35

ttg ctg ccg cgc agg ggc ccc agg ttg ggt gtg cgc gcg aca agg aag 381
Leu Leu Pro Arg Arg Gly Pro Arg Leu Gly Val Arg Ala Thr Arg Lys
                40                  45                  50

act tcc gag cga tcc cag ccg cgt ggg aga cgc cag ccc atc ccg aaa 429
Thr Ser Glu Arg Ser Gln Pro Arg Gly Arg Arg Gln Pro Ile Pro Lys
            55                  60                  65

gat cgg cgc tcc acc ggc aag tcc tgg gga aag cca gga tat cct tgg 477
Asp Arg Arg Ser Thr Gly Lys Ser Trp Gly Lys Pro Gly Tyr Pro Trp
        70                  75                  80

ccc ctg tac gga aac gag ggt tgc ggc tgg gca ggt tgg ctc ctg tcc 525
Pro Leu Tyr Gly Asn Glu Gly Cys Gly Trp Ala Gly Trp Leu Leu Ser
    85                  90                  95

ccc cgc ggg tct cgt cct act tgg ggc ccc acc gac ccc cgg cac aga 573
Pro Arg Gly Ser Arg Pro Thr Trp Gly Pro Thr Asp Pro Arg His Arg
100                 105                 110                 115

tca cgc aat ttg ggt aaa gtc atc gat acc ctt acg tgt ggt ttt gcc 621
Ser Arg Asn Leu Gly Lys Val Ile Asp Thr Leu Thr Cys Gly Phe Ala
                120                 125                 130

gac ctc atg ggg tac atc cct gtc att ggc gcc ccg gtc gga ggc gtt 669
Asp Leu Met Gly Tyr Ile Pro Val Ile Gly Ala Pro Val Gly Gly Val
            135                 140                 145

gcc aga gcc cta gcg cac ggt gtt agg gtc ctg gaa gac ggg gtg aat 717
Ala Arg Ala Leu Ala His Gly Val Arg Val Leu Glu Asp Gly Val Asn
        150                 155                 160

tac gca aca ggg aat cta ccc ggt tgc tct ttt tct atc ttc ttg ctt 765
Tyr Ala Thr Gly Asn Leu Pro Gly Cys Ser Phe Ser Ile Phe Leu Leu
    165                 170                 175

gcc ctt ctg tcg tgc gtc aca gtg cca gtg tct gca gtg gag gtc agg 813
Ala Leu Leu Ser Cys Val Thr Val Pro Val Ser Ala Val Glu Val Arg
180                 185                 190                 195

aac att agt tct agc tac tat gcc act gac gat tgc tcg aac aac agc 861
Asn Ile Ser Ser Ser Tyr Tyr Ala Thr Asp Asp Cys Ser Asn Asn Ser
                200                 205                 210

atc acc tgg cag cgc ctt gtg ttt gaa gtc aca aaa tgg ctg tta gca 909
Ile Thr Trp Gln Arg Leu Val Phe Glu Val Thr Lys Trp Leu Leu Ala
            215                 220                 225

atc ctg ggg tct gcc cac ctc ctt aaa gcg tcc ctg cta cgg gtg cca 957
Ile Leu Gly Ser Ala His Leu Leu Lys Ala Ser Leu Leu Arg Val Pro
        230                 235                 240

tac ttt gtg agg gct cac gct ctg cta cgg gtg tgt acc ctg gtg agg 1005
Tyr Phe Val Arg Ala His Ala Leu Leu Arg Val Cys Thr Leu Val Arg
    245                 250                 255

cac ctt gca gga gct aag tac atc cag atg ctg ttg atc act gta ggc 1053
His Leu Ala Gly Ala Lys Tyr Ile Gln Met Leu Leu Ile Thr Val Gly
260                 265                 270                 275

agg tgg acc ggc act tac atc tat gtc cac ctc tcc ccc tta tca act 1101
Arg Trp Thr Gly Thr Tyr Ile Tyr Val His Leu Ser Pro Leu Ser Thr
                280                 285                 290

tgg gca gct cag ggt ttg cgg gac ctg gcg gtc gcc gtg gag cct gtg 1149
Trp Ala Ala Gln Gly Leu Arg Asp Leu Ala Val Ala Val Glu Pro Val
            295                 300                 305

gtg ttc agc cca 1161
Val Phe Ser Pro
        310

<210>  12
<211>  311
<212>  PRT
<213>  Hepatitis C virus

<400>  12

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Asp Arg Arg Ser Thr Gly Lys Ser Trp Gly Lys Pro Gly
65                  70                  75                  80

Tyr Pro Trp Pro Leu Tyr Gly Asn Glu Gly Cys Gly Trp Ala Gly Trp
                85                  90                  95

Leu Leu Ser Pro Arg Gly Ser Arg Pro Thr Trp Gly Pro Thr Asp Pro
            100                 105                 110

Arg His Arg Ser Arg Asn Leu Gly Lys Val Ile Asp Thr Leu Thr Cys
        115                 120                 125

Gly Phe Ala Asp Leu Met Gly Tyr Ile Pro Val Ile Gly Ala Pro Val
    130                 135                 140

Gly Gly Val Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Val Thr Val Pro Val Ser Ala Val
            180                 185                 190

Glu Val Arg Asn Ile Ser Ser Ser Tyr Tyr Ala Thr Asp Asp Cys Ser
        195                 200                 205

Asn Asn Ser Ile Thr Trp Gln Arg Leu Val Phe Glu Val Thr Lys Trp
    210                 215                 220

Leu Leu Ala Ile Leu Gly Ser Ala His Leu Leu Lys Ala Ser Leu Leu
225                 230                 235                 240

Arg Val Pro Tyr Phe Val Arg Ala His Ala Leu Leu Arg Val Cys Thr
                245                 250                 255

Leu Val Arg His Leu Ala Gly Ala Lys Tyr Ile Gln Met Leu Leu Ile
            260                 265                 270

Thr Val Gly Arg Trp Thr Gly Thr Tyr Ile Tyr Val His Leu Ser Pro
        275                 280                 285

Leu Ser Thr Trp Ala Ala Gln Gly Leu Arg Asp Leu Ala Val Ala Val
    290                 295                 300

Glu Pro Val Val Phe Ser Pro
305                 310

<210>  13
<211>  1095
<212>  DNA
<213>  Hepatitis C virus

<220>
<221>  CDS
<222>  (229)..(1095)
<223>

<400>  13
ccaggtcccc ccctcccggg agagccatag tggtctgcgg aaccggtgag tacaccggaa      60

ttgccaggac gaccgggtcc tttcttggat caacccgctc aatgcctgga gatttgggcg     120

tgcccccgcg agactgctag ccgagtagtg ttgggtcgcg aaaggccttg tggtactgcc     180

tgatagggtg cttgcgagtg ccccgggagg tctcgtagac cgtgcatc atg agc aca      237
                                                     Met Ser Thr

                                                     1

aat cct aaa cct caa aga aaa acc aaa cgt aac acc aac cgc cgc cca 285
Asn Pro Lys Pro Gln Arg Lys Thr Lys Arg Asn Thr Asn Arg Arg Pro
    5                   10                  15

cag gac gtc aag ttc ccg ggc ggt ggt cag atc gtt ggt gga gtt tac 333
Gln Asp Val Lys Phe Pro Gly Gly Gly Gln Ile Val Gly Gly Val Tyr
20                  25                  30                  35

ctg ttg ccg cgc agg ggc ccc agg ttg ggt gtg cgc gcg act agg aag 381
Leu Leu Pro Arg Arg Gly Pro Arg Leu Gly Val Arg Ala Thr Arg Lys
                40                  45                  50

act tcc gag cgg tcg caa cct cgt gga agg cga caa cct atc ccc aag 429
Thr Ser Glu Arg Ser Gln Pro Arg Gly Arg Arg Gln Pro Ile Pro Lys
            55                  60                  65

gct cgc cag ccc gag ggt agg gcc tgg gct cag ccc ggg tac cct cgg 477
Ala Arg Gln Pro Glu Gly Arg Ala Trp Ala Gln Pro Gly Tyr Pro Arg
        70                  75                  80

cct agt tgg ggc ccc acg gac ccc cgg cgt agg tcg cgt aat ttg ggt 525
Pro Ser Trp Gly Pro Thr Asp Pro Arg Arg Arg Ser Arg Asn Leu Gly
    85                  90                  95

aag gtc atc gat acc ctt aca tgc ggc ttc gcc gac ctc atg ggg tac 573
Lys Val Ile Asp Thr Leu Thr Cys Gly Phe Ala Asp Leu Met Gly Tyr
100                 105                 110                 115

atc ccg ctc gtc ggc gcc ccc cta ggg ggc gct gcc agg gcc ttg gcg 621
Ile Pro Leu Val Gly Ala Pro Leu Gly Gly Ala Ala Arg Ala Leu Ala
                120                 125                 130

cat ggc gtc cgg gtt ctg gag gac ggc gtg aac tat gca aca ggg aac 669
His Gly Val Arg Val Leu Glu Asp Gly Val Asn Tyr Ala Thr Gly Asn

            135                 140                 145

ctt ccc ggt tgc tct ttc tct atc ttc ctc ttg gct ttg ctg tcc tgt 717
Leu Pro Gly Cys Ser Phe Ser Ile Phe Leu Leu Ala Leu Leu Ser Cys
        150                 155                 160

ttg acc att cca gcc tcc gcc cat gtc ccc cct ctc aac gtc cgg gga 765
Leu Thr Ile Pro Ala Ser Ala His Val Pro Pro Leu Asn Val Arg Gly
    165                 170                 175

ggc cgc gac gcc atc atc ctt ctc aca tgt gcg gtc cac tca gag cta 813
Gly Arg Asp Ala Ile Ile Leu Leu Thr Cys Ala Val His Ser Glu Leu
180                 185                 190                 195

gtt ttt aaa atc acc aaa atc ctg ctt gca ata ctt ggt ccg ctc atg 861
Val Phe Lys Ile Thr Lys Ile Leu Leu Ala Ile Leu Gly Pro Leu Met
                200                 205                 210

gtg ctc cag gct ggt ctc att agg gtg ccg tac ttc gtg cgc gcc caa 909
Val Leu Gln Ala Gly Leu Ile Arg Val Pro Tyr Phe Val Arg Ala Gln
            215                 220                 225

ggg ctt atc cgt gca tgc atg ttg gtg cgg aag atc gct ggg ggt cat 957
Gly Leu Ile Arg Ala Cys Met Leu Val Arg Lys Ile Ala Gly Gly His
        230                 235                 240

tat gtc caa atg gct ctc gtg aag ctg gcc gca ctg acg ggc acg tac 1005
Tyr Val Gln Met Ala Leu Val Lys Leu Ala Ala Leu Thr Gly Thr Tyr
    245                 250                 255

gtc tat gac cat ctt act cca ctg cgg gac tgg gcc cac acg ggc ctg 1053
Val Tyr Asp His Leu Thr Pro Leu Arg Asp Trp Ala His Thr Gly Leu
260                 265                 270                 275

cga gac ctc gcg gtg gcg gtc gag ccc gtc gtc ttc tct gac 1095
Arg Asp Leu Ala Val Ala Val Glu Pro Val Val Phe Ser Asp

280                    285

<210>  14
<211>  289
<212>  PRT
<213>  Hepatitis C virus

<400>  14

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Ala Arg Gln Pro Glu Gly Arg Ala Trp Ala Gln Pro Gly
65                  70                  75                  80

Tyr Pro Arg Pro Ser Trp Gly Pro Thr Asp Pro Arg Arg Arg Ser Arg
                85                  90                  95

Asn Leu Gly Lys Val Ile Asp Thr Leu Thr Cys Gly Phe Ala Asp Leu
            100                 105                 110

Met Gly Tyr Ile Pro Leu Val Gly Ala Pro Leu Gly Gly Ala Ala Arg
        115                 120                 125

Ala Leu Ala His Gly Val Arg Val Leu Glu Asp Gly Val Asn Tyr Ala
    130                 135                 140

Thr Gly Asn Leu Pro Gly Cys Ser Phe Ser Ile Phe Leu Leu Ala Leu
145                 150                 155                 160

Leu Ser Cys Leu Thr Ile Pro Ala Ser Ala His Val Pro Pro Leu Asn
                165                 170                 175

Val Arg Gly Gly Arg Asp Ala Ile Ile Leu Leu Thr Cys Ala Val His
            180                 185                 190

Ser Glu Leu Val Phe Lys Ile Thr Lys Ile Leu Leu Ala Ile Leu Gly
        195                 200                 205

Pro Leu Met Val Leu Gln Ala Gly Leu Ile Arg Val Pro Tyr Phe Val
    210                 215                 220

Arg Ala Gln Gly Leu Ile Arg Ala Cys Met Leu Val Arg Lys Ile Ala
225                 230                 235                 240

Gly Gly His Tyr Val Gln Met Ala Leu Val Lys Leu Ala Ala Leu Thr
                245                 250                 255

Gly Thr Tyr Val Tyr Asp His Leu Thr Pro Leu Arg Asp Trp Ala His
            260                 265                 270

Thr Gly Leu Arg Asp Leu Ala Val Ala Val Glu Pro Val Val Phe Ser
        275                 280                 285

Asp

<210> 15
<211> 788
<212> DNA
<213> Hepatitis C virus

<220>
<221> CDS
<222> (1)..(786)
<223>

<400> 15
ctc ttg gct ctg ctg tct tgt ctg acc atc cta gct tcc gcc tat gaa 48
Leu Leu Ala Leu Leu Ser Cys Leu Thr Ile Leu Ala Ser Ala Tyr Glu
1               5                   10                  15

gtg cgc aac gtg tcc ggg ttg tac cat gtc acg aac gac tgc tcc aac 96
Val Arg Asn Val Ser Gly Leu Tyr His Val Thr Asn Asp Cys Ser Asn
            20                  25                  30

tca agt att gtg tat gag gca gcg gac atg atc atg cat acc ccc ggg 144
Ser Ser Ile Val Tyr Glu Ala Ala Asp Met Ile Met His Thr Pro Gly
        35                  40                  45

tgc gtg ccc tgc gtc cgg gag aac aac cgc tct cgc tgc tgg gta gcg 192
Cys Val Pro Cys Val Arg Glu Asn Asn Arg Ser Arg Cys Trp Val Ala
    50                  55                  60

ctc acc cct acg ctc gcg gcc aga aac agc agc atc ccc act gcg aca 240
Leu Thr Pro Thr Leu Ala Ala Arg Asn Ser Ser Ile Pro Thr Ala Thr
65                  70                  75                  80

ata cga cgc cat gtc gat ttg ctc gtt ggg gca gcc gct ctc tgc tcc 288
Ile Arg Arg His Val Asp Leu Leu Val Gly Ala Ala Ala Leu Cys Ser
                85                  90                  95

gcc atg tat gtg ggg gat ctc tgc gga tct gtc ttc ctc gtg ttc ttc 336
Ala Met Tyr Val Gly Asp Leu Cys Gly Ser Val Phe Leu Val Phe Phe
            100                 105                 110

tgt gct gcc tgg tat atc aag ggt aag ctg gtc ccc ggg gcg gca tat 384
Cys Ala Ala Trp Tyr Ile Lys Gly Lys Leu Val Pro Gly Ala Ala Tyr
        115                 120                 125

gct ttt tat agc gta tgg ccg ctg ctc ctg ctc ttg ctg gcg cta cca 432
Ala Phe Tyr Ser Val Trp Pro Leu Leu Leu Leu Leu Leu Ala Leu Pro
    130                 135                 140

cca cga gcg tac gct atg gac cgg gag atg gct gca tca tgt gga ggc 480
Pro Arg Ala Tyr Ala Met Asp Arg Glu Met Ala Ala Ser Cys Gly Gly
145                 150                 155                 160

ggg gtc ttc ata ggt cta ata atc ttg act ttg tca ccg cac tat aaa 528
Gly Val Phe Ile Gly Leu Ile Ile Leu Thr Leu Ser Pro His Tyr Lys
                165                 170                 175

gca ttc ctc gct agg ctt ata tgg tgg tta caa tat ttt atc acc agg 576
Ala Phe Leu Ala Arg Leu Ile Trp Trp Leu Gln Tyr Phe Ile Thr Arg
            180                 185                 190

acc gag gcg cac ttg caa gtg tgg atc ccc cct ctc aac gtt cgg ggg 624
Thr Glu Ala His Leu Gln Val Trp Ile Pro Pro Leu Asn Val Arg Gly
        195                 200                 205

ggc cgt gat gcc atc atc ctc ctc atg tgc gtg gtc cat cca gag cta 672
Gly Arg Asp Ala Ile Ile Leu Leu Met Cys Val Val His Pro Glu Leu
    210                 215                 220

att ttt gaa atc acc aag atc ttg ctc gcc ata ctg ggt ccg ccc atg 720
Ile Phe Glu Ile Thr Lys Ile Leu Leu Ala Ile Leu Gly Pro Pro Met
225                 230                 235                 240

gtg ctc cag gcc ggc ctg att agg gtg ccg tac ttc gtg cgc gct caa 768
Val Leu Gln Ala Gly Leu Ile Arg Val Pro Tyr Phe Val Arg Ala Gln
                245                 250                 255

ggg ctc att cgt gca tgc at 788
Gly Leu Ile Arg Ala Cys
            260

<210> 16
<211> 262
<212> PRT
<213> Hepatitis C virus

<400> 16

Leu Leu Ala Leu Leu Ser Cys Leu Thr Ile Leu Ala Ser Ala Tyr Glu
1               5                   10                  15

Val Arg Asn Val Ser Gly Leu Tyr His Val Thr Asn Asp Cys Ser Asn
            20                  25                  30

Ser Ser Ile Val Tyr Glu Ala Ala Asp Met Ile Met His Thr Pro Gly
        35                  40                  45

Cys Val Pro Cys Val Arg Glu Asn Asn Arg Ser Arg Cys Trp Val Ala
    50                  55                  60

Leu Thr Pro Thr Leu Ala Ala Arg Asn Ser Ser Ile Pro Thr Ala Thr
65                  70                  75                  80

Ile Arg Arg His Val Asp Leu Leu Val Gly Ala Ala Ala Leu Cys Ser
                85                  90                  95

Ala Met Tyr Val Gly Asp Leu Cys Gly Ser Val Phe Leu Val Phe Phe
            100                 105                 110

Cys Ala Ala Trp Tyr Ile Lys Gly Lys Leu Val Pro Gly Ala Ala Tyr
        115                 120                 125

Ala Phe Tyr Ser Val Trp Pro Leu Leu Leu Leu Leu Leu Ala Leu Pro
    130                 135                 140

Pro Arg Ala Tyr Ala Met Asp Arg Glu Met Ala Ala Ser Cys Gly Gly
145                 150                 155                 160

Gly Val Phe Ile Gly Leu Ile Ile Leu Thr Leu Ser Pro His Tyr Lys
                165                 170                 175

Ala Phe Leu Ala Arg Leu Ile Trp Trp Leu Gln Tyr Phe Ile Thr Arg
            180                 185                 190

Thr Glu Ala His Leu Gln Val Trp Ile Pro Pro Leu Asn Val Arg Gly
        195                 200                 205

Gly Arg Asp Ala Ile Ile Leu Leu Met Cys Val Val His Pro Glu Leu
    210                 215                 220

Ile Phe Glu Ile Thr Lys Ile Leu Leu Ala Ile Leu Gly Pro Pro Met
225                 230                 235                 240

Val Leu Gln Ala Gly Leu Ile Arg Val Pro Tyr Phe Val Arg Ala Gln
                245                 250                 255

Gly Leu Ile Arg Ala Cys
            260

<210>  17
<211>  1504
<212>  DNA
<213>  Hepatitis C virus

<220>
<221>  CDS
<222>  (233)..(1504)
<223>

<400>  17

ccaggtcccc ccctcccggg agagccatag tggtctgcgg aaccggtgag tacaccggaa 60

ttgccaggac gaccgggtcc tttcttggat taacccgctc aatgcctgga gatttgggcg 120

tgcccccgcg agactgctag ccgagtagtg ttgggtcgcg aaaggccttg tggtactgcc 180

tgatagggtg cttgcgagtg ccccgggagg tctcgtagac cgtgcaccat gg atg aat 238
Met Asn
1

acc acc ggg ttc acc aag acg tgc ggg ggc ccc ccg tgt aac atc ggg 286
Thr Thr Gly Phe Thr Lys Thr Cys Gly Gly Pro Pro Cys Asn Ile Gly
        5                   10                  15

ggg gtc ggc aat aac acc ctg acc tgt ccc acg gac tgc ttc cgg aag 334
Gly Val Gly Asn Asn Thr Leu Thr Cys Pro Thr Asp Cys Phe Arg Lys
    20                  25                  30

cac ccc gag gct acg tac acg cga tgc ggt tcg ggg cct tgg ttg aca 382
His Pro Glu Ala Thr Tyr Thr Arg Cys Gly Ser Gly Pro Trp Leu Thr
35                  40                  45                  50

cct agg tgc atg gtt gat tac cca tac agg ctt tgg cac tac ccc tgc 430
Pro Arg Cys Met Val Asp Tyr Pro Tyr Arg Leu Trp His Tyr Pro Cys
                55                  60                  65

act gtc aac ttt acc atc ttc aag gtt agg atg tac gtg ggg ggc gtg 478
Thr Val Asn Phe Thr Ile Phe Lys Val Arg Met Tyr Val Gly Gly Val
            70                  75                  80

gag cac agg ctt agt gct gca tgc aac tgg act cga gga gag cgt tgt 526
Glu His Arg Leu Ser Ala Ala Cys Asn Trp Thr Arg Gly Glu Arg Cys
        85                  90                  95

gac ttg gag gac agg gat aga tca gag ctc agt ccg cta ttg cta tcc 574
Asp Leu Glu Asp Arg Asp Arg Ser Glu Leu Ser Pro Leu Leu Leu Ser

    100                 105                 110

acg aca gag tgg caa ata ctg ccc tgc tcc ttc acc acc cta ccg gct 622
Thr Thr Glu Trp Gln Ile Leu Pro Cys Ser Phe Thr Thr Leu Pro Ala
115                 120                 125                 130

ctg tcc act ggt tta atc cat ctc cat cag aac atc gtg gac gtg caa 670
Leu Ser Thr Gly Leu Ile His Leu His Gln Asn Ile Val Asp Val Gln
                135                 140                 145

tac ctg tac ggt gta ggg tcg gcg gtt gtc tcc ttt gta atc aag tgg 718
Tyr Leu Tyr Gly Val Gly Ser Ala Val Val Ser Phe Val Ile Lys Trp
            150                 155                 160

gag tat gtc gtg ctg ctt ttc ctt ctc ctg gcg gac gcg cgt gtc tgt 766
Glu Tyr Val Val Leu Leu Phe Leu Leu Leu Ala Asp Ala Arg Val Cys
        165                 170                 175

gcc tgc ttg tgg atg atg ctg cta ata gcc cag gct gag gcc gcc tta 814
Ala Cys Leu Trp Met Met Leu Leu Ile Ala Gln Ala Glu Ala Ala Leu
    180                 185                 190

gag aac ctg gtg gtc ctc aat gcg gcg tcc ata gtc gga acg cat ggc 862
Glu Asn Leu Val Val Leu Asn Ala Ala Ser Ile Val Gly Thr His Gly
195                 200                 205                 210

att ctc tcc ctc ctt gta ttc ttc tgt gcc gcc tgg tac atc aag ggc 910
Ile Leu Ser Leu Leu Val Phe Phe Cys Ala Ala Trp Tyr Ile Lys Gly
                215                 220                 225

agg ctg gtc cct ggg gcg gca tat gtt ctt tat ggt gta tgg ccg ctg 958
Arg Leu Val Pro Gly Ala Ala Tyr Val Leu Tyr Gly Val Trp Pro Leu
            230                 235                 240

ctc cgg ctc ctg ctg gcg tta cca caa cga gct tac gcc atg gac cgg 1006
Leu Arg Leu Leu Leu Ala Leu Pro Gln Arg Ala Tyr Ala Met Asp Arg

        245                 250                 255

gag atg gct gca tca tgc gga ggc gcg gtt ttt ata ggc ttg gca ctc 1054
Glu Met Ala Ala Ser Cys Gly Gly Ala Val Phe Ile Gly Leu Ala Leu
    260                 265                 270

ttg acc ttg tca cca tac tac aaa gtg ttc ctc gct agg ctc ata tgg 1102
Leu Thr Leu Ser Pro Tyr Tyr Lys Val Phe Leu Ala Arg Leu Ile Trp
275                 280                 285                 290

tgg tta caa tac ttt atc act aga gcc gag gcg cac ttg caa gtg tgg 1150
Trp Leu Gln Tyr Phe Ile Thr Arg Ala Glu Ala His Leu Gln Val Trp
                295                 300                 305

gtc ccc ccc ctt aac gct cgg gga ggc cgc gat gcc atc atc ctt ctc 1198
Val Pro Pro Leu Asn Ala Arg Gly Gly Arg Asp Ala Ile Ile Leu Leu
            310                 315                 320

aca tgt gca gtc cat cca gag cta atc ttt gac atc acc aaa atc ctg 1246
Thr Cys Ala Val His Pro Glu Leu Ile Phe Asp Ile Thr Lys Ile Leu
        325                 330                 335

ctc gcc ata ttt ggt cca ctc atg gtc ctc cag gct agt ata act gca 1294
Leu Ala Ile Phe Gly Pro Leu Met Val Leu Gln Ala Ser Ile Thr Ala
    340                 345                 350

gtg ccg tac ttt gtg cgc gct caa ggg ctc att cgt gca tgc atg ttg 1342
Val Pro Tyr Phe Val Arg Ala Gln Gly Leu Ile Arg Ala Cys Met Leu
355                 360                 365                 370

gtg cgg aaa gtt gct ggg ggc cat tat gtc caa atg gcc ttc atg aag 1390
Val Arg Lys Val Ala Gly Gly His Tyr Val Gln Met Ala Phe Met Lys
                375                 380                 385

ctg gca gca ctg aca ggt acg tac gtt tac gac cat ctt act ccg ctg 1438
Leu Ala Ala Leu Thr Gly Thr Tyr Val Tyr Asp His Leu Thr Pro Leu

390                    395                    400

cgg gac tgg gcc cac gcg ggc cta cgg gac ctt gcg gtg gca gta gag        1486
Arg Asp Trp Ala His Ala Gly Leu Arg Asp Leu Ala Val Ala Val Glu
        405                    410                    415

ccc gtt gtc ttc tct gac                                                1504
Pro Val Val Phe Ser Asp
    420

<210>  18
<211>  424
<212>  PRT
<213>  Hepatitis C virus

<400>  18

Met Asn Thr Thr Gly Phe Thr Lys Thr Cys Gly Gly Pro Pro Cys Asn
1               5                   10                  15

Ile Gly Gly Val Gly Asn Asn Thr Leu Thr Cys Pro Thr Asp Cys Phe
            20                  25                  30

Arg Lys His Pro Glu Ala Thr Tyr Thr Arg Cys Gly Ser Gly Pro Trp
        35                  40                  45

Leu Thr Pro Arg Cys Met Val Asp Tyr Pro Tyr Arg Leu Trp His Tyr
    50                  55                  60

Pro Cys Thr Val Asn Phe Thr Ile Phe Lys Val Arg Met Tyr Val Gly
65                  70                  75                  80

Gly Val Glu His Arg Leu Ser Ala Ala Cys Asn Trp Thr Arg Gly Glu
                85                  90                  95

Arg Cys Asp Leu Glu Asp Arg Asp Arg Ser Glu Leu Ser Pro Leu Leu
            100                 105                 110

Leu Ser Thr Thr Glu Trp Gln Ile Leu Pro Cys Ser Phe Thr Thr Leu
        115                 120                 125

Pro Ala Leu Ser Thr Gly Leu Ile His Leu His Gln Asn Ile Val Asp
    130                 135                 140

Val Gln Tyr Leu Tyr Gly Val Gly Ser Ala Val Val Ser Phe Val Ile
145                 150                 155                 160

Lys Trp Glu Tyr Val Val Leu Leu Phe Leu Leu Leu Ala Asp Ala Arg
                165                 170                 175

Val Cys Ala Cys Leu Trp Met Met Leu Leu Ile Ala Gln Ala Glu Ala
            180                 185                 190

Ala Leu Glu Asn Leu Val Val Leu Asn Ala Ala Ser Ile Val Gly Thr
        195                 200                 205

His Gly Ile Leu Ser Leu Leu Val Phe Phe Cys Ala Ala Trp Tyr Ile
    210                 215                 220

Lys Gly Arg Leu Val Pro Gly Ala Ala Tyr Val Leu Tyr Gly Val Trp
225                 230                 235                 240

Pro Leu Leu Arg Leu Leu Leu Ala Leu Pro Gln Arg Ala Tyr Ala Met
                245                 250                 255

Asp Arg Glu Met Ala Ala Ser Cys Gly Gly Ala Val Phe Ile Gly Leu
            260                 265                 270

Ala Leu Leu Thr Leu Ser Pro Tyr Tyr Lys Val Phe Leu Ala Arg Leu
        275                 280                 285

Ile Trp Trp Leu Gln Tyr Phe Ile Thr Arg Ala Glu Ala His Leu Gln
    290                 295                 300

Val Trp Val Pro Pro Leu Asn Ala Arg Gly Gly Arg Asp Ala Ile Ile
305                 310                 315                 320

Leu Leu Thr Cys Ala Val His Pro Glu Leu Ile Phe Asp Ile Thr Lys
                325                 330                 335

Ile Leu Leu Ala Ile Phe Gly Pro Leu Met Val Leu Gln Ala Ser Ile
            340                 345                 350

Thr Ala Val Pro Tyr Phe Val Arg Ala Gln Gly Leu Ile Arg Ala Cys
        355                 360                 365

Met Leu Val Arg Lys Val Ala Gly Gly His Tyr Val Gln Met Ala Phe
    370                 375                 380

Met Lys Leu Ala Ala Leu Thr Gly Thr Tyr Val Tyr Asp His Leu Thr
385                 390                 395                 400

Pro Leu Arg Asp Trp Ala His Ala Gly Leu Arg Asp Leu Ala Val Ala
                405                 410                 415

Val Glu Pro Val Val Phe Ser Asp
            420

<210>  19
<211>  245
<212>  DNA
<213>  Hepatitis C virus

<220>
<221>  CDS
<222>  (1)..(243)
<223>

<400>  19
ctc ttg gct ttg ttg tcc tgt ttg acc gtc ccg act tcc gct tat gaa      48
Leu Leu Ala Leu Leu Ser Cys Leu Thr Val Pro Thr Ser Ala Tyr Glu
1               5                   10                  15

gtg cgc aac gtg tcc ggg atg tac caa gtc acg aac gac tgc tcc aac      96
Val Arg Asn Val Ser Gly Met Tyr Gln Val Thr Asn Asp Cys Ser Asn
            20                  25                  30

tca agc att gtg tat gag gca gcg ggc gcg atc atc ttt gag att acc     144
Ser Ser Ile Val Tyr Glu Ala Ala Gly Ala Ile Ile Phe Glu Ile Thr
        35                  40                  45

aaa atc ttg ctc gcc atg ctt ggt ccg ctc atg atg ctc cag gct ggc     192
Lys Ile Leu Leu Ala Met Leu Gly Pro Leu Met Met Leu Gln Ala Gly
    50                  55                  60

cta att aga gtg ccg tac ttc gtg cgc gct caa ggg ctc att cgt gcg     240
Leu Ile Arg Val Pro Tyr Phe Val Arg Ala Gln Gly Leu Ile Arg Ala
65                  70                  75                  80

tgc tc                                                              245
Cys

<210>  20
<211>  81
<212>  PRT
<213>  Hepatitis C virus

<400>  20

Leu Leu Ala Leu Leu Ser Cys Leu Thr Val Pro Thr Ser Ala Tyr Glu
1               5                   10                  15

Val Arg Asn Val Ser Gly Met Tyr Gln Val Thr Asn Asp Cys Ser Asn
            20                  25                  30

Ser Ser Ile Val Tyr Glu Ala Ala Gly Ala Ile Ile Phe Glu Ile Thr
        35                  40                  45

Lys Ile Leu Leu Ala Met Leu Gly Pro Leu Met Met Leu Gln Ala Gly
    50                  55                  60

Leu Ile Arg Val Pro Tyr Phe Val Arg Ala Gln Gly Leu Ile Arg Ala
65                  70                  75                  80

Cys

<210>  21
<211>  1119
<212>  DNA
<213>  Hepatitis C virus

<220>
<221>  CDS
<222>  (229)..(1119)
<223>

<400>  21
ccaggacccc ccctcccggg agagccatag tggtctgcgg aaccggtgag tacaccggaa      60

ttgccaggac gaccgggtcc tttcttggat taacccgctc aatgcccgga gatttgggcg     120

tgcccccgca agactgctag ccgagtagtg ttgggtcgcg aagggccttg tggtactgcc     180

tgatagggtg cttgcgagtg ccccgggagg tctcgtagac cgtgcacc atg agc acg     237
                                                     Met Ser Thr
                                                     1

aat cct aaa ccc caa aga aaa acc aac cga aac acc aac cgc cgt cca     285
Asn Pro Lys Pro Gln Arg Lys Thr Asn Arg Asn Thr Asn Arg Arg Pro

5                   10                   15

cag gac gtt aag ttc ccg ggc ggt ggt cag atc gtc ggt gga gtt tac     333
Gln Asp Val Lys Phe Pro Gly Gly Gly Gln Ile Val Gly Gly Val Tyr
20                  25                  30                  35

ctg ttg ccg cgc agg ggc ccc agg ttg ggt gtg cgc gcg act agg aag     381
Leu Leu Pro Arg Arg Gly Pro Arg Leu Gly Val Arg Ala Thr Arg Lys
                40                  45                  50

act tcc gag cgg tcg caa cct cgt gga agg cga caa cct atc ccc aag     429
Thr Ser Glu Arg Ser Gln Pro Arg Gly Arg Arg Gln Pro Ile Pro Lys
            55                  60                  65

gtt cgc cgg ccc gag ggc agg acc tgg gct cag ccc ggg tat cct tgg     477
Val Arg Arg Pro Glu Gly Arg Thr Trp Ala Gln Pro Gly Tyr Pro Trp
        70                  75                  80

ccc ctc tat ggc aat gag ggc ttg ggg tgg gca gga tgg ctc ctg tca     525
Pro Leu Tyr Gly Asn Glu Gly Leu Gly Trp Ala Gly Trp Leu Leu Ser
    85                  90                  95

ccc cgt ggc tac cgg cct agt tgg ggc ccc acg gac ccc cgg cgt agg     573
Pro Arg Gly Tyr Arg Pro Ser Trp Gly Pro Thr Asp Pro Arg Arg Arg
100                 105                 110                 115

tcg cgt aat ttg ggt aag gtc atc gat acc ctc aca tgc ggc ttc gcc     621
Ser Arg Asn Leu Gly Lys Val Ile Asp Thr Leu Thr Cys Gly Phe Ala
                120                 125                 130

gac ctc atg ggg tat att ccg ctt gtc ggc gcc cct tta gga ggc gct     669
Asp Leu Met Gly Tyr Ile Pro Leu Val Gly Ala Pro Leu Gly Gly Ala
            135                 140                 145

gcc agg gcc ctg gca cat ggt gtc cgg gtt ctg gag gac ggc gtg aat     717
Ala Arg Ala Leu Ala His Gly Val Arg Val Leu Glu Asp Gly Val Asn

        150                 155                 160

tat gca aca ggg aat ttg cct ggt tgc tct ttc tct atc ttc ctc ttg 765
Tyr Ala Thr Gly Asn Leu Pro Gly Cys Ser Phe Ser Ile Phe Leu Leu
    165                 170                 175

gct ctg ctg tcc tgt ttt acc acc cca gct tcc gct tat gga gtg cgc 813
Ala Leu Leu Ser Cys Phe Thr Thr Pro Ala Ser Ala Tyr Gly Val Arg
180                 185                 190                 195

acg tgc gcg gtc cat cca gag cca atc ttt gac atc acc aac ctc ctg 861
Thr Cys Ala Val His Pro Glu Pro Ile Phe Asp Ile Thr Asn Leu Leu
                200                 205                 210

ctc gcc ata ctc ggc ccg ctc atg gtg ctc cag gct ggc ata act aga 909
Leu Ala Ile Leu Gly Pro Leu Met Val Leu Gln Ala Gly Ile Thr Arg
            215                 220                 225

gtg ccg tac ttc gta cgc gct cag ggg ctc att cgt gca tgc atg tta 957
Val Pro Tyr Phe Val Arg Ala Gln Gly Leu Ile Arg Ala Cys Met Leu
        230                 235                 240

gtg agg aaa gcg cct ggg ggt cat tat gtc caa atg gcc ctc atg agg 1005
Val Arg Lys Ala Pro Gly Gly His Tyr Val Gln Met Ala Leu Met Arg
    245                 250                 255

ctg gcc gcg ctg aca ggt acg tac gtg tat gac cat ctc gcc cca ttg 1053
Leu Ala Ala Leu Thr Gly Thr Tyr Val Tyr Asp His Leu Ala Pro Leu
260                 265                 270                 275

cag cat tgg gcc cac gcg ggc cta cga gac ctt gcg gtg gca gta gaa 1101
Gln His Trp Ala His Ala Gly Leu Arg Asp Leu Ala Val Ala Val Glu
                280                 285                 290

ccc gtc atc ttc tct gac 1119
Pro Val Ile Phe Ser Asp

            295

<210> 22
<211> 297
<212> PRT
<213> Hepatitis C virus

<400> 22

Met Ser Thr Asn Pro Lys Pro Gln Arg Lys Thr Asn Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Val Arg Arg Pro Glu Gly Arg Thr Trp Ala Gln Pro Gly
65                  70                  75                  80

Tyr Pro Trp Pro Leu Tyr Gly Asn Glu Gly Leu Gly Trp Ala Gly Trp
                85                  90                  95

Leu Leu Ser Pro Arg Gly Tyr Arg Pro Ser Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Ala Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Phe Thr Thr Pro Ala Ser Ala Tyr
            180                 185                 190

Gly Val Arg Thr Cys Ala Val His Pro Glu Pro Ile Phe Asp Ile Thr
        195                 200                 205

Asn Leu Leu Leu Ala Ile Leu Gly Pro Leu Met Val Leu Gln Ala Gly
    210                 215                 220

Ile Thr Arg Val Pro Tyr Phe Val Arg Ala Gln Gly Leu Ile Arg Ala
225                 230                 235                 240

Cys Met Leu Val Arg Lys Ala Pro Gly Gly His Tyr Val Gln Met Ala
                245                 250                 255

Leu Met Arg Leu Ala Ala Leu Thr Gly Thr Tyr Val Tyr Asp His Leu
            260                 265                 270

Ala Pro Leu Gln His Trp Ala His Ala Gly Leu Arg Asp Leu Ala Val
        275                 280                 285

Ala Val Glu Pro Val Ile Phe Ser Asp
    290                 295

<210> 23
<211> 1143
<212> DNA
<213> Hepatitis C virus

<220>
<221> CDS
<222> (229)..(1143)
<223>

<400> 23
ccaggtcccc ccctcccggg agagccatag tggtctgcgg aaccggtgag tacaccggaa 60

ttgccaggac gaccgggtcc tttcttggat caacccgctc aatgcctgga gatttgggcg 120

tgcccccgcg agactactag ccgagtagtg ttgggtcgcg aaaggccttg tggtactgcc 180

tgatagggtg cttgcgagtg ccccgggagg tctcgtagac cgtgcatc atg agc aca 237
Met Ser Thr
1

aat cct aaa cct caa aga aaa acc aaa cgt aac acc aac cgc cgc cca 285

Asn Pro Lys Pro Gln Arg Lys Thr Lys Arg Asn Thr Asn Arg Arg Pro
    5                   10                  15

cag gac gtc aag ttc ccg ggc ggt ggc cag atc gtt ggt gga gtt tac     333
Gln Asp Val Lys Phe Pro Gly Gly Gly Gln Ile Val Gly Gly Val Tyr
20                  25                  30                  35

ctg ttg ccg cgc agg ggc ccc agg ttg ggt gtg cgc gcg act agg aag     381
Leu Leu Pro Arg Arg Gly Pro Arg Leu Gly Val Arg Ala Thr Arg Lys
                40                  45                  50

act tcc gag cgg tcg caa cct cgt gga agg cga caa cct atc ccc aag     429
Thr Ser Glu Arg Ser Gln Pro Arg Gly Arg Arg Gln Pro Ile Pro Lys
            55                  60                  65

gct cgc cga ccc gag ggc agg gcc tgg gca cag ccc ggg tac cct tgg     477
Ala Arg Arg Pro Glu Gly Arg Ala Trp Ala Gln Pro Gly Tyr Pro Trp
        70                  75                  80

cct ctc tat ggc aat gag ggc ctg ggg tgg gct gga tgg ctc ctg tca     525
Pro Leu Tyr Gly Asn Glu Gly Leu Gly Trp Ala Gly Trp Leu Leu Ser
    85                  90                  95

ccc cgc ggc tcc cgg cct agt tgg ggc ccc acg gac ccc cgg cgt agg     573
Pro Arg Gly Ser Arg Pro Ser Trp Gly Pro Thr Asp Pro Arg Arg Arg
100                 105                 110                 115

tcg cgc aat ttg ggt aag gtc atc gat acc ctc act tgc ggc ttc gcc     621
Ser Arg Asn Leu Gly Lys Val Ile Asp Thr Leu Thr Cys Gly Phe Ala
                120                 125                 130

gac ctc atg ggg tac att ccg ctc gtc ggc gcc ccc cta gga ggt gct     669
Asp Leu Met Gly Tyr Ile Pro Leu Val Gly Ala Pro Leu Gly Gly Ala
            135                 140                 145

gcc agg gcc ctg gcg cat ggc gtc cgg gtt ctg gaa gac ggc gtg aac     717

Ala Arg Ala Leu Ala His Gly Val Arg Val Leu Glu Asp Gly Val Asn
        150                 155                 160

tac gca aca ggg aat ttg ccc ggt tgc tct ttc tct atc ttc ctc ttg 765
Tyr Ala Thr Gly Asn Leu Pro Gly Cys Ser Phe Ser Ile Phe Leu Leu
    165                 170                 175

gct tta ctg tcc tgt ttg acc atc cca gct tcc gct cat caa gtg cgc 813
Ala Leu Leu Ser Cys Leu Thr Ile Pro Ala Ser Ala His Gln Val Arg
180                 185                 190                 195

aac gtg tcc ggg gtg tac cat gtc acg aac aac tgc tcc aac tca aga 861
Asn Val Ser Gly Val Tyr His Val Thr Asn Asn Cys Ser Asn Ser Arg
                200                 205                 210

att gtg gac atc acc aag att ttg ctc gcc ata ttt ggc ccg ctc atg 909
Ile Val Asp Ile Thr Lys Ile Leu Leu Ala Ile Phe Gly Pro Leu Met
            215                 220                 225

gcg ctc cag gct ggt tta act aga gtg ccg tac ttt gta cgc gct cat 957
Ala Leu Gln Ala Gly Leu Thr Arg Val Pro Tyr Phe Val Arg Ala His
        230                 235                 240

ggg ctc atc cgt gtg tgc atg ttg gtg cgg aaa gtc tct ggg ggt cat 1005
Gly Leu Ile Arg Val Cys Met Leu Val Arg Lys Val Ser Gly Gly His
    245                 250                 255

tac gtc cag atg gct ctc atg agg ctg gcc gca ctg acg ggc acg tac 1053
Tyr Val Gln Met Ala Leu Met Arg Leu Ala Ala Leu Thr Gly Thr Tyr
260                 265                 270                 275

gtc tat aac cat ctt act ccg ctg cgg gac tgg gcc cac gtg ggc ctg 1101
Val Tyr Asn His Leu Thr Pro Leu Arg Asp Trp Ala His Val Gly Leu
                280                 285                 290

cga gac ctt gca gtg gca gtt gag cct gtc atc ttc tct gac 1143

Arg Asp Leu Ala Val Ala Val Glu Pro Val Ile Phe Ser Asp
            295                 300                 305

<210>  24
<211>  305
<212>  PRT
<213>  Hepatitis C virus

<400>  24

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Ala Arg Arg Pro Glu Gly Arg Ala Trp Ala Gln Pro Gly
65                  70                  75                  80

Tyr Pro Trp Pro Leu Tyr Gly Asn Glu Gly Leu Gly Trp Ala Gly Trp
                85                  90                  95

Leu Leu Ser Pro Arg Gly Ser Arg Pro Ser Trp Gly Pro Thr Asp Pro

            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Ala Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Leu Thr Ile Pro Ala Ser Ala His
            180                 185                 190

Gln Val Arg Asn Val Ser Gly Val Tyr His Val Thr Asn Asn Cys Ser
        195                 200                 205

Asn Ser Arg Ile Val Asp Ile Thr Lys Ile Leu Leu Ala Ile Phe Gly
    210                 215                 220

Pro Leu Met Ala Leu Gln Ala Gly Leu Thr Arg Val Pro Tyr Phe Val
225                 230                 235                 240

Arg Ala His Gly Leu Ile Arg Val Cys Met Leu Val Arg Lys Val Ser

                245                 250                 255

Gly Gly His Tyr Val Gln Met Ala Leu Met Arg Leu Ala Ala Leu Thr
            260                 265                 270

Gly Thr Tyr Val Tyr Asn His Leu Thr Pro Leu Arg Asp Trp Ala His
        275                 280                 285

Val Gly Leu Arg Asp Leu Ala Val Ala Val Glu Pro Val Ile Phe Ser
    290                 295                 300

Asp
305

<210> 25
<211> 844
<212> DNA
<213> Hepatitis C virus

<220>
<221> CDS
<222> (1)..(843)
<223>

<400> 25
ctt ttg act tta ctg tcc tgt ttg acc atc cca gct tcc gct cat caa 48
Leu Leu Thr Leu Leu Ser Cys Leu Thr Ile Pro Ala Ser Ala His Gln
1               5                   10                  15

gtg cgc aac gtg tcc ggg gtg tac cat gtc atg aac aac tgc tcc aac 96
Val Arg Asn Val Ser Gly Val Tyr His Val Met Asn Asn Cys Ser Asn

            20                  25                  30

tca agt att gtg gac atc acc aag att ttg ctc gcc ata ttt ggc ccg 144
Ser Ser Ile Val Asp Ile Thr Lys Ile Leu Leu Ala Ile Phe Gly Pro
        35                  40                  45

ctc atg gtg ctc cag gct ggt tta act aga gtg ccg tac ttc gta cgc 192
Leu Met Val Leu Gln Ala Gly Leu Thr Arg Val Pro Tyr Phe Val Arg
    50                  55                  60

gct cat ggg ctc atc cgt gtg tgc atg ttg gtg cgg aaa gtc tct ggg 240
Ala His Gly Leu Ile Arg Val Cys Met Leu Val Arg Lys Val Ser Gly
65                  70                  75                  80

ggt cat tac gtc cag atg gct ctc atg agg ctg gcc gca ctg acg ggt 288
Gly His Tyr Val Gln Met Ala Leu Met Arg Leu Ala Ala Leu Thr Gly
                85                  90                  95

acg tac gtc tat aac cat ctt act ccg ctg cgg gac tgg ggc cac gcg 336
Thr Tyr Val Tyr Asn His Leu Thr Pro Leu Arg Asp Trp Gly His Ala
            100                 105                 110

ggc ctg cga gac ctt gca gtg gca gtt gag cct gtc atc ttc tct gac 384
Gly Leu Arg Asp Leu Ala Val Ala Val Glu Pro Val Ile Phe Ser Asp
        115                 120                 125

atg gag acc aag atc atc acc tgg ggg gcg gac acc gcg gcg tgc ggg 432
Met Glu Thr Lys Ile Ile Thr Trp Gly Ala Asp Thr Ala Ala Cys Gly
    130                 135                 140

gac atc atc tca ggt cta ccc gtc tcc gcc cga agg ggg agg gag ata 480
Asp Ile Ile Ser Gly Leu Pro Val Ser Ala Arg Arg Gly Arg Glu Ile
145                 150                 155                 160

tta ctg gga ccg gcc gac agt ttt gga gag cga ggg tgg cga ctc ctt 528
Leu Leu Gly Pro Ala Asp Ser Phe Gly Glu Arg Gly Trp Arg Leu Leu

                165                 170                 175

gcg cct att acg gcc tac tcc caa caa acc cgg ggc ctg ctt ggc tgc 576
Ala Pro Ile Thr Ala Tyr Ser Gln Gln Thr Arg Gly Leu Leu Gly Cys
            180                 185                 190

atc atc act agc ctt aca ggt cgg gac aag aac cag gtt gag ggg gag 624
Ile Ile Thr Ser Leu Thr Gly Arg Asp Lys Asn Gln Val Glu Gly Glu
        195                 200                 205

gtt cag gtg gtt tcc acc gca acg caa tct ttc ccg gcg acc tgc gtc 672
Val Gln Val Val Ser Thr Ala Thr Gln Ser Phe Pro Ala Thr Cys Val
    210                 215                 220

aac ggc gta cgt tgg act gtc tac cat ggt gcc ggc tca aag acc cta 720
Asn Gly Val Arg Trp Thr Val Tyr His Gly Ala Gly Ser Lys Thr Leu
225                 230                 235                 240

gcc ggc cca aag ggc cca gtc acc cag atg tac acc aat gta gac cgg 768
Ala Gly Pro Lys Gly Pro Val Thr Gln Met Tyr Thr Asn Val Asp Arg
                245                 250                 255

gac ctc gtt ggt tgg ccg gcg ccc tct ggg gcg cgc tcc ttg aca cca 816
Asp Leu Val Gly Trp Pro Ala Pro Ser Gly Ala Arg Ser Leu Thr Pro
            260                 265                 270

tgc acc tgt ggc agc tca gac ctc tac c 844
Cys Thr Cys Gly Ser Ser Asp Leu Tyr
        275                 280

<210> 26
<211> 281
<212> PRT
<213> Hepatitis C virus

<400> 26

Leu Leu Thr Leu Leu Ser Cys Leu Thr Ile Pro Ala Ser Ala His Gln
1               5                   10                  15

Val Arg Asn Val Ser Gly Val Tyr His Val Met Asn Asn Cys Ser Asn
            20                  25                  30

Ser Ser Ile Val Asp Ile Thr Lys Ile Leu Leu Ala Ile Phe Gly Pro
        35                  40                  45

Leu Met Val Leu Gln Ala Gly Leu Thr Arg Val Pro Tyr Phe Val Arg
    50                  55                  60

Ala His Gly Leu Ile Arg Val Cys Met Leu Val Arg Lys Val Ser Gly
65                  70                  75                  80

Gly His Tyr Val Gln Met Ala Leu Met Arg Leu Ala Ala Leu Thr Gly
                85                  90                  95

Thr Tyr Val Tyr Asn His Leu Thr Pro Leu Arg Asp Trp Gly His Ala
            100                 105                 110

Gly Leu Arg Asp Leu Ala Val Ala Val Glu Pro Val Ile Phe Ser Asp
        115                 120                 125

Met Glu Thr Lys Ile Ile Thr Trp Gly Ala Asp Thr Ala Ala Cys Gly
    130                 135                 140

Asp Ile Ile Ser Gly Leu Pro Val Ser Ala Arg Arg Gly Arg Glu Ile
145                 150                 155                 160

Leu Leu Gly Pro Ala Asp Ser Phe Gly Glu Arg Gly Trp Arg Leu Leu
                165                 170                 175

Ala Pro Ile Thr Ala Tyr Ser Gln Gln Thr Arg Gly Leu Leu Gly Cys
            180                 185                 190

Ile Ile Thr Ser Leu Thr Gly Arg Asp Lys Asn Gln Val Glu Gly Glu
        195                 200                 205

Val Gln Val Val Ser Thr Ala Thr Gln Ser Phe Pro Ala Thr Cys Val
    210                 215                 220

Asn Gly Val Arg Trp Thr Val Tyr His Gly Ala Gly Ser Lys Thr Leu
225                 230                 235                 240

Ala Gly Pro Lys Gly Pro Val Thr Gln Met Tyr Thr Asn Val Asp Arg
                245                 250                 255

Asp Leu Val Gly Trp Pro Ala Pro Ser Gly Ala Arg Ser Leu Thr Pro
            260                 265                 270

Cys Thr Cys Gly Ser Ser Asp Leu Tyr
        275                 280

<210> 27
<211> 1042
<212> DNA
<213> Hepatitis C virus

<220>
<221> CDS
<222> (229)..(1041)
<223>

<400> 27
ccaggtcccc ccctcccggg agagccatag tggtctgcgg aaccggtgag tacaccggaa 60

ttgccaggac gaccgggtcc tttcttggat caacccgctc aatgcctgga gatttgggcg 120

tgcccccgcg agactactag ccgagtagtg ttgggtcgcg aaaggccttg tggtactgcc 180

tgatagggtg cttgcgagtg ccccgggagg tctcgtagac cgtgcatc atg agc aca 237
Met Ser Thr
1

aat cct aaa cct caa aga aaa acc aaa cgt aac acc aac cgc cgc cca 285
Asn Pro Lys Pro Gln Arg Lys Thr Lys Arg Asn Thr Asn Arg Arg Pro
    5                   10                  15

cag gac gtc aag ttc ccg ggc ggt ggc cag atc gtt ggt gga gtt tac 333
Gln Asp Val Lys Phe Pro Gly Gly Gly Gln Ile Val Gly Gly Val Tyr
20                  25                  30                  35

ctg ttg ccg cgc agg ggc ccc agg ttg ggt gtg cgc gcg act agg aag 381
Leu Leu Pro Arg Arg Gly Pro Arg Leu Gly Val Arg Ala Thr Arg Lys
                40                  45                  50

act tcc gag cgg tcg caa cct cgt gga agg cga caa cct atc ccc aag 429

Thr Ser Glu Arg Ser Gln Pro Arg Gly Arg Arg Gln Pro Ile Pro Lys
            55                  60                  65

gct cgc cga ccc gag ggc agg gcc tgg gca cag ccc ggg tac cct tgg 477
Ala Arg Arg Pro Glu Gly Arg Ala Trp Ala Gln Pro Gly Tyr Pro Trp
        70                  75                  80

cct ctc tat ggc aat gag ggc ctg ggg tgg gca gga tgg ttc ctg tca 525
Pro Leu Tyr Gly Asn Glu Gly Leu Gly Trp Ala Gly Trp Phe Leu Ser
    85                  90                  95

ccc cgc ggc tcc cgg cct agt tgg ggc ccc acg gac ccc cgg cgt agg 573
Pro Arg Gly Ser Arg Pro Ser Trp Gly Pro Thr Asp Pro Arg Arg Arg
100                 105                 110                 115

tcg cgc aat ttg ggt aag gtc atc gat acc ctc act tgc ggc ttc gcc 621
Ser Arg Asn Leu Gly Lys Val Ile Asp Thr Leu Thr Cys Gly Phe Ala
                120                 125                 130

gac ctc atg ggg tac att ccg ctc gtc ggc gcc ccc tta gga ggt gct 669
Asp Leu Met Gly Tyr Ile Pro Leu Val Gly Ala Pro Leu Gly Gly Ala
            135                 140                 145

gcc agg gcc ctg gcg cat ggc gtc cgg gtt ctg gaa gac ggc gtg gac 717
Ala Arg Ala Leu Ala His Gly Val Arg Val Leu Glu Asp Gly Val Asp
        150                 155                 160

tac gca aca ggg aat ttg ccc ggt tgc tct ttc tct atc ttc ctc ttg 765
Tyr Ala Thr Gly Asn Leu Pro Gly Cys Ser Phe Ser Ile Phe Leu Leu
    165                 170                 175

gct tta ctg tcc tgt ttg acc atc cca gct tcc gct cat caa gtg cgc 813
Ala Leu Leu Ser Cys Leu Thr Ile Pro Ala Ser Ala His Gln Val Arg
180                 185                 190                 195

gac gtg tcc ggg gtg tac cat gtc acg aac aac tgc tcc aac tca agt 861

Asp Val Ser Gly Val Tyr His Val Thr Asn Asn Cys Ser Asn Ser Ser
                200                 205                 210

att gtg gtc atc acc aag att ctg ctc gcc ata ttt ggc ccg ctc atg 909
Ile Val Val Ile Thr Lys Ile Leu Leu Ala Ile Phe Gly Pro Leu Met
            215                 220                 225

gcg ctc cag gct ggt tta act aga gtg ccg tac ttc gta cgc gct cat 957
Ala Leu Gln Ala Gly Leu Thr Arg Val Pro Tyr Phe Val Arg Ala His
        230                 235                 240

ggg ctc atc cgt gta tgc atg ttg gtg cgg aaa gtc tct ggg ggt cat 1005
Gly Leu Ile Arg Val Cys Met Leu Val Arg Lys Val Ser Gly Gly His
    245                 250                 255

tac gtc cag atg gct ctc atg agg ctg gcc gca ctg a 1042
Tyr Val Gln Met Ala Leu Met Arg Leu Ala Ala Leu
260                 265                 270

<210> 28
<211> 271
<212> PRT
<213> Hepatitis C virus

<400> 28

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala

        35                  40                  45

Thr Arg Lys Thr Ser Glu Arg Ser Gln Pro Arg Gly Arg Arg Gln Pro
    50                  55                  60

Ile Pro Lys Ala Arg Arg Pro Glu Gly Arg Ala Trp Ala Gln Pro Gly
65                  70                  75                  80

Tyr Pro Trp Pro Leu Tyr Gly Asn Glu Gly Leu Gly Trp Ala Gly Trp
                85                  90                  95

Phe Leu Ser Pro Arg Gly Ser Arg Pro Ser Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Ala Ala Arg Ala Leu Ala His Gly Val Arg Val Leu Glu Asp
145                 150                 155                 160

Gly Val Asp Tyr Ala Thr Gly Asn Leu Pro Gly Cys Ser Phe Ser Ile
                165                 170                 175

Phe Leu Leu Ala Leu Leu Ser Cys Leu Thr Ile Pro Ala Ser Ala His

            180                 185                 190

Gln Val Arg Asp Val Ser Gly Val Tyr His Val Thr Asn Asn Cys Ser
        195                 200                 205

Asn Ser Ser Ile Val Val Ile Thr Lys Ile Leu Leu Ala Ile Phe Gly
    210                 215                 220

Pro Leu Met Ala Leu Gln Ala Gly Leu Thr Arg Val Pro Tyr Phe Val
225                 230                 235                 240

Arg Ala His Gly Leu Ile Arg Val Cys Met Leu Val Arg Lys Val Ser
                245                 250                 255

Gly Gly His Tyr Val Gln Met Ala Leu Met Arg Leu Ala Ala Leu
            260                 265                 270

<210> 29
<211> 232
<212> DNA
<213> Hepatitis C virus

<220>
<221> CDS
<222> (1)..(231)
<223>

<400> 29
ctc ttg gct ttg ctg tcc tgt ttg acc att cca gcc tcc gcc cat gtc 48
Leu Leu Ala Leu Leu Ser Cys Leu Thr Ile Pro Ala Ser Ala His Val

1                5                   10                  15

ccc cct ctc aac gtc cgg gga ggc cgc gac gcc atc atc ctt ctc aca          96
Pro Pro Leu Asn Val Arg Gly Gly Arg Asp Ala Ile Ile Leu Leu Thr
            20                  25                  30

tgt gcg gtc cac tca gag cta gtt ttt aaa atc acc aaa atc ttg ctt          144
Cys Ala Val His Ser Glu Leu Val Phe Lys Ile Thr Lys Ile Leu Leu
        35                  40                  45

gca ata ctt ggt ccg ctc atg gtg ctc cag gct ggt ctc att agg gtg          192
Ala Ile Leu Gly Pro Leu Met Val Leu Gln Ala Gly Leu Ile Arg Val
    50                  55                  60

ccg tac ttc gtg cgc gcc caa ggg ctt atc cgt gca tgc a                    232
Pro Tyr Phe Val Arg Ala Gln Gly Leu Ile Arg Ala Cys
65                  70                  75

<210>  30
<211>  77
<212>  PRT
<213>  Hepatitis C virus

<400>  30

Leu Leu Ala Leu Leu Ser Cys Leu Thr Ile Pro Ala Ser Ala His Val
1               5                   10                  15

Pro Pro Leu Asn Val Arg Gly Gly Arg Asp Ala Ile Ile Leu Leu Thr
            20                  25                  30

Cys Ala Val His Ser Glu Leu Val Phe Lys Ile Thr Lys Ile Leu Leu
        35                  40                  45

Ala Ile Leu Gly Pro Leu Met Val Leu Gln Ala Gly Leu Ile Arg Val
    50                  55                  60

Pro Tyr Phe Val Arg Ala Gln Gly Leu Ile Arg Ala Cys
65                  70                  75

<210>  31
<211>  1823
<212>  DNA
<213>  Hepatitis C virus

<220>
<221>  CDS
<222>  (228)..(1823)
<223>

<400>  31
ccaggtcccc cctcccggga gagccatagt ggtctgcgga accggtgagt acaccggaat      60

tgccaggacg accgggtcct ttcttggatc aacccgctca atgcctggag atttgggcgt     120

gcccccgcga gactgctagc cgagtagtgt tgggtcgcga aaggccttgt ggtactgcct     180

gatagggtgc ttgcgagtgc cccgggaggt ctcgtagacc gtgcatc atg agc aca       236
                                                    Met Ser Thr
                                                    1

aat cct aaa cct caa aga aaa acc aaa cgt aac acc aac cgc cgc cca       284
Asn Pro Lys Pro Gln Arg Lys Thr Lys Arg Asn Thr Asn Arg Arg Pro
    5                   10                  15

cag gac gtc aag ttc ccg ggc ggt ggt cag atc gtt ggt gga gtt tac       332

Gln Asp Val Lys Phe Pro Gly Gly Gly Gln Ile Val Gly Gly Val Tyr
20                  25                  30                  35

ctg ttg ccg cgc agg ggc ccc agg ttg ggt gtg cgc gcg act agg aag     380
Leu Leu Pro Arg Arg Gly Pro Arg Leu Gly Val Arg Ala Thr Arg Lys
                40                  45                  50

act tcc gag cgg tcg caa cct cgt gga agg cga caa cct atc ccc aag     428
Thr Ser Glu Arg Ser Gln Pro Arg Gly Arg Arg Gln Pro Ile Pro Lys
            55                  60                  65

gct cgc cag ccc gag ggt agg gcc tgg gct cag ccc ggg tac cct tgg     476
Ala Arg Gln Pro Glu Gly Arg Ala Trp Ala Gln Pro Gly Tyr Pro Trp
        70                  75                  80

ccc ctc tac ggc aat gag ggc ctg ggg tgg gca gga tgg ctc ctg tca     524
Pro Leu Tyr Gly Asn Glu Gly Leu Gly Trp Ala Gly Trp Leu Leu Ser
    85                  90                  95

ccc cgc ggc tct cgg cct agt tgg ggc ccc aca gac ccc cgg cgt agg     572
Pro Arg Gly Ser Arg Pro Ser Trp Gly Pro Thr Asp Pro Arg Arg Arg
100                 105                 110                 115

tcg cgt aat ttg ggt aag gtc atc gat acc ctt aca tgc ggc ttc gcc     620
Ser Arg Asn Leu Gly Lys Val Ile Asp Thr Leu Thr Cys Gly Phe Ala
                120                 125                 130

gac ctc acg ggg tac atc ccg ctc gtc ggc gcc ccc cta ggg ggc gct     668
Asp Leu Thr Gly Tyr Ile Pro Leu Val Gly Ala Pro Leu Gly Gly Ala
            135                 140                 145

gcc agg gcc ttg gcg cat ggc gtc cgg gtt ctg gag gac ggc gtg aac     716
Ala Arg Ala Leu Ala His Gly Val Arg Val Leu Glu Asp Gly Val Asn
        150                 155                 160

tat gca aca ggg aac ctt ccc ggt tgc tct ttc tct atc ttc ctc ttg     764

Tyr Ala Thr Gly Asn Leu Pro Gly Cys Ser Phe Ser Ile Phe Leu Leu
    165                 170                 175

gct ttg ctg tcc tgt ttg acc att cca gcc tcc gcc cat gtc ccc cct 812
Ala Leu Leu Ser Cys Leu Thr Ile Pro Ala Ser Ala His Val Pro Pro
180                 185                 190                 195

ctc aac gtc cgg gga ggc cgc gac gcc atc atc ctt ctc aca tgt gcg 860
Leu Asn Val Arg Gly Gly Arg Asp Ala Ile Ile Leu Leu Thr Cys Ala
                200                 205                 210

gtc cac tca gag cta gtt ttt aaa atc acc aaa atc ttg ctt gca ata 908
Val His Ser Glu Leu Val Phe Lys Ile Thr Lys Ile Leu Leu Ala Ile
            215                 220                 225

ctt ggt ccg ctc atg gtg ctc cag gct ggt ctc gtt agg gtg ccg tac 956
Leu Gly Pro Leu Met Val Leu Gln Ala Gly Leu Val Arg Val Pro Tyr
        230                 235                 240

ttc gtg cgc gcc caa ggg ctt atc cgt gca tgc atg ttg gtg cgg aag 1004
Phe Val Arg Ala Gln Gly Leu Ile Arg Ala Cys Met Leu Val Arg Lys
    245                 250                 255

atc gct ggg ggt cat tat gtc caa atg gct ttc gtg aag ctg gcc gca 1052
Ile Ala Gly Gly His Tyr Val Gln Met Ala Phe Val Lys Leu Ala Ala
260                 265                 270                 275

ctg acg ggc acg tac gtc tat gac cat ctt act cca ctg cgg gac tgg 1100
Leu Thr Gly Thr Tyr Val Tyr Asp His Leu Thr Pro Leu Arg Asp Trp
                280                 285                 290

gcc cac acg ggc ctg cga gac ctc gcg gtg gcg gtc gag ccc gtc gtc 1148
Ala His Thr Gly Leu Arg Asp Leu Ala Val Ala Val Glu Pro Val Val
            295                 300                 305

ttc tct gac atg ggg acc aag atc atc acc tgg ggg gcg gac acc gcg 1196

Phe Ser Asp Met Gly Thr Lys Ile Ile Thr Trp Gly Ala Asp Thr Ala
        310                 315                 320

gcg tgc ggg gac atc atc tcg ggt ctg ccc gtc tcc gct cgg agg ggg 1244
Ala Cys Gly Asp Ile Ile Ser Gly Leu Pro Val Ser Ala Arg Arg Gly
    325                 330                 335

agg gag ata ctc ctg gga ctg gcc gat agt ttc gga gag cag gga tgg 1292
Arg Glu Ile Leu Leu Gly Leu Ala Asp Ser Phe Gly Glu Gln Gly Trp
340                 345                 350                 355

cga ctc ctt gcg cct atc acg gcc tac tcc caa cag acg cgg ggt tta 1340
Arg Leu Leu Ala Pro Ile Thr Ala Tyr Ser Gln Gln Thr Arg Gly Leu
                360                 365                 370

ctt ggc tgc atc atc act agc ctc aca ggc cgg gac aag aac cag gtc 1388
Leu Gly Cys Ile Ile Thr Ser Leu Thr Gly Arg Asp Lys Asn Gln Val
            375                 380                 385

gag ggg gaa gtc cag gtg gtt tcc acc gca acg cag tct ttc ctc gcg 1436
Glu Gly Glu Val Gln Val Val Ser Thr Ala Thr Gln Ser Phe Leu Ala
        390                 395                 400

aca tgt gta aat ggt gtg tgt tgg act gtc tac cat ggt gcc ggc tca 1484
Thr Cys Val Asn Gly Val Cys Trp Thr Val Tyr His Gly Ala Gly Ser
    405                 410                 415

aag acc tta gcc ggc cct aag ggt ccg atc act caa atg tac acc aat 1532
Lys Thr Leu Ala Gly Pro Lys Gly Pro Ile Thr Gln Met Tyr Thr Asn
420                 425                 430                 435

gtg gac cag gac ctc gtt ggc tgg cag gcg ccc cct ggg gcg cgt tcc 1580
Val Asp Gln Asp Leu Val Gly Trp Gln Ala Pro Pro Gly Ala Arg Ser
                440                 445                 450

atg aca cca tgc acc tgc ggc agc tcg gac ctc tac ctg gtc acg aga 1628

Met Thr Pro Cys Thr Cys Gly Ser Ser Asp Leu Tyr Leu Val Thr Arg
            455                 460                 465

cat gcc gat gtc att ccg gtg cgt cgg cgg ggc gac agc aga ggg agc      1676
His Ala Asp Val Ile Pro Val Arg Arg Arg Gly Asp Ser Arg Gly Ser
        470                 475                 480

cta ctc tcc ccc agg cct gtg tcc tat ttg aag ggc tcc tcg ggt ggt      1724
Leu Leu Ser Pro Arg Pro Val Ser Tyr Leu Lys Gly Ser Ser Gly Gly
    485                 490                 495

cca ctg ctc tgc ccc ttg ggg cac gtc gtg ggc atc ttc cgg gct gct      1772
Pro Leu Leu Cys Pro Leu Gly His Val Val Gly Ile Phe Arg Ala Ala
500                 505                 510                 515

gtg tgc acc cgg ggg gtt gcg aag gcg gtg gac ttt gta ccc gtt gag      1820
Val Cys Thr Arg Gly Val Ala Lys Ala Val Asp Phe Val Pro Val Glu
                520                 525                 530

tct                                                                  1823
Ser

<210>  32
<211>  532
<212>  PRT
<213>  Hepatitis C virus

<400>  32

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly

            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Ala Arg Gln Pro Glu Gly Arg Ala Trp Ala Gln Pro Gly
65                  70                  75                  80

Tyr Pro Trp Pro Leu Tyr Gly Asn Glu Gly Leu Gly Trp Ala Gly Trp
                85                  90                  95

Leu Leu Ser Pro Arg Gly Ser Arg Pro Ser Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Thr Gly Tyr Ile Pro Leu Val Gly Ala Pro Leu
    130                 135                 140

Gly Gly Ala Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile

                165                 170                 175

Phe Leu Leu Ala Leu Leu Ser Cys Leu Thr Ile Pro Ala Ser Ala His
            180                 185                 190

Val Pro Pro Leu Asn Val Arg Gly Gly Arg Asp Ala Ile Ile Leu Leu
        195                 200                 205

Thr Cys Ala Val His Ser Glu Leu Val Phe Lys Ile Thr Lys Ile Leu
    210                 215                 220

Leu Ala Ile Leu Gly Pro Leu Met Val Leu Gln Ala Gly Leu Val Arg
225                 230                 235                 240

Val Pro Tyr Phe Val Arg Ala Gln Gly Leu Ile Arg Ala Cys Met Leu
                245                 250                 255

Val Arg Lys Ile Ala Gly Gly His Tyr Val Gln Met Ala Phe Val Lys
            260                 265                 270

Leu Ala Ala Leu Thr Gly Thr Tyr Val Tyr Asp His Leu Thr Pro Leu
        275                 280                 285

Arg Asp Trp Ala His Thr Gly Leu Arg Asp Leu Ala Val Ala Val Glu
    290                 295                 300

Pro Val Val Phe Ser Asp Met Gly Thr Lys Ile Ile Thr Trp Gly Ala

305                 310                 315                 320

Asp Thr Ala Ala Cys Gly Asp Ile Ile Ser Gly Leu Pro Val Ser Ala
                325                 330                 335

Arg Arg Gly Arg Glu Ile Leu Leu Gly Leu Ala Asp Ser Phe Gly Glu
            340                 345                 350

Gln Gly Trp Arg Leu Leu Ala Pro Ile Thr Ala Tyr Ser Gln Gln Thr
        355                 360                 365

Arg Gly Leu Leu Gly Cys Ile Ile Thr Ser Leu Thr Gly Arg Asp Lys
    370                 375                 380

Asn Gln Val Glu Gly Glu Val Gln Val Val Ser Thr Ala Thr Gln Ser
385                 390                 395                 400

Phe Leu Ala Thr Cys Val Asn Gly Val Cys Trp Thr Val Tyr His Gly
                405                 410                 415

Ala Gly Ser Lys Thr Leu Ala Gly Pro Lys Gly Pro Ile Thr Gln Met
            420                 425                 430

Tyr Thr Asn Val Asp Gln Asp Leu Val Gly Trp Gln Ala Pro Pro Gly
        435                 440                 445

Ala Arg Ser Met Thr Pro Cys Thr Cys Gly Ser Ser Asp Leu Tyr Leu

450                 455                 460

Val Thr Arg His Ala Asp Val Ile Pro Val Arg Arg Arg Gly Asp Ser
465                 470                 475                 480

Arg Gly Ser Leu Leu Ser Pro Arg Pro Val Ser Tyr Leu Lys Gly Ser
                485                 490                 495

Ser Gly Gly Pro Leu Leu Cys Pro Leu Gly His Val Val Gly Ile Phe
            500                 505                 510

Arg Ala Ala Val Cys Thr Arg Gly Val Ala Lys Ala Val Asp Phe Val
        515                 520                 525

Pro Val Glu Ser
    530

<210>  33
<211>  1824
<212>  DNA
<213>  Hepatitis C virus

<220>
<221>  CDS
<222>  (229)..(1824)
<223>

<400>  33
ccaggtcccc ccctcccggg agagccatag tggtctgcgg aaccggtgag tacaccggaa      60

ttgccaggac gaccgggtcc tttcttggat caacccgctc aatgcctgga gatttgggcg 120

tgcccccgcg aggctgctag ccgagtagtg ttgggtcgcg aaaggccttg tggtactgcc 180

tgatagggtg cttgcgagtg ccccgggagg tctcgtagac cgtgcatc atg agc aca 237
Met Ser Thr
1

aat cct aaa cct caa aga aaa acc aaa cgt aac acc aac cgc cgc cca 285
Asn Pro Lys Pro Gln Arg Lys Thr Lys Arg Asn Thr Asn Arg Arg Pro
    5                   10                  15

cag gac gtc aat ttc ccg ggc ggt ggt cag atc gtt ggt gga gtt tac 333
Gln Asp Val Asn Phe Pro Gly Gly Gly Gln Ile Val Gly Gly Val Tyr
20                  25                  30                  35

ctg ttg ccg cgc agg ggc ccc agg ttg ggt gtg cgc gcg act agg aag 381
Leu Leu Pro Arg Arg Gly Pro Arg Leu Gly Val Arg Ala Thr Arg Lys
                40                  45                  50

act tcc gag cgg tcg caa cct cgt gga agg cga caa cct atc ccc aag 429
Thr Ser Glu Arg Ser Gln Pro Arg Gly Arg Arg Gln Pro Ile Pro Lys
            55                  60                  65

gct cgc cag ccc gag ggt agg gcc tgg gct cag ccc ggg tac cct tgg 477
Ala Arg Gln Pro Glu Gly Arg Ala Trp Ala Gln Pro Gly Tyr Pro Trp
        70                  75                  80

ccc ctc tac ggc aat gag ggc ctg ggg tgg aca gga tgg ctc ctg tca 525
Pro Leu Tyr Gly Asn Glu Gly Leu Gly Trp Thr Gly Trp Leu Leu Ser
    85                  90                  95

ccc cgc ggc tct cgg cct agt tgg ggc ccc acg gac ccc cgg cgt agg 573
Pro Arg Gly Ser Arg Pro Ser Trp Gly Pro Thr Asp Pro Arg Arg Arg
100                 105                 110                 115

tcg cgt aat ttg ggt aag gtc atc gat acc ctt aca tgc ggc ttc gcc 621
Ser Arg Asn Leu Gly Lys Val Ile Asp Thr Leu Thr Cys Gly Phe Ala
                120                 125                 130

gac ctc atg ggg tac atc ccg ctc gtc ggc gcc ccc cta ggg ggc gct 669
Asp Leu Met Gly Tyr Ile Pro Leu Val Gly Ala Pro Leu Gly Gly Ala
            135                 140                 145

gcc agg gcc ttg gcg cat ggc gtc cgg gtt ctg gag gac ggc gtg aac 717
Ala Arg Ala Leu Ala His Gly Val Arg Val Leu Glu Asp Gly Val Asn
        150                 155                 160

tat gca aca ggg aac ctt ccc ggt tgc tct ttc tct atc ttc ctc ttg 765
Tyr Ala Thr Gly Asn Leu Pro Gly Cys Ser Phe Ser Ile Phe Leu Leu
    165                 170                 175

gct ttg ctg tcc tgt ttg acc att cca gcc tcc gcc cat gtc ccc cct 813
Ala Leu Leu Ser Cys Leu Thr Ile Pro Ala Ser Ala His Val Pro Pro
180                 185                 190                 195

ctc aac gtc cgg gga ggc cgc gac gcc atc atc ctt ctc aca tgt gcg 861
Leu Asn Val Arg Gly Gly Arg Asp Ala Ile Ile Leu Leu Thr Cys Ala
                200                 205                 210

gtc cac tca gag cta gtt ttt aaa atc acc aaa atc ttg ctt gca ata 909
Val His Ser Glu Leu Val Phe Lys Ile Thr Lys Ile Leu Leu Ala Ile
            215                 220                 225

ctt ggt ccg ctc atg gtg ctc cag gct ggt ctc att agg gtg ccg tac 957
Leu Gly Pro Leu Met Val Leu Gln Ala Gly Leu Ile Arg Val Pro Tyr
        230                 235                 240

ttc gtg cgc gcc caa ggg ctt atc cgt gca tgc atg ttg gtg cgg aag 1005
Phe Val Arg Ala Gln Gly Leu Ile Arg Ala Cys Met Leu Val Arg Lys
    245                 250                 255

atc gct ggg ggt cat tat gtc caa atg gct ttc gtg aag ctg gcc gca 1053
Ile Ala Gly Gly His Tyr Val Gln Met Ala Phe Val Lys Leu Ala Ala
260                 265                 270                 275

ctg acg ggc acg tac gtc tat gac cat ctt act cca ctg cgg gac tgg 1101
Leu Thr Gly Thr Tyr Val Tyr Asp His Leu Thr Pro Leu Arg Asp Trp
                280                 285                 290

gcc cac acg ggc ctg cga gac ctc gcg gtg gcg gtc gag ccc gtc gtc 1149
Ala His Thr Gly Leu Arg Asp Leu Ala Val Ala Val Glu Pro Val Val
            295                 300                 305

ttc tct gac atg gag acc aag atc atc acc tgg ggg gcg gac acc gcg 1197
Phe Ser Asp Met Glu Thr Lys Ile Ile Thr Trp Gly Ala Asp Thr Ala
        310                 315                 320

gcg tgc ggg gac atc atc tcg ggt ctg ccc gtc tcc gct cgg agg ggg 1245
Ala Cys Gly Asp Ile Ile Ser Gly Leu Pro Val Ser Ala Arg Arg Gly
    325                 330                 335

agg gag ata ctc ctg gga cgg gcc gat agt ttc gga gag cag gga tgg 1293
Arg Glu Ile Leu Leu Gly Arg Ala Asp Ser Phe Gly Glu Gln Gly Trp
340                 345                 350                 355

cga ctc ctt gcg cct atc acg gcc tac tcc caa cag acg cgg ggt tta 1341
Arg Leu Leu Ala Pro Ile Thr Ala Tyr Ser Gln Gln Thr Arg Gly Leu
                360                 365                 370

ctt ggc tgc atc atc act agc ctc aca ggc cgg gac aag aac cag gtc 1389
Leu Gly Cys Ile Ile Thr Ser Leu Thr Gly Arg Asp Lys Asn Gln Val
            375                 380                 385

gag ggg gaa gtc cag gtg gtt tcc acc gca acg cag tct ttc ctc gcg 1437
Glu Gly Glu Val Gln Val Val Ser Thr Ala Thr Gln Ser Phe Leu Ala
        390                 395                 400

aca tgt gta aat ggt gtg tgt tgg act gtc tac cat ggt gcc ggc tca 1485
Thr Cys Val Asn Gly Val Cys Trp Thr Val Tyr His Gly Ala Gly Ser
    405                 410                 415

aag acc tta gcc ggc cct aag ggt ccg atc act caa atg tac acc aat 1533
Lys Thr Leu Ala Gly Pro Lys Gly Pro Ile Thr Gln Met Tyr Thr Asn
420                 425                 430                 435

gtg gac cag gac ctc gtt ggt tgg cag gcg ccc cct ggg gcg cgt tcc 1581
Val Asp Gln Asp Leu Val Gly Trp Gln Ala Pro Pro Gly Ala Arg Ser
                440                 445                 450

atg aca cca tgc acc tgc ggc agc tcg gac ctc tac ctg gtc acg aga 1629
Met Thr Pro Cys Thr Cys Gly Ser Ser Asp Leu Tyr Leu Val Thr Arg
            455                 460                 465

cat gcc gat gtc att ccg gtg cgt cgg cgg ggc gac agc aga ggg agc 1677
His Ala Asp Val Ile Pro Val Arg Arg Arg Gly Asp Ser Arg Gly Ser
        470                 475                 480

cta ctc tcc ccc agg cct gtg tcc tat ttg aag ggc tcc tcg ggt ggt 1725
Leu Leu Ser Pro Arg Pro Val Ser Tyr Leu Lys Gly Ser Ser Gly Gly
    485                 490                 495

cca ctg ctc tgc ccc ttg ggg cac gtc gtg ggc atc ttc cgg gct gct 1773
Pro Leu Leu Cys Pro Leu Gly His Val Val Gly Ile Phe Arg Ala Ala
500                 505                 510                 515

gtg tgc acc cgg ggg gtt gcg aag gcg gtg gac ttt gta ccc gtt gag 1821
Val Cys Thr Arg Gly Val Ala Lys Ala Val Asp Phe Val Pro Val Glu
                520                 525                 530

tct 1824
Ser

<210> 34
<211> 532
<212> PRT
<213> Hepatitis C virus

<400> 34

Met Ser Thr Asn Pro Lys Pro Gln Arg Lys Thr Lys Arg Asn Thr Asn
1               5                   10                  15

Arg Arg Pro Gln Asp Val Asn Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Ala Arg Gln Pro Glu Gly Arg Ala Trp Ala Gln Pro Gly
65                  70                  75                  80

Tyr Pro Trp Pro Leu Tyr Gly Asn Glu Gly Leu Gly Trp Thr Gly Trp
                85                  90                  95

Leu Leu Ser Pro Arg Gly Ser Arg Pro Ser Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Ala Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Leu Thr Ile Pro Ala Ser Ala His
            180                 185                 190

Val Pro Pro Leu Asn Val Arg Gly Gly Arg Asp Ala Ile Ile Leu Leu
        195                 200                 205

Thr Cys Ala Val His Ser Glu Leu Val Phe Lys Ile Thr Lys Ile Leu
    210                 215                 220

Leu Ala Ile Leu Gly Pro Leu Met Val Leu Gln Ala Gly Leu Ile Arg
225                 230                 235                 240

Val Pro Tyr Phe Val Arg Ala Gln Gly Leu Ile Arg Ala Cys Met Leu
                245                 250                 255

Val Arg Lys Ile Ala Gly Gly His Tyr Val Gln Met Ala Phe Val Lys
            260                 265                 270

Leu Ala Ala Leu Thr Gly Thr Tyr Val Tyr Asp His Leu Thr Pro Leu
        275                 280                 285

Arg Asp Trp Ala His Thr Gly Leu Arg Asp Leu Ala Val Ala Val Glu
    290                 295                 300

Pro Val Val Phe Ser Asp Met Glu Thr Lys Ile Ile Thr Trp Gly Ala
305                 310                 315                 320

Asp Thr Ala Ala Cys Gly Asp Ile Ile Ser Gly Leu Pro Val Ser Ala
                325                 330                 335

Arg Arg Gly Arg Glu Ile Leu Leu Gly Arg Ala Asp Ser Phe Gly Glu
            340                 345                 350

Gln Gly Trp Arg Leu Leu Ala Pro Ile Thr Ala Tyr Ser Gln Gln Thr
        355                 360                 365

Arg Gly Leu Leu Gly Cys Ile Ile Thr Ser Leu Thr Gly Arg Asp Lys
    370                 375                 380

Asn Gln Val Glu Gly Glu Val Gln Val Val Ser Thr Ala Thr Gln Ser
385                 390                 395                 400

Phe Leu Ala Thr Cys Val Asn Gly Val Cys Trp Thr Val Tyr His Gly
                405                 410                 415

Ala Gly Ser Lys Thr Leu Ala Gly Pro Lys Gly Pro Ile Thr Gln Met
            420                 425                 430

Tyr Thr Asn Val Asp Gln Asp Leu Val Gly Trp Gln Ala Pro Pro Gly
        435                 440                 445

Ala Arg Ser Met Thr Pro Cys Thr Cys Gly Ser Ser Asp Leu Tyr Leu
    450                 455                 460

Val Thr Arg His Ala Asp Val Ile Pro Val Arg Arg Arg Gly Asp Ser
465                 470                 475                 480

Arg Gly Ser Leu Leu Ser Pro Arg Pro Val Ser Tyr Leu Lys Gly Ser
                485                 490                 495

Ser Gly Gly Pro Leu Leu Cys Pro Leu Gly His Val Val Gly Ile Phe
            500                 505                 510

Arg Ala Ala Val Cys Thr Arg Gly Val Ala Lys Ala Val Asp Phe Val
        515                 520                 525

Pro Val Glu Ser
    530

<210> 35
<211> 1115
<212> DNA
<213> Hepatitis C virus

<220>
<221> CDS
<222> (229)..(1113)
<223>

<400> 35
   ccaggacccc ccctcccggg agagccatag tggtctgcgg aaccggtgag tacaccggaa 60
   ttgccaggac gaccgggtcc tttcttggat caacccgctc aatgcctgga gatttgggcg 120
   tgcccccgcg agactgctag ccgagtagtg ttgggtcgcg aaaggccttg tggtactgcc 180
   tgatagggtg cttgcgagtg ccccgggagg tctcgtagac cgtgcacc atg agc acg 237
   Met Ser Thr
   1
   aat cct aaa cct caa aaa aaa ccc aaa tgt aac acc aac cgc cgc cca 285
   Asn Pro Lys Pro Gln Lys Lys Pro Lys Cys Asn Thr Asn Arg Arg Pro
   5 10 15
   cag gac gtc aag ttc ccg ggc ggt ggt cag atc gtt ggt gga gtt tac 333
   Gln Asp Val Lys Phe Pro Gly Gly Gly Gln Ile Val Gly Gly Val Tyr
   20 25 30 35
   ctg ttg ccg cgc agg ggc ccc agg ttg ggt gtg cgc gcg act agg aag 381
   Leu Leu Pro Arg Arg Gly Pro Arg Leu Gly Val Arg Ala Thr Arg Lys
   40 45 50
   act tcc gag cgg tcg caa cct cgt gga agg cga caa cct atc ccc aag 429
   Thr Ser Glu Arg Ser Gln Pro Arg Gly Arg Arg Gln Pro Ile Pro Lys
   55 60 65
   gct cgc cgg ccc gag ggt agg gcc tgg gct cag ccc ggg tac cct tgg 477
   Ala Arg Arg Pro Glu Gly Arg Ala Trp Ala Gln Pro Gly Tyr Pro Trp
   70 75 80
   ccc ctc tat ggc gat gag ggc cta ggg tgg gca gga tgg ctc ctg tca 525
   Pro Leu Tyr Gly Asp Glu Gly Leu Gly Trp Ala Gly Trp Leu Leu Ser
   85 90 95
   ccc cgc ggc tcc cgg cct agt tgg ggc ccc act gac ccc cgg cgt agg 573
   Pro Arg Gly Ser Arg Pro Ser Trp Gly Pro Thr Asp Pro Arg Arg Arg
   100 105 110 115
   tcg cgt aat ctg ggt aag gtc atc gat acc ctc aca tgc ggc ttc gcc 621
   Ser Arg Asn Leu Gly Lys Val Ile Asp Thr Leu Thr Cys Gly Phe Ala
   120 125 130
   gac ctc atg ggg tac att ccg ctc gtc ggc gcc ccc tta gga ggc gtt 669
   Asp Leu Met Gly Tyr Ile Pro Leu Val Gly Ala Pro Leu Gly Gly Val
   135 140 145
   gcc agg gcc ctg gcg cat ggc gtc cgg gtt ctg gaa gac agc gtg aac 717
   Ala Arg Ala Leu Ala His Gly Val Arg Val Leu Glu Asp Ser Val Asn
   150 155 160
   tac gca aca ggg aat ctg ccc ggt tgc tct ttc tct atc ttc ctc tta 765
   Tyr Ala Thr Gly Asn Leu Pro Gly Cys Ser Phe Ser Ile Phe Leu Leu
   165 170 175
   gct ttg ctg tcc tgc ttg act gtc ccg gct tcc gct tgc aaa act ccc 813
   Ala Leu Leu Ser Cys Leu Thr Val Pro Ala Ser Ala Cys Lys Thr Pro
   180 185 190 195
   acg ctc gcg gcc agg gag cta aac ctt gga atc gcc aca atc ttg ctc 861
   Thr Leu Ala Ala Arg Glu Leu Asn Leu Gly Ile Ala Thr Ile Leu Leu
   200 205 210
   gcc ata ttt ggt ccg ctc gtg gcg ctc cag act ggc cta ttt agg gtg 909
   Ala Ile Phe Gly Pro Leu Val Ala Leu Gln Thr Gly Leu Phe Arg Val
   215 220 225
   ccg tac ttc gtg cgc gcc caa ggg ctc atc cgt gcg tgc atg ttg gtg 957
   Pro Tyr Phe Val Arg Ala Gln Gly Leu Ile Arg Ala Cys Met Leu Val
   230 235 240
   cgg aaa gtc tct ggg ggt cat cat gtc caa atg gct ctt gtg agg cta 1005
   Arg Lys Val Ser Gly Gly His His Val Gln Met Ala Leu Val Arg Leu
   245 250 255
   gct gct cta acg ggc acg tac gtt tat gac cat ctt act ccg ctg cgg 1053
   Ala Ala Leu Thr Gly Thr Tyr Val Tyr Asp His Leu Thr Pro Leu Arg
   260 265 270 275
   gac tgg ccc acg cgg gcc tgc gag atc ttg cgg tgg ctg ttg agc ccg 1101
   Asp Trp Pro Thr Arg Ala Cys Glu Ile Leu Arg Trp Leu Leu Ser Pro
   280 285 290
   tca tct tct ctg ac 1115
   Ser Ser Ser Leu
   295

<210> 36
<211> 295
<212> PRT
<213> Hepatitis C virus

<400> 36

Met Ser Thr Asn Pro Lys Pro Gln Lys Lys Pro Lys Cys Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Ala Arg Arg Pro Glu Gly Arg Ala Trp Ala Gln Pro Gly
65                  70                  75                  80

Tyr Pro Trp Pro Leu Tyr Gly Asp Glu Gly Leu Gly Trp Ala Gly Trp
                85                  90                  95

Leu Leu Ser Pro Arg Gly Ser Arg Pro Ser Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Val Ala Arg Ala Leu Ala His Gly Val Arg Val Leu Glu Asp
145                 150                 155                 160

Ser Val Asn Tyr Ala Thr Gly Asn Leu Pro Gly Cys Ser Phe Ser Ile
                165                 170                 175

Phe Leu Leu Ala Leu Leu Ser Cys Leu Thr Val Pro Ala Ser Ala Cys
            180                 185                 190

Lys Thr Pro Thr Leu Ala Ala Arg Glu Leu Asn Leu Gly Ile Ala Thr
        195                 200                 205

Ile Leu Leu Ala Ile Phe Gly Pro Leu Val Ala Leu Gln Thr Gly Leu
    210                 215                 220

Phe Arg Val Pro Tyr Phe Val Arg Ala Gln Gly Leu Ile Arg Ala Cys
225                 230                 235                 240

Met Leu Val Arg Lys Val Ser Gly Gly His His Val Gln Met Ala Leu
                245                 250                 255

Val Arg Leu Ala Ala Leu Thr Gly Thr Tyr Val Tyr Asp His Leu Thr
            260                 265                 270

Pro Leu Arg Asp Trp Pro Thr Arg Ala Cys Glu Ile Leu Arg Trp Leu
        275                 280                 285

Leu Ser Pro Ser Ser Ser Leu
    290                 295

<210> 37
<211> 817
<212> DNA
<213> Hepatitis C virus

<220>
<221> CDS
<222> (1)..(816)
<223>

<400> 37
ctc tta gct ttg ctg tcc tgc ttg act gtc cca gct tcc gct tgc gaa 48
Leu Leu Ala Leu Leu Ser Cys Leu Thr Val Pro Ala Ser Ala Cys Glu
1               5                   10                  15

act ccc acg ctc gcg gcc agg gag cta aac ctt gga atc gcc aaa atc 96
Thr Pro Thr Leu Ala Ala Arg Glu Leu Asn Leu Gly Ile Ala Lys Ile
            20                  25                  30

ttg ctc gcc ata ttt ggt ccg ctc atg gtg ctc cag act ggc cta att 144
Leu Leu Ala Ile Phe Gly Pro Leu Met Val Leu Gln Thr Gly Leu Ile
        35                  40                  45

agg gtg ccg tac ttc gtg cgc gcc cag ggg ctc atc cgt gcg tgc atg 192
Arg Val Pro Tyr Phe Val Arg Ala Gln Gly Leu Ile Arg Ala Cys Met
    50                  55                  60

ttg gtg cgg aaa gtc tct ggg ggt cat tat gtc caa atg gct ctt gtg 240
Leu Val Arg Lys Val Ser Gly Gly His Tyr Val Gln Met Ala Leu Val
65                  70                  75                  80

agg cta gct gcg cta acg ggc acg tac gtt tat gac cat ctt act ccg 288
Arg Leu Ala Ala Leu Thr Gly Thr Tyr Val Tyr Asp His Leu Thr Pro
                85                  90                  95

ctg cgg gac tgg gcc cac gcg ggc ctg cga gat ctc gcg gtg gca gtt 336
Leu Arg Asp Trp Ala His Ala Gly Leu Arg Asp Leu Ala Val Ala Val
            100                 105                 110

gag ccc gtc atc ttc tct gac atg gag acc aag atc atc acc tgg gag 384
Glu Pro Val Ile Phe Ser Asp Met Glu Thr Lys Ile Ile Thr Trp Glu
        115                 120                 125

gca gac acc gcg gcg tgc ggg gac atc atc tcg ggc cta ccc gtc tcc 432
Ala Asp Thr Ala Ala Cys Gly Asp Ile Ile Ser Gly Leu Pro Val Ser
    130                 135                 140

gcc cga agg ggg agg gag ata ctt ttg ggg ccg gcc gat agt ttt aga 480
Ala Arg Arg Gly Arg Glu Ile Leu Leu Gly Pro Ala Asp Ser Phe Arg
145                 150                 155                 160

gat cag ggg tgg caa ctc ctt gcg ccc atc acg gcc tac tcc caa cag 528
Asp Gln Gly Trp Gln Leu Leu Ala Pro Ile Thr Ala Tyr Ser Gln Gln
                165                 170                 175

acg cgg ggc cta ctt ggc tgc atc atc act agc ctc aca ggc cgg gac 576
Thr Arg Gly Leu Leu Gly Cys Ile Ile Thr Ser Leu Thr Gly Arg Asp
            180                 185                 190

aag aac cag gtc gag gga gag gct cag gtg gtt tcc acc gca aca caa 624
Lys Asn Gln Val Glu Gly Glu Ala Gln Val Val Ser Thr Ala Thr Gln
        195                 200                 205

tcc ttc ctg gcg acc tgt gtt aat ggc gtg tgt tgg acc gcc tac cgt 672
Ser Phe Leu Ala Thr Cys Val Asn Gly Val Cys Trp Thr Ala Tyr Arg
    210                 215                 220

ggc gcc ggt gca aag acc cta gcc ggc cca aag ggt cca atc acc caa 720
Gly Ala Gly Ala Lys Thr Leu Ala Gly Pro Lys Gly Pro Ile Thr Gln
225                 230                 235                 240

atg tat acc aat gta gac cag gac ctc gtc ggt tgg cag gcg ccc tcc     768
Met Tyr Thr Asn Val Asp Gln Asp Leu Val Gly Trp Gln Ala Pro Ser
                245                 250                 255

ggg tcg cgt tcc tta acg cca tgc acc tgc ggt agc tcg gac ctt tac t   817
Gly Ser Arg Ser Leu Thr Pro Cys Thr Cys Gly Ser Ser Asp Leu Tyr
            260                 265                 270

<210>  38
<211>  272
<212>  PRT
<213>  Hepatitis C virus

<400>  38

Leu Leu Ala Leu Leu Ser Cys Leu Thr Val Pro Ala Ser Ala Cys Glu
1               5                   10                  15

Thr Pro Thr Leu Ala Ala Arg Glu Leu Asn Leu Gly Ile Ala Lys Ile
            20                  25                  30

Leu Leu Ala Ile Phe Gly Pro Leu Met Val Leu Gln Thr Gly Leu Ile
        35                  40                  45

Arg Val Pro Tyr Phe Val Arg Ala Gln Gly Leu Ile Arg Ala Cys Met
    50                  55                  60

Leu Val Arg Lys Val Ser Gly Gly His Tyr Val Gln Met Ala Leu Val
65                  70                  75                  80

Arg Leu Ala Ala Leu Thr Gly Thr Tyr Val Tyr Asp His Leu Thr Pro
                85                  90                  95

Leu Arg Asp Trp Ala His Ala Gly Leu Arg Asp Leu Ala Val Ala Val
            100                 105                 110

Glu Pro Val Ile Phe Ser Asp Met Glu Thr Lys Ile Ile Thr Trp Glu
        115                 120                 125

Ala Asp Thr Ala Ala Cys Gly Asp Ile Ile Ser Gly Leu Pro Val Ser
    130                 135                 140

Ala Arg Arg Gly Arg Glu Ile Leu Leu Gly Pro Ala Asp Ser Phe Arg
145                 150                 155                 160

Asp Gln Gly Trp Gln Leu Leu Ala Pro Ile Thr Ala Tyr Ser Gln Gln
                165                 170                 175

Thr Arg Gly Leu Leu Gly Cys Ile Ile Thr Ser Leu Thr Gly Arg Asp
            180                 185                 190

Lys Asn Gln Val Glu Gly Glu Ala Gln Val Val Ser Thr Ala Thr Gln
        195                 200                 205

Ser Phe Leu Ala Thr Cys Val Asn Gly Val Cys Trp Thr Ala Tyr Arg
    210                 215                 220

Gly Ala Gly Ala Lys Thr Leu Ala Gly Pro Lys Gly Pro Ile Thr Gln
225                 230                 235                 240

Met Tyr Thr Asn Val Asp Gln Asp Leu Val Gly Trp Gln Ala Pro Ser
                245                 250                 255

Gly Ser Arg Ser Leu Thr Pro Cys Thr Cys Gly Ser Ser Asp Leu Tyr
            260                 265                 270

<210>  39
<211>  2302
<212>  DNA
<213>  Hepatitis C virus

<220>
<221>  CDS
<222>  (1)..(2301)
<223>

<400>  39
cct ttg gct ctg cta tcc tgt ctg act gtc ccg gct tcc gct tat gag      48
Pro Leu Ala Leu Leu Ser Cys Leu Thr Val Pro Ala Ser Ala Tyr Glu
1               5                   10                  15

gtg cgc aac ttc tcc ggg ata tac cgt gtc acg aac gac tgc tcc aac      96
Val Arg Asn Phe Ser Gly Ile Tyr Arg Val Thr Asn Asp Cys Ser Asn
            20                  25                  30

tca agc att gtg tat gag gca gcg gac atg atc atg cat act ccc ggg      144
Ser Ser Ile Val Tyr Glu Ala Ala Asp Met Ile Met His Thr Pro Gly
        35                  40                  45

tgt gtg ccc tgc gtt cgg gag ggt aac tcc tcc cgt tgc tgg gta gcg      192

Cys Val Pro Cys Val Arg Glu Gly Asn Ser Ser Arg Cys Trp Val Ala
    50                  55                  60

ctc act ccc acg cta gcg gcc agg aat atc agc gtc ccc act acg aca 240
Leu Thr Pro Thr Leu Ala Ala Arg Asn Ile Ser Val Pro Thr Thr Thr
65                  70                  75                  80

ata cga cgc aat gtc gac ttg ctc gtt ggg gcg gct gct ttc tgc tcc 288
Ile Arg Arg Asn Val Asp Leu Leu Val Gly Ala Ala Ala Phe Cys Ser
                85                  90                  95

gcc atg tac gtg gga gac ctc tgc gga tct gtt ttc ctc gtc tcc cag 336
Ala Met Tyr Val Gly Asp Leu Cys Gly Ser Val Phe Leu Val Ser Gln
            100                 105                 110

ctt ttc acc ttc tcg cct cgc cgg cat gag aca gta cag gac tgc aat 384
Leu Phe Thr Phe Ser Pro Arg Arg His Glu Thr Val Gln Asp Cys Asn
        115                 120                 125

tgt tca atc tat tcc ggc cat gtg tca ggt cac cgt atg gct tgg gat 432
Cys Ser Ile Tyr Ser Gly His Val Ser Gly His Arg Met Ala Trp Asp
    130                 135                 140

atg atg atg aac tgg tca cct aca gca acc cta gta gtg tca cag tta 480
Met Met Met Asn Trp Ser Pro Thr Ala Thr Leu Val Val Ser Gln Leu
145                 150                 155                 160

ctc cgg atc cca caa gcc gtc gtg gac atg gtg gcg ggg gct cac tgg 528
Leu Arg Ile Pro Gln Ala Val Val Asp Met Val Ala Gly Ala His Trp
                165                 170                 175

gga gtc cta gcg ggc ctt gcc tac tat ccc atg gcg gga aac tgg gct 576
Gly Val Leu Ala Gly Leu Ala Tyr Tyr Pro Met Ala Gly Asn Trp Ala
            180                 185                 190

aag gtg tta att gta ttg ctg ctc ttc gcc ggc gtt gac ggg cag acc 624

Lys Val Leu Ile Val Leu Leu Leu Phe Ala Gly Val Asp Gly Gln Thr
        195                 200                 205

cgc gtg aca ggg ggg gcg gca gct cac acc gcc cgt ggg ctc act tcc 672
Arg Val Thr Gly Gly Ala Ala Ala His Thr Ala Arg Gly Leu Thr Ser
    210                 215                 220

atc ctt cca cct ggg ccg tct cag aac atc cag ctt gta aac acc aat 720
Ile Leu Pro Pro Gly Pro Ser Gln Asn Ile Gln Leu Val Asn Thr Asn
225                 230                 235                 240

ggc agc tgg cac atc aac agg act gct ctg aac tgc aat gac agc ctc 768
Gly Ser Trp His Ile Asn Arg Thr Ala Leu Asn Cys Asn Asp Ser Leu
                245                 250                 255

cag act ggg ttt ctt gcc gcg ctg ttc ttc aca cac aag ttc aac gcg 816
Gln Thr Gly Phe Leu Ala Ala Leu Phe Phe Thr His Lys Phe Asn Ala
            260                 265                 270

tct gga tgc cca gaa cgc atg gcc agc tgc cgt acc att gac aag ttc 864
Ser Gly Cys Pro Glu Arg Met Ala Ser Cys Arg Thr Ile Asp Lys Phe
        275                 280                 285

aat caa ggg tgg ggt ccc atc acc tat gat ggg cat ggc ggc cag gac 912
Asn Gln Gly Trp Gly Pro Ile Thr Tyr Asp Gly His Gly Gly Gln Asp
    290                 295                 300

cag agg cct tat tgc tgg cac tac gcg cct aag ccg tgc ggt atc gta 960
Gln Arg Pro Tyr Cys Trp His Tyr Ala Pro Lys Pro Cys Gly Ile Val
305                 310                 315                 320

ccc gcg tcg cag gtg tgt ggt cca gtg tat tgt ttc acc cca agc cca 1008
Pro Ala Ser Gln Val Cys Gly Pro Val Tyr Cys Phe Thr Pro Ser Pro
                325                 330                 335

gtt gtg gtg ggg acg acc gat cgc tcc ggt gtc cct acg tat agc tgg 1056

Val Val Val Gly Thr Thr Asp Arg Ser Gly Val Pro Thr Tyr Ser Trp
            340                 345                 350

ggg gag aat gag aca gac gtg ctg ctt ctt aac aac acg cgg ccg ccg 1104
Gly Glu Asn Glu Thr Asp Val Leu Leu Leu Asn Asn Thr Arg Pro Pro
        355                 360                 365

cta ggc aac tgg ttc ggc tgt aca tgg atg aat tgc act ggg ttc acc 1152
Leu Gly Asn Trp Phe Gly Cys Thr Trp Met Asn Cys Thr Gly Phe Thr
    370                 375                 380

aag acg tgc ggg ggc ccc ccg tgt aat atc ggg gga gtc ggc aac aac 1200
Lys Thr Cys Gly Gly Pro Pro Cys Asn Ile Gly Gly Val Gly Asn Asn
385                 390                 395                 400

acc ttg acc tgc ccc acg gat tgc ttc cgg aag cac ccc gaa gcc act 1248
Thr Leu Thr Cys Pro Thr Asp Cys Phe Arg Lys His Pro Glu Ala Thr
                405                 410                 415

tac acc aaa tgt ggt tcg ggg cct tgg ttg aca ccc aga tgc ata gtt 1296
Tyr Thr Lys Cys Gly Ser Gly Pro Trp Leu Thr Pro Arg Cys Ile Val
            420                 425                 430

gac tac cca tac agg ctc tgg cac tac ccc tgc act gtc aac ttc acc 1344
Asp Tyr Pro Tyr Arg Leu Trp His Tyr Pro Cys Thr Val Asn Phe Thr
        435                 440                 445

atc ttc aag gtt agg atg tat gtg ggg ggc gtg gag cac agg ctc aat 1392
Ile Phe Lys Val Arg Met Tyr Val Gly Gly Val Glu His Arg Leu Asn
    450                 455                 460

gct gca tgc aat tgg acc cga ggg gag cgt tgt ggg ttg gag gac agg 1440
Ala Ala Cys Asn Trp Thr Arg Gly Glu Arg Cys Gly Leu Glu Asp Arg
465                 470                 475                 480

gat aga tcg gag ctc agc ccg ctg ctg cta tct aca aca gag tgg cag 1488

Asp Arg Ser Glu Leu Ser Pro Leu Leu Leu Ser Thr Thr Glu Trp Gln
                485                 490                 495

ata ctg cct tgc tcc ttc acc aca cta ccg gct ctg tcc act ggt tta     1536
Ile Leu Pro Cys Ser Phe Thr Thr Leu Pro Ala Leu Ser Thr Gly Leu
            500                 505                 510

atc cat ctt cac cag aac atc gtg gac gtg caa tat ctg tac ggc ata     1584
Ile His Leu His Gln Asn Ile Val Asp Val Gln Tyr Leu Tyr Gly Ile
        515                 520                 525

ggg tcg gtg gtt gtc tcc tct gca atc aag tgg gag tat gtc gtg ttg     1632
Gly Ser Val Val Val Ser Ser Ala Ile Lys Trp Glu Tyr Val Val Leu
    530                 535                 540

ctc ttc ctt ctc ctg gcg gac gca cgc gtc tgt gcc tgc ttg tgg atg     1680
Leu Phe Leu Leu Leu Ala Asp Ala Arg Val Cys Ala Cys Leu Trp Met
545                 550                 555                 560

atg cta ctg gta gcc cag gcc gag gct gct tta gag aac cta gtg gtt     1728
Met Leu Leu Val Ala Gln Ala Glu Ala Ala Leu Glu Asn Leu Val Val
                565                 570                 575

ctc aac gcg gca tcc gtg gct ggg acg cac ggc att atc ccc ttc ctt     1776
Leu Asn Ala Ala Ser Val Ala Gly Thr His Gly Ile Ile Pro Phe Leu
            580                 585                 590

gtg ttc ttc tgt gcc gcc tgg tac atc aaa ggc agg ctc gtc cct gca     1824
Val Phe Phe Cys Ala Ala Trp Tyr Ile Lys Gly Arg Leu Val Pro Ala
        595                 600                 605

gcg gca tat gct ttc tat ggc gta tgg ccg ctg ctc ctg ctc ctg ctg     1872
Ala Ala Tyr Ala Phe Tyr Gly Val Trp Pro Leu Leu Leu Leu Leu Leu
    610                 615                 620

gcg tta cca cca cga gct tac gcc atg gac cgg gag atg gct gca tcg     1920

Ala Leu Pro Pro Arg Ala Tyr Ala Met Asp Arg Glu Met Ala Ala Ser
625                 630                 635                 640

tgt gga ggc ggg gtt ttt gta ggt ctg gca ttc ttg acc ttg tca cca     1968
Cys Gly Gly Gly Val Phe Val Gly Leu Ala Phe Leu Thr Leu Ser Pro
                645                 650                 655

tac tac aag gtg ttc ctc gct aag ctc ata tgg tgg tta caa tat ttt     2016
Tyr Tyr Lys Val Phe Leu Ala Lys Leu Ile Trp Trp Leu Gln Tyr Phe
            660                 665                 670

atc acc aga gcc gag gcg cac ttg caa gtg tgg atc ccc ccc ctc aac     2064
Ile Thr Arg Ala Glu Ala His Leu Gln Val Trp Ile Pro Pro Leu Asn
        675                 680                 685

gtt cgg gga ggc cgt gat gcc atc atc ctc ctc gca tgc gca gtc cac     2112
Val Arg Gly Gly Arg Asp Ala Ile Ile Leu Leu Ala Cys Ala Val His
    690                 695                 700

ccg gag cta atc ttt gac atc acc aaa ctt ctg ctc gcc ata ctc ggc     2160
Pro Glu Leu Ile Phe Asp Ile Thr Lys Leu Leu Leu Ala Ile Leu Gly
705                 710                 715                 720

ccg ctc atg gtg ttc cag gcc agc ata acc cga gtg ccg tac ttt gtg     2208
Pro Leu Met Val Phe Gln Ala Ser Ile Thr Arg Val Pro Tyr Phe Val
                725                 730                 735

cgc gct caa ggg ctc att cgt gca tgc atg tta gtg cgg aaa gcc gct     2256
Arg Ala Gln Gly Leu Ile Arg Ala Cys Met Leu Val Arg Lys Ala Ala
            740                 745                 750

ggg ggt cat tat atc caa atg gcc ctc gtg aaa ctg gcc gcg ctg a       2302
Gly Gly His Tyr Ile Gln Met Ala Leu Val Lys Leu Ala Ala Leu
        755                 760                 765

<210>  40
<211>  767
<212>  PRT
<213>  Hepatitis C virus

<400>  40

Pro Leu Ala Leu Leu Ser Cys Leu Thr Val Pro Ala Ser Ala Tyr Glu
1               5                   10                  15

Val Arg Asn Phe Ser Gly Ile Tyr Arg Val Thr Asn Asp Cys Ser Asn
            20                  25                  30

Ser Ser Ile Val Tyr Glu Ala Ala Asp Met Ile Met His Thr Pro Gly
        35                  40                  45

Cys Val Pro Cys Val Arg Glu Gly Asn Ser Ser Arg Cys Trp Val Ala
    50                  55                  60

Leu Thr Pro Thr Leu Ala Ala Arg Asn Ile Ser Val Pro Thr Thr Thr
65                  70                  75                  80

Ile Arg Arg Asn Val Asp Leu Leu Val Gly Ala Ala Ala Phe Cys Ser
                85                  90                  95

Ala Met Tyr Val Gly Asp Leu Cys Gly Ser Val Phe Leu Val Ser Gln
            100                 105                 110

Leu Phe Thr Phe Ser Pro Arg Arg His Glu Thr Val Gln Asp Cys Asn

        115                 120                 125

Cys Ser Ile Tyr Ser Gly His Val Ser Gly His Arg Met Ala Trp Asp
    130                 135                 140

Met Met Met Asn Trp Ser Pro Thr Ala Thr Leu Val Val Ser Gln Leu
145                 150                 155                 160

Leu Arg Ile Pro Gln Ala Val Val Asp Met Val Ala Gly Ala His Trp
                165                 170                 175

Gly Val Leu Ala Gly Leu Ala Tyr Tyr Pro Met Ala Gly Asn Trp Ala
            180                 185                 190

Lys Val Leu Ile Val Leu Leu Leu Phe Ala Gly Val Asp Gly Gln Thr
        195                 200                 205

Arg Val Thr Gly Gly Ala Ala Ala His Thr Ala Arg Gly Leu Thr Ser
    210                 215                 220

Ile Leu Pro Pro Gly Pro Ser Gln Asn Ile Gln Leu Val Asn Thr Asn
225                 230                 235                 240

Gly Ser Trp His Ile Asn Arg Thr Ala Leu Asn Cys Asn Asp Ser Leu
                245                 250                 255

Gln Thr Gly Phe Leu Ala Ala Leu Phe Phe Thr His Lys Phe Asn Ala

            260                 265                 270

Ser Gly Cys Pro Glu Arg Met Ala Ser Cys Arg Thr Ile Asp Lys Phe
        275                 280                 285

Asn Gln Gly Trp Gly Pro Ile Thr Tyr Asp Gly His Gly Gly Gln Asp
    290                 295                 300

Gln Arg Pro Tyr Cys Trp His Tyr Ala Pro Lys Pro Cys Gly Ile Val
305                 310                 315                 320

Pro Ala Ser Gln Val Cys Gly Pro Val Tyr Cys Phe Thr Pro Ser Pro
                325                 330                 335

Val Val Val Gly Thr Thr Asp Arg Ser Gly Val Pro Thr Tyr Ser Trp
            340                 345                 350

Gly Glu Asn Glu Thr Asp Val Leu Leu Leu Asn Asn Thr Arg Pro Pro
        355                 360                 365

Leu Gly Asn Trp Phe Gly Cys Thr Trp Met Asn Cys Thr Gly Phe Thr
    370                 375                 380

Lys Thr Cys Gly Gly Pro Pro Cys Asn Ile Gly Gly Val Gly Asn Asn
385                 390                 395                 400

Thr Leu Thr Cys Pro Thr Asp Cys Phe Arg Lys His Pro Glu Ala Thr

                405                 410                 415

Tyr Thr Lys Cys Gly Ser Gly Pro Trp Leu Thr Pro Arg Cys Ile Val
            420                 425                 430

Asp Tyr Pro Tyr Arg Leu Trp His Tyr Pro Cys Thr Val Asn Phe Thr
        435                 440                 445

Ile Phe Lys Val Arg Met Tyr Val Gly Gly Val Glu His Arg Leu Asn
    450                 455                 460

Ala Ala Cys Asn Trp Thr Arg Gly Glu Arg Cys Gly Leu Glu Asp Arg
465                 470                 475                 480

Asp Arg Ser Glu Leu Ser Pro Leu Leu Leu Ser Thr Thr Glu Trp Gln
                485                 490                 495

Ile Leu Pro Cys Ser Phe Thr Thr Leu Pro Ala Leu Ser Thr Gly Leu
            500                 505                 510

Ile His Leu His Gln Asn Ile Val Asp Val Gln Tyr Leu Tyr Gly Ile
        515                 520                 525

Gly Ser Val Val Val Ser Ser Ala Ile Lys Trp Glu Tyr Val Val Leu
    530                 535                 540

Leu Phe Leu Leu Leu Ala Asp Ala Arg Val Cys Ala Cys Leu Trp Met

545                 550                 555                 560

Met Leu Leu Val Ala Gln Ala Glu Ala Ala Leu Glu Asn Leu Val Val
                565                 570                 575

Leu Asn Ala Ala Ser Val Ala Gly Thr His Gly Ile Ile Pro Phe Leu
            580                 585                 590

Val Phe Phe Cys Ala Ala Trp Tyr Ile Lys Gly Arg Leu Val Pro Ala
        595                 600                 605

Ala Ala Tyr Ala Phe Tyr Gly Val Trp Pro Leu Leu Leu Leu Leu Leu
    610                 615                 620

Ala Leu Pro Pro Arg Ala Tyr Ala Met Asp Arg Glu Met Ala Ala Ser
625                 630                 635                 640

Cys Gly Gly Gly Val Phe Val Gly Leu Ala Phe Leu Thr Leu Ser Pro
                645                 650                 655

Tyr Tyr Lys Val Phe Leu Ala Lys Leu Ile Trp Trp Leu Gln Tyr Phe
            660                 665                 670

Ile Thr Arg Ala Glu Ala His Leu Gln Val Trp Ile Pro Pro Leu Asn
        675                 680                 685

Val Arg Gly Gly Arg Asp Ala Ile Ile Leu Leu Ala Cys Ala Val His

690                 695                 700

Pro Glu Leu Ile Phe Asp Ile Thr Lys Leu Leu Leu Ala Ile Leu Gly
705                 710                 715                 720

Pro Leu Met Val Phe Gln Ala Ser Ile Thr Arg Val Pro Tyr Phe Val
                725                 730                 735

Arg Ala Gln Gly Leu Ile Arg Ala Cys Met Leu Val Arg Lys Ala Ala
            740                 745                 750

Gly Gly His Tyr Ile Gln Met Ala Leu Val Lys Leu Ala Ala Leu
        755                 760                 765

<210>  41
<211>  2240
<212>  DNA
<213>  Hepatitis C virus

<220>
<221>  CDS
<222>  (1)..(2238)
<223>

<400>  41
ctc ttg gct ctg ctg tcc tgt ctg act atc cca gct tcc gcc tat gag      48
Leu Leu Ala Leu Leu Ser Cys Leu Thr Ile Pro Ala Ser Ala Tyr Glu
1               5                   10                  15

gtg cgc aac gtg tcc ggg ttg tac cat gtc acg aac gac tgc tcc aac      96
Val Arg Asn Val Ser Gly Leu Tyr His Val Thr Asn Asp Cys Ser Asn

            20                  25                  30

tca agt att gtg tat gag gca gcg gac atg atc atg cat acc ccc ggg 144
Ser Ser Ile Val Tyr Glu Ala Ala Asp Met Ile Met His Thr Pro Gly
        35                  40                  45

tgc gtg ccc tgc gtc cgg gag aac aac cgc cct cgc tgc tgg gta gcg 192
Cys Val Pro Cys Val Arg Glu Asn Asn Arg Pro Arg Cys Trp Val Ala
    50                  55                  60

ctc act ccc acg ctc gcg gcc aga aac agc agc atc ccc act gcg aca 240
Leu Thr Pro Thr Leu Ala Ala Arg Asn Ser Ser Ile Pro Thr Ala Thr
65                  70                  75                  80

ata cga cgc cat gtc gat ttg ctc gtt ggg gca gcc gct ctc tgc tcc 288
Ile Arg Arg His Val Asp Leu Leu Val Gly Ala Ala Ala Leu Cys Ser
                85                  90                  95

gcc atg tat gtg ggg gat ctt tgc gga tct gtc ttc ctc gtc tcc cag 336
Ala Met Tyr Val Gly Asp Leu Cys Gly Ser Val Phe Leu Val Ser Gln
            100                 105                 110

ctg ttc acc ttc tcg cct cgc cgg tat gag acg gta caa gac tgc aat 384
Leu Phe Thr Phe Ser Pro Arg Arg Tyr Glu Thr Val Gln Asp Cys Asn
        115                 120                 125

tgc tca ctc tat cct ggc cac gta aca ggt cac cgc atg gcc tgg gat 432
Cys Ser Leu Tyr Pro Gly His Val Thr Gly His Arg Met Ala Trp Asp
    130                 135                 140

atg atg atg aac tgg tcg cct aca aca gcc cta gtg gta tcg cag ata 480
Met Met Met Asn Trp Ser Pro Thr Thr Ala Leu Val Val Ser Gln Ile
145                 150                 155                 160

ctg cgg atc cca caa gcc gtc atg gac atg gtg acg ggg gcc cac tgg 528
Leu Arg Ile Pro Gln Ala Val Met Asp Met Val Thr Gly Ala His Trp

                165                 170                 175

gga gtc ctg gcg ggc ctc gcc tac tat tcc atg gtg gga aac tgg gct 576
Gly Val Leu Ala Gly Leu Ala Tyr Tyr Ser Met Val Gly Asn Trp Ala
            180                 185                 190

aag gtc ctg att gtg ttg tta ctc ttt gcc ggc gtt gat ggg acc acc 624
Lys Val Leu Ile Val Leu Leu Leu Phe Ala Gly Val Asp Gly Thr Thr
        195                 200                 205

cac ata acg ggg ggg act gca ggc caa act gcc ttt agc ctc aca agt 672
His Ile Thr Gly Gly Thr Ala Gly Gln Thr Ala Phe Ser Leu Thr Ser
    210                 215                 220

ctc ctc gca tct ggg ccg act cag aag atc caa att ata aac act aac 720
Leu Leu Ala Ser Gly Pro Thr Gln Lys Ile Gln Ile Ile Asn Thr Asn
225                 230                 235                 240

ggc agc tgg cac atc aac aga act gcc ttg agt tgt aac gac tcc ctt 768
Gly Ser Trp His Ile Asn Arg Thr Ala Leu Ser Cys Asn Asp Ser Leu
                245                 250                 255

cag act ggg ttc att gcc gcg ctg ttc tac aag cac agg ttc aac tcg 816
Gln Thr Gly Phe Ile Ala Ala Leu Phe Tyr Lys His Arg Phe Asn Ser
            260                 265                 270

tcc gga tgc cca gag cgc atg gcc agc tgc cgc ccc atc gac agg ttt 864
Ser Gly Cys Pro Glu Arg Met Ala Ser Cys Arg Pro Ile Asp Arg Phe
        275                 280                 285

aat cag ggg tgg ggt ccc att act tat gat gat aag ctt ccc gtc tca 912
Asn Gln Gly Trp Gly Pro Ile Thr Tyr Asp Asp Lys Leu Pro Val Ser
    290                 295                 300

gac cag agg cct tac tgc agg cac tac gcg cct cgg ccg tgc ggt atc 960
Asp Gln Arg Pro Tyr Cys Arg His Tyr Ala Pro Arg Pro Cys Gly Ile

305                 310                 315                 320

gtg ccc gcg tcg gag gtg tgt ggt ccg gtg tat tgc ttc acc cca agc 1008
Val Pro Ala Ser Glu Val Cys Gly Pro Val Tyr Cys Phe Thr Pro Ser
                325                 330                 335

cct gtt gtg gtg ggg acg acc gat cgc ttc ggc gct ccc acg tat aac 1056
Pro Val Val Val Gly Thr Thr Asp Arg Phe Gly Ala Pro Thr Tyr Asn
            340                 345                 350

tgg ggg gag aat gag acg gac gtg cta atc ctc aac aac acg cgg ccg 1104
Trp Gly Glu Asn Glu Thr Asp Val Leu Ile Leu Asn Asn Thr Arg Pro
        355                 360                 365

ccg caa ggc aac tgg ttt ggc tgt aca tgg atg aat aac acc ggg ttc 1152
Pro Gln Gly Asn Trp Phe Gly Cys Thr Trp Met Asn Asn Thr Gly Phe
    370                 375                 380

acc aag acg tgc gga ggc cct ccg tgt aac atc ggg ggg gtc ggc aat 1200
Thr Lys Thr Cys Gly Gly Pro Pro Cys Asn Ile Gly Gly Val Gly Asn
385                 390                 395                 400

gag acc ttg acc tgc cct acg gat tgc ttc cgg aag cac ccc gaa gcc 1248
Glu Thr Leu Thr Cys Pro Thr Asp Cys Phe Arg Lys His Pro Glu Ala
                405                 410                 415

acg tac acc aaa tgc ggc tcg ggg cct tgg ttg aca cct agg tgt atg 1296
Thr Tyr Thr Lys Cys Gly Ser Gly Pro Trp Leu Thr Pro Arg Cys Met
            420                 425                 430

gtt gat tac cca tac aga ctt tgg cac tac ccc tgc act gta aac ttt 1344
Val Asp Tyr Pro Tyr Arg Leu Trp His Tyr Pro Cys Thr Val Asn Phe
        435                 440                 445

act atc ttc aag atc agg atg tac gtg ggg ggt gta gag cac agg ttc 1392
Thr Ile Phe Lys Ile Arg Met Tyr Val Gly Gly Val Glu His Arg Phe

450                 455                 460

aca gcc gcg tgc aat tgg gcc cga gga gag cgc tgt gac gta gag gac      1440
Thr Ala Ala Cys Asn Trp Ala Arg Gly Glu Arg Cys Asp Val Glu Asp
465                 470                 475                 480

agg gat aga gca gag ctc agc ccg cta cta ctg tct aca act gag tgg      1488
Arg Asp Arg Ala Glu Leu Ser Pro Leu Leu Leu Ser Thr Thr Glu Trp
                485                 490                 495

cag ata ctg ccc tgt tcc ttt acc acc cta cca gct ctg tcc acc gga      1536
Gln Ile Leu Pro Cys Ser Phe Thr Thr Leu Pro Ala Leu Ser Thr Gly
            500                 505                 510

tcg atc cac ctc cat cag aac acc gtg gac gtg caa tac ctg tac ggt      1584
Ser Ile His Leu His Gln Asn Thr Val Asp Val Gln Tyr Leu Tyr Gly
        515                 520                 525

gta ggg tca gcg gtt gtt tcc atc gcg atc aaa tgg gag tat gtc ctg      1632
Val Gly Ser Ala Val Val Ser Ile Ala Ile Lys Trp Glu Tyr Val Leu
    530                 535                 540

ctg ctt ttc ctt ctc ttg gcg gac gca cgc gtc tgc gcc tgt ttg tgg      1680
Leu Leu Phe Leu Leu Leu Ala Asp Ala Arg Val Cys Ala Cys Leu Trp
545                 550                 555                 560

atg atg ctg ctg ata gcc cag gct gag gcc gct ttg gag aac ctg gtg      1728
Met Met Leu Leu Ile Ala Gln Ala Glu Ala Ala Leu Glu Asn Leu Val
                565                 570                 575

atc ctc aat gcg gcg tcc gta gct gga gcg cac ggc att ctt tcc ttc      1776
Ile Leu Asn Ala Ala Ser Val Ala Gly Ala His Gly Ile Leu Ser Phe
            580                 585                 590

ctc atg ttc ttc tgt gct gcc tgg tat atc aag ggt aag ctg gtc ccc      1824
Leu Met Phe Phe Cys Ala Ala Trp Tyr Ile Lys Gly Lys Leu Val Pro

        595                 600                 605

ggg gcg gca tat gct ttc tat agc gta tgg ccg ctg ctc ctg ctc ctg 1872
Gly Ala Ala Tyr Ala Phe Tyr Ser Val Trp Pro Leu Leu Leu Leu Leu
    610                 615                 620

ctg gcg cta cca cca cga gcg tac gct atg gac cgg gag atg gct gca 1920
Leu Ala Leu Pro Pro Arg Ala Tyr Ala Met Asp Arg Glu Met Ala Ala
625                 630                 635                 640

tca tgt ggg ggt ggg gtc ttc ata ggt ttg gta gtc ttg act ttg tca 1968
Ser Cys Gly Gly Gly Val Phe Ile Gly Leu Val Val Leu Thr Leu Ser
                645                 650                 655

ccg cac tat aaa gca ttc ctc gct agg ctt ata tgg tgg tta caa tat 2016
Pro His Tyr Lys Ala Phe Leu Ala Arg Leu Ile Trp Trp Leu Gln Tyr
            660                 665                 670

ttt atc acc agg acc gag gcg cac ttg caa gtg tgg atc ccc ccc ctc 2064
Phe Ile Thr Arg Thr Glu Ala His Leu Gln Val Trp Ile Pro Pro Leu
        675                 680                 685

aac gtt cgg ggg ggc cgt gat gcc atc atc ctc ctc atg tgc gtg gtc 2112
Asn Val Arg Gly Gly Arg Asp Ala Ile Ile Leu Leu Met Cys Val Val
    690                 695                 700

cat cca gag cta att ttt gaa atc acc aag atc ttg ctc gcc ata ctg 2160
His Pro Glu Leu Ile Phe Glu Ile Thr Lys Ile Leu Leu Ala Ile Leu
705                 710                 715                 720

ggt ccg ccc atg gtg ctc cag gcc ggc ctg att agg gtg ccg tac ttc 2208
Gly Pro Pro Met Val Leu Gln Ala Gly Leu Ile Arg Val Pro Tyr Phe
                725                 730                 735

gtg cgc gct caa ggg ctc att cgt gcg tgc at 2240
Val Arg Ala Gln Gly Leu Ile Arg Ala Cys

740                 745

<210>  42
<211>  746
<212>  PRT
<213>  Hepatitis C virus

<400>  42

Leu Leu Ala Leu Leu Ser Cys Leu Thr Ile Pro Ala Ser Ala Tyr Glu
1               5                   10                  15

Val Arg Asn Val Ser Gly Leu Tyr His Val Thr Asn Asp Cys Ser Asn
            20                  25                  30

Ser Ser Ile Val Tyr Glu Ala Ala Asp Met Ile Met His Thr Pro Gly
        35                  40                  45

Cys Val Pro Cys Val Arg Glu Asn Asn Arg Pro Arg Cys Trp Val Ala
    50                  55                  60

Leu Thr Pro Thr Leu Ala Ala Arg Asn Ser Ser Ile Pro Thr Ala Thr
65                  70                  75                  80

Ile Arg Arg His Val Asp Leu Leu Val Gly Ala Ala Ala Leu Cys Ser
                85                  90                  95

Ala Met Tyr Val Gly Asp Leu Cys Gly Ser Val Phe Leu Val Ser Gln
            100                 105                 110

Leu Phe Thr Phe Ser Pro Arg Arg Tyr Glu Thr Val Gln Asp Cys Asn
        115                 120                 125

Cys Ser Leu Tyr Pro Gly His Val Thr Gly His Arg Met Ala Trp Asp
    130                 135                 140

Met Met Met Asn Trp Ser Pro Thr Thr Ala Leu Val Val Ser Gln Ile
145                 150                 155                 160

Leu Arg Ile Pro Gln Ala Val Met Asp Met Val Thr Gly Ala His Trp
                165                 170                 175

Gly Val Leu Ala Gly Leu Ala Tyr Tyr Ser Met Val Gly Asn Trp Ala
            180                 185                 190

Lys Val Leu Ile Val Leu Leu Leu Phe Ala Gly Val Asp Gly Thr Thr
        195                 200                 205

His Ile Thr Gly Gly Thr Ala Gly Gln Thr Ala Phe Ser Leu Thr Ser
    210                 215                 220

Leu Leu Ala Ser Gly Pro Thr Gln Lys Ile Gln Ile Ile Asn Thr Asn
225                 230                 235                 240

Gly Ser Trp His Ile Asn Arg Thr Ala Leu Ser Cys Asn Asp Ser Leu
                245                 250                 255

Gln Thr Gly Phe Ile Ala Ala Leu Phe Tyr Lys His Arg Phe Asn Ser
            260                 265                 270

Ser Gly Cys Pro Glu Arg Met Ala Ser Cys Arg Pro Ile Asp Arg Phe
        275                 280                 285

Asn Gln Gly Trp Gly Pro Ile Thr Tyr Asp Asp Lys Leu Pro Val Ser
    290                 295                 300

Asp Gln Arg Pro Tyr Cys Arg His Tyr Ala Pro Arg Pro Cys Gly Ile
305                 310                 315                 320

Val Pro Ala Ser Glu Val Cys Gly Pro Val Tyr Cys Phe Thr Pro Ser
                325                 330                 335

Pro Val Val Val Gly Thr Thr Asp Arg Phe Gly Ala Pro Thr Tyr Asn
            340                 345                 350

Trp Gly Glu Asn Glu Thr Asp Val Leu Ile Leu Asn Asn Thr Arg Pro
        355                 360                 365

Pro Gln Gly Asn Trp Phe Gly Cys Thr Trp Met Asn Asn Thr Gly Phe
    370                 375                 380

Thr Lys Thr Cys Gly Gly Pro Pro Cys Asn Ile Gly Gly Val Gly Asn
385                 390                 395                 400

Glu Thr Leu Thr Cys Pro Thr Asp Cys Phe Arg Lys His Pro Glu Ala
                405                 410                 415

Thr Tyr Thr Lys Cys Gly Ser Gly Pro Trp Leu Thr Pro Arg Cys Met
            420                 425                 430

Val Asp Tyr Pro Tyr Arg Leu Trp His Tyr Pro Cys Thr Val Asn Phe
        435                 440                 445

Thr Ile Phe Lys Ile Arg Met Tyr Val Gly Gly Val Glu His Arg Phe
    450                 455                 460

Thr Ala Ala Cys Asn Trp Ala Arg Gly Glu Arg Cys Asp Val Glu Asp
465                 470                 475                 480

Arg Asp Arg Ala Glu Leu Ser Pro Leu Leu Leu Ser Thr Thr Glu Trp
                485                 490                 495

Gln Ile Leu Pro Cys Ser Phe Thr Thr Leu Pro Ala Leu Ser Thr Gly
            500                 505                 510

Ser Ile His Leu His Gln Asn Thr Val Asp Val Gln Tyr Leu Tyr Gly
        515                 520                 525

Val Gly Ser Ala Val Val Ser Ile Ala Ile Lys Trp Glu Tyr Val Leu
    530                 535                 540

Leu Leu Phe Leu Leu Leu Ala Asp Ala Arg Val Cys Ala Cys Leu Trp
545                 550                 555                 560

Met Met Leu Leu Ile Ala Gln Ala Glu Ala Ala Leu Glu Asn Leu Val
                565                 570                 575

Ile Leu Asn Ala Ala Ser Val Ala Gly Ala His Gly Ile Leu Ser Phe
            580                 585                 590

Leu Met Phe Phe Cys Ala Ala Trp Tyr Ile Lys Gly Lys Leu Val Pro
        595                 600                 605

Gly Ala Ala Tyr Ala Phe Tyr Ser Val Trp Pro Leu Leu Leu Leu Leu
    610                 615                 620

Leu Ala Leu Pro Pro Arg Ala Tyr Ala Met Asp Arg Glu Met Ala Ala
625                 630                 635                 640

Ser Cys Gly Gly Gly Val Phe Ile Gly Leu Val Val Leu Thr Leu Ser
                645                 650                 655

Pro His Tyr Lys Ala Phe Leu Ala Arg Leu Ile Trp Trp Leu Gln Tyr
            660                 665                 670

Phe Ile Thr Arg Thr Glu Ala His Leu Gln Val Trp Ile Pro Pro Leu
        675                 680                 685

Asn Val Arg Gly Gly Arg Asp Ala Ile Ile Leu Leu Met Cys Val Val
    690                 695                 700

His Pro Glu Leu Ile Phe Glu Ile Thr Lys Ile Leu Leu Ala Ile Leu
705                 710                 715                 720

Gly Pro Pro Met Val Leu Gln Ala Gly Leu Ile Arg Val Pro Tyr Phe
                725                 730                 735

Val Arg Ala Gln Gly Leu Ile Arg Ala Cys
            740                 745

<210>  43
<211>  2237
<212>  DNA
<213>  Hepatitis C virus

<220>
<221>  CDS
<222>  (1)..(2226)
<223>

<400>  43
ctc ttg gct ttg ttg tcc tgt ttg acc gtc cca act tcc gct tat gaa        48
Leu Leu Ala Leu Leu Ser Cys Leu Thr Val Pro Thr Ser Ala Tyr Glu
1               5                   10                  15

gtg cgc aac gtg tcc ggg atg tac caa gtc acg aac gac tgc tcc aac        96
Val Arg Asn Val Ser Gly Met Tyr Gln Val Thr Asn Asp Cys Ser Asn
            20                  25                  30

tca agc att gtg tat gag gca gcg gac gtg atc atg cac acc ccc ggg 144
Ser Ser Ile Val Tyr Glu Ala Ala Asp Val Ile Met His Thr Pro Gly
        35                  40                  45

tgc gtg ccc tgt gtc cgg gag agc aac ctc tcc cgc tgc tgg gtt gcg 192
Cys Val Pro Cys Val Arg Glu Ser Asn Leu Ser Arg Cys Trp Val Ala
    50                  55                  60

ctc acg ccc acg ctc gcg gcc agg aac agc agt atc ccc act acg aca 240
Leu Thr Pro Thr Leu Ala Ala Arg Asn Ser Ser Ile Pro Thr Thr Thr
65                  70                  75                  80

ata cga cgt cat gtc gat ttg ctc gtt ggg gca tct gcc ttc tgc tcc 288
Ile Arg Arg His Val Asp Leu Leu Val Gly Ala Ser Ala Phe Cys Ser
                85                  90                  95

gct atg tac gtg ggg gat ctt tgc gga tct gtc ttc ctc atc tcc cag 336
Ala Met Tyr Val Gly Asp Leu Cys Gly Ser Val Phe Leu Ile Ser Gln
            100                 105                 110

ctg ttc acc ttc tca cct cgc cgg tac gag acg gtg caa gac tgc aat 384
Leu Phe Thr Phe Ser Pro Arg Arg Tyr Glu Thr Val Gln Asp Cys Asn
        115                 120                 125

tgc tca ctc tat ccc ggc cac gta tca ggt cat cgc atg gct tgg gat 432
Cys Ser Leu Tyr Pro Gly His Val Ser Gly His Arg Met Ala Trp Asp
    130                 135                 140

atg atg atg aac tgg tcg cct aca aca gcc tta gtg gta tcg cag tta 480
Met Met Met Asn Trp Ser Pro Thr Thr Ala Leu Val Val Ser Gln Leu
145                 150                 155                 160

ctc cgg atc cca caa gcc atc gtg gac atg gtg aca ggg gcc cac tgg 528
Leu Arg Ile Pro Gln Ala Ile Val Asp Met Val Thr Gly Ala His Trp
                165                 170                 175

ggg gtc ttg gcg ggt ctc gcc tat tac tcc atg gtg ggg aac tgg gct 576
Gly Val Leu Ala Gly Leu Ala Tyr Tyr Ser Met Val Gly Asn Trp Ala
            180                 185                 190

aag gtc ttg att gtg atg cta ctc ttt tcc ggc gtt gac ggc gcc act 624
Lys Val Leu Ile Val Met Leu Leu Phe Ser Gly Val Asp Gly Ala Thr
        195                 200                 205

cgc ctg tca ggg ggg gcg gca ggt cgt gat acc cgc ggc ttc gcg gcc 672
Arg Leu Ser Gly Gly Ala Ala Gly Arg Asp Thr Arg Gly Phe Ala Ala
    210                 215                 220

ctc ttc cag cca ggg tca gct cag aac atc cag ctt ata aac tcc aac 720
Leu Phe Gln Pro Gly Ser Ala Gln Asn Ile Gln Leu Ile Asn Ser Asn
225                 230                 235                 240

ggc agc tgg cac gtc aac agg aca gcc ctg aat tgc aat gac acc ctc 768
Gly Ser Trp His Val Asn Arg Thr Ala Leu Asn Cys Asn Asp Thr Leu
                245                 250                 255

cac act ggg ttc att gcc ggg ctg ctc tac aca acc aaa ttc aac tcg 816
His Thr Gly Phe Ile Ala Gly Leu Leu Tyr Thr Thr Lys Phe Asn Ser
            260                 265                 270

tcc ggg tgc cca ggg cgc ctg gcc agc tgc cgc ccc att gac aag ttc 864
Ser Gly Cys Pro Gly Arg Leu Ala Ser Cys Arg Pro Ile Asp Lys Phe
        275                 280                 285

gcc cag ggg tgg ggt ccc atc act tat gct gag cca gga gcc tcg gac 912
Ala Gln Gly Trp Gly Pro Ile Thr Tyr Ala Glu Pro Gly Ala Ser Asp
    290                 295                 300

cag agg ccc tat tgc tgg cac tac gcg cct cgg ccg tgc ggt att gta 960
Gln Arg Pro Tyr Cys Trp His Tyr Ala Pro Arg Pro Cys Gly Ile Val
305                 310                 315                 320

ccc gcg tcg cag gtg tgt ggt cca gta tat tgc ttc acc cca agc ccc 1008
Pro Ala Ser Gln Val Cys Gly Pro Val Tyr Cys Phe Thr Pro Ser Pro
                325                 330                 335

gtc gtg gtg ggt acg acc gat cgc tcc ggt gcc ccc acg tat acc tgg 1056
Val Val Val Gly Thr Thr Asp Arg Ser Gly Ala Pro Thr Tyr Thr Trp
            340                 345                 350

ggg gag aat gag acg gac gtg cta ctt ctc aac aac aca cgg ccg ccg 1104
Gly Glu Asn Glu Thr Asp Val Leu Leu Leu Asn Asn Thr Arg Pro Pro
        355                 360                 365

caa ggc aac tgg ttc ggc tgt aca tgg atg aat agc acc ggg ttc acc 1152
Gln Gly Asn Trp Phe Gly Cys Thr Trp Met Asn Ser Thr Gly Phe Thr
    370                 375                 380

aag acg tgt ggg gcc cct ccg tgc aac atc ggg ggg agc ggc aac aac 1200
Lys Thr Cys Gly Ala Pro Pro Cys Asn Ile Gly Gly Ser Gly Asn Asn
385                 390                 395                 400

acc ttg atc tgc cct acg gat tgc ttc cgg aag cac ccc gag gcc act 1248
Thr Leu Ile Cys Pro Thr Asp Cys Phe Arg Lys His Pro Glu Ala Thr
                405                 410                 415

tac atc aaa tgc ggc tcg ggg ccg tgg ttg aca cct agg tgt cta gtt 1296
Tyr Ile Lys Cys Gly Ser Gly Pro Trp Leu Thr Pro Arg Cys Leu Val
            420                 425                 430

gat tac cca tac agg ctt tgg cac tac ccc tgc acc gtc aac ttt acc 1344
Asp Tyr Pro Tyr Arg Leu Trp His Tyr Pro Cys Thr Val Asn Phe Thr
        435                 440                 445

atc ttc aag atc agg atg tat gtg ggg ggc gtg gag cac aga ctc act 1392
Ile Phe Lys Ile Arg Met Tyr Val Gly Gly Val Glu His Arg Leu Thr
    450                 455                 460

gcc gca tgc aat tgg act cga gga gag cgt tgc gat ttg gag gac agg 1440
Ala Ala Cys Asn Trp Thr Arg Gly Glu Arg Cys Asp Leu Glu Asp Arg
465                 470                 475                 480

gat aga tcg gaa ctt agc cca ctg tta ctc tct aca acg gag tgg cag 1488
Asp Arg Ser Glu Leu Ser Pro Leu Leu Leu Ser Thr Thr Glu Trp Gln
                485                 490                 495

ata ctg ccc tgt tcc ttc acc acc cta ccg gct ttg tcc act ggt ttg 1536
Ile Leu Pro Cys Ser Phe Thr Thr Leu Pro Ala Leu Ser Thr Gly Leu
            500                 505                 510

att cat ctc cat cag aac att gtg gac gta caa tac ctg tac ggt gta 1584
Ile His Leu His Gln Asn Ile Val Asp Val Gln Tyr Leu Tyr Gly Val
        515                 520                 525

ggg tca gcg gtt gtc tcc att gcg atc aaa tgg gag tac gtc gtg ctg 1632
Gly Ser Ala Val Val Ser Ile Ala Ile Lys Trp Glu Tyr Val Val Leu
    530                 535                 540

ctc ttt ctc ctc ctg gcg gac gca cgc ttc tgc gcc tgc ttg tgg atg 1680
Leu Phe Leu Leu Leu Ala Asp Ala Arg Phe Cys Ala Cys Leu Trp Met
545                 550                 555                 560

atg ctg ctg ata gcc cag gct gag gcc gcc tta gag aac ctg gtg atc 1728
Met Leu Leu Ile Ala Gln Ala Glu Ala Ala Leu Glu Asn Leu Val Ile
                565                 570                 575

ctc aat gca gcg tcc gtg gcc gga gcc cgt ggc att ctc tcc ttc ctt 1776
Leu Asn Ala Ala Ser Val Ala Gly Ala Arg Gly Ile Leu Ser Phe Leu
            580                 585                 590

gtg ttc ttc tgt gct gcc tgg tac atc aag ggc aaa ctg gtc cct ggg 1824
Val Phe Phe Cys Ala Ala Trp Tyr Ile Lys Gly Lys Leu Val Pro Gly
        595                 600                 605

gcg gca tat gcc ctc tac ggt gta tgg ccg ctg ctt ctg ctc ctg ctg 1872
Ala Ala Tyr Ala Leu Tyr Gly Val Trp Pro Leu Leu Leu Leu Leu Leu
    610                 615                 620

gcg tta cca cca cga gca tac gcc ttt gac cgg gaa atg gct gca tcg 1920
Ala Leu Pro Pro Arg Ala Tyr Ala Phe Asp Arg Glu Met Ala Ala Ser
625                 630                 635                 640

tgc gga ggc gcg gtt ttc ata ggt ctg atg ctt ctg acc ttg tca cca 1968
Cys Gly Gly Ala Val Phe Ile Gly Leu Met Leu Leu Thr Leu Ser Pro
                645                 650                 655

cac tat aag gca ctc ctc gcc agg ctt ata tgg tgg tta caa tat ttt 2016
His Tyr Lys Ala Leu Leu Ala Arg Leu Ile Trp Trp Leu Gln Tyr Phe
            660                 665                 670

atc acc agg gcc gag gcg cac ttg caa gtg tgg atc ccc ccc ctt aac 2064
Ile Thr Arg Ala Glu Ala His Leu Gln Val Trp Ile Pro Pro Leu Asn
        675                 680                 685

gtt cgg ggg ggc cgc gat gcc att atc ctc ctc aca tgt gcg gtc cat 2112
Val Arg Gly Gly Arg Asp Ala Ile Ile Leu Leu Thr Cys Ala Val His
    690                 695                 700

tca gag cta att ttt gaa atc acc aaa atc ttg ctc gcc atg ctt ggt 2160
Ser Glu Leu Ile Phe Glu Ile Thr Lys Ile Leu Leu Ala Met Leu Gly
705                 710                 715                 720

ccg ctc atg atg ctc cag gct ggc cta att aga gtg ccg tac ttt gtg 2208
Pro Leu Met Met Leu Gln Ala Gly Leu Ile Arg Val Pro Tyr Phe Val
                725                 730                 735

cgc gct caa ggg ctt att cgtgcgtgct t 2237
Arg Ala Gln Gly Leu Ile
            740

<210>  44
<211>  742
<212>  PRT
<213>  Hepatitis C virus

<400>  44
   Leu Leu Ala Leu Leu Ser Cys Leu Thr Val Pro Thr Ser Ala Tyr Glu
   1               5                   10                  15
   Val Arg Asn Val Ser Gly Met Tyr Gln Val Thr Asn Asp Cys Ser Asn
               20                  25                  30
   Ser Ser Ile Val Tyr Glu Ala Ala Asp Val Ile Met His Thr Pro Gly
           35                  40                  45
   Cys Val Pro Cys Val Arg Glu Ser Asn Leu Ser Arg Cys Trp Val Ala
       50                  55                  60
   Leu Thr Pro Thr Leu Ala Ala Arg Asn Ser Ser Ile Pro Thr Thr Thr
   65                  70                  75                  80
   Ile Arg Arg His Val Asp Leu Leu Val Gly Ala Ser Ala Phe Cys Ser
                   85                  90                  95
   Ala Met Tyr Val Gly Asp Leu Cys Gly Ser Val Phe Leu Ile Ser Gln
               100                 105                 110
   Leu Phe Thr Phe Ser Pro Arg Arg Tyr Glu Thr Val Gln Asp Cys Asn
           115                 120                 125
   Cys Ser Leu Tyr Pro Gly His Val Ser Gly His Arg Met Ala Trp Asp
       130                 135                 140
   Met Met Met Asn Trp Ser Pro Thr Thr Ala Leu Val Val Ser Gln Leu
   145                 150                 155                 160
   Leu Arg Ile Pro Gln Ala Ile Val Asp Met Val Thr Gly Ala His Trp
                   165                 170                 175
   Gly Val Leu Ala Gly Leu Ala Tyr Tyr Ser Met Val Gly Asn Trp Ala
               180                 185                 190
   Lys Val Leu Ile Val Met Leu Leu Phe Ser Gly Val Asp Gly Ala Thr
           195                 200                 205
   Arg Leu Ser Gly Gly Ala Ala Gly Arg Asp Thr Arg Gly Phe Ala Ala
       210                 215                 220
   Leu Phe Gln Pro Gly Ser Ala Gln Asn Ile Gln Leu Ile Asn Ser Asn
   225                 230                 235                 240
   Gly Ser Trp His Val Asn Arg Thr Ala Leu Asn Cys Asn Asp Thr Leu
                   245                 250                 255
   His Thr Gly Phe Ile Ala Gly Leu Leu Tyr Thr Thr Lys Phe Asn Ser
   260 265 270
   Ser Gly Cys Pro Gly Arg Leu Ala Ser Cys Arg Pro Ile Asp Lys Phe
   275 280 285
   Ala Gln Gly Trp Gly Pro Ile Thr Tyr Ala Glu Pro Gly Ala Ser Asp
   290 295 300
   Gln Arg Pro Tyr Cys Trp His Tyr Ala Pro Arg Pro Cys Gly Ile Val
   305 310 315 320
   Pro Ala Ser Gln Val Cys Gly Pro Val Tyr Cys Phe Thr Pro Ser Pro
   325 330 335
   Val Val Val Gly Thr Thr Asp Arg Ser Gly Ala Pro Thr Tyr Thr Trp
   340 345 350
   Gly Glu Asn Glu Thr Asp Val Leu Leu Leu Asn Asn Thr Arg Pro Pro
   355 360 365
   Gln Gly Asn Trp Phe Gly Cys Thr Trp Met Asn Ser Thr Gly Phe Thr
   370 375 380
   Lys Thr Cys Gly Ala Pro Pro Cys Asn Ile Gly Gly Ser Gly Asn Asn
   385 390 395 400
   Thr Leu Ile Cys Pro Thr Asp Cys Phe Arg Lys His Pro Glu Ala Thr
                       405                 410                 415
   Tyr Ile Lys Cys Gly Ser Gly Pro Trp Leu Thr Pro Arg Cys Leu Val
                   420                 425                 430
   Asp Tyr Pro Tyr Arg Leu Trp His Tyr Pro Cys Thr Val Asn Phe Thr
               435                 440                 445
   Ile Phe Lys Ile Arg Met Tyr Val Gly Gly Val Glu His Arg Leu Thr
       450                 455                 460
   Ala Ala Cys Asn Trp Thr Arg Gly Glu Arg Cys Asp Leu Glu Asp Arg
   465                 470                 475                 480
   Asp Arg Ser Glu Leu Ser Pro Leu Leu Leu Ser Thr Thr Glu Trp Gln
                       485                 490                 495
   Ile Leu Pro Cys Ser Phe Thr Thr Leu Pro Ala Leu Ser Thr Gly Leu
                   500                 505                 510
   Ile His Leu His Gln Asn Ile Val Asp Val Gln Tyr Leu Tyr Gly Val
               515                 520                 525
   Gly Ser Ala Val Val Ser Ile Ala Ile Lys Trp Glu Tyr Val Val Leu
       530                 535                 540
   Leu Phe Leu Leu Leu Ala Asp Ala Arg Phe Cys Ala Cys Leu Trp Met
   545                 550                 555                 560
   Met Leu Leu Ile Ala Gln Ala Glu Ala Ala Leu Glu Asn Leu Val Ile
                   565                 570                 575
   Leu Asn Ala Ala Ser Val Ala Gly Ala Arg Gly Ile Leu Ser Phe Leu
               580                 585                 590
   Val Phe Phe Cys Ala Ala Trp Tyr Ile Lys Gly Lys Leu Val Pro Gly
           595                 600                 605
   Ala Ala Tyr Ala Leu Tyr Gly Val Trp Pro Leu Leu Leu Leu Leu Leu
       610                 615                 620
   Ala Leu Pro Pro Arg Ala Tyr Ala Phe Asp Arg Glu Met Ala Ala Ser
   625                 630                 635                 640
   Cys Gly Gly Ala Val Phe Ile Gly Leu Met Leu Leu Thr Leu Ser Pro
                   645                 650                 655
   His Tyr Lys Ala Leu Leu Ala Arg Leu Ile Trp Trp Leu Gln Tyr Phe
               660                 665                 670
   Ile Thr Arg Ala Glu Ala His Leu Gln Val Trp Ile Pro Pro Leu Asn
           675                 680                 685
   Val Arg Gly Gly Arg Asp Ala Ile Ile Leu Leu Thr Cys Ala Val His
       690                 695                 700
   Ser Glu Leu Ile Phe Glu Ile Thr Lys Ile Leu Leu Ala Met Leu Gly
   705                 710                 715                 720
   Pro Leu Met Met Leu Gln Ala Gly Leu Ile Arg Val Pro Tyr Phe Val
                   725                 730                 735
   Arg Ala Gln Gly Leu Ile
               740

<210>  45
<211>  490
<212>  DNA
<213>  Hepatitis C virus

<220>
<221>  CDS
<222>  (1).. (489)
<223>

<400>  45
ctc ttg gct ctg ctg tcc tgt ctg acc atc cca gct tcc gct tat gaa        48
Leu Leu Ala Leu Leu Ser Cys Leu Thr Ile Pro Ala Ser Ala Tyr Glu
1               5                   10                  15

gtg cgc aac gtg tcc gga ata tac cat gtc acg aac gac tgc tcc aac        96
Val Arg Asn Val Ser Gly Ile Tyr His Val Thr Asn Asp Cys Ser Asn
            20                  25                  30

tca agc att gtg tat gag gca gcg gac gtg atc atg cat acc ccc ggg 144
Ser Ser Ile Val Tyr Glu Ala Ala Asp Val Ile Met His Thr Pro Gly
        35                  40                  45

tgc gtg ccc tgt gtt cgg gag ggt aac gcc tcc cgt tgt tgg gca gcg 192
Cys Val Pro Cys Val Arg Glu Gly Asn Ala Ser Arg Cys Trp Ala Ala
    50                  55                  60

ctc act ccc acg ctc gcg gtc ggg aat gcc agc gtc ccc act aag gca 240
Leu Thr Pro Thr Leu Ala Val Gly Asn Ala Ser Val Pro Thr Lys Ala
65                  70                  75                  80

ata cgg cgc cac gtc gat ctg ctt gtt ggg acg gct gct ttc tgc tcc 288
Ile Arg Arg His Val Asp Leu Leu Val Gly Thr Ala Ala Phe Cys Ser
                85                  90                  95

gcc atg tac gtg ggg gat ctc tgc gga tac atc gcc aaa ctc ctg ctc 336
Ala Met Tyr Val Gly Asp Leu Cys Gly Tyr Ile Ala Lys Leu Leu Leu
            100                 105                 110

gcc aca ctc ggt ctg ctc atg gtg ctc cag gct gcc ata gct aga gtg 384
Ala Thr Leu Gly Leu Leu Met Val Leu Gln Ala Ala Ile Ala Arg Val
        115                 120                 125

ccg tac ttc gta cgc act cag ggg ctc att cgt gtg tgt atg tta gtg 432
Pro Tyr Phe Val Arg Thr Gln Gly Leu Ile Arg Val Cys Met Leu Val
    130                 135                 140

cgg aaa gtc gcc ggg ggt cac tat gcc cag atg gcc ttc atc aag ctg 480
Arg Lys Val Ala Gly Gly His Tyr Ala Gln Met Ala Phe Ile Lys Leu
145                 150                 155                 160

gcc gca ctg a 490
Ala Ala Leu

<210> 46
<211> 163
<212> PRT
<213> Hepatitis C virus

<400> 46

Leu Leu Ala Leu Leu Ser Cys Leu Thr Ile Pro Ala Ser Ala Tyr Glu
1               5                   10                  15

Val Arg Asn Val Ser Gly Ile Tyr His Val Thr Asn Asp Cys Ser Asn
            20                  25                  30

Ser Ser Ile Val Tyr Glu Ala Ala Asp Val Ile Met His Thr Pro Gly
        35                  40                  45

Cys Val Pro Cys Val Arg Glu Gly Asn Ala Ser Arg Cys Trp Ala Ala
    50                  55                  60

Leu Thr Pro Thr Leu Ala Val Gly Asn Ala Ser Val Pro Thr Lys Ala
65                  70                  75                  80

Ile Arg Arg His Val Asp Leu Leu Val Gly Thr Ala Ala Phe Cys Ser
                85                  90                  95

Ala Met Tyr Val Gly Asp Leu Cys Gly Tyr Ile Ala Lys Leu Leu Leu
            100                 105                 110

Ala Thr Leu Gly Leu Leu Met Val Leu Gln Ala Ala Ile Ala Arg Val
        115                 120                 125

Pro Tyr Phe Val Arg Thr Gln Gly Leu Ile Arg Val Cys Met Leu Val
    130                 135                 140

Arg Lys Val Ala Gly Gly His Tyr Ala Gln Met Ala Phe Ile Lys Leu
145                 150                 155                 160

Ala Ala Leu

<210> 47
<211> 1018
<212> DNA
<213> Hepatitis C virus

<220>
<221> CDS
<222> (229)..(1017)
<223>

<400> 47
ccaggacccc ccctcccggg agagccatag tggtctgcgg aaccggtgag tacaccggaa      60

ttgccaggac gaccgggtcc tttcttggat taacccgctc aatgcctgga gatttgggcg     120

tgcccccgcg agactgctag ccgagtagtg ttgggtcgcg aaaggccttg tggtactgcc     180

tgatagggtg cttgcgagtg ccccgggagg tctcgtagac cgtgcacc atg agc acg     237
                                                     Met Ser Thr
                                                     1

aat cct aaa ccc caa aga aaa acc aac cga aac acc aac cgc cgt cca 285
Asn Pro Lys Pro Gln Arg Lys Thr Asn Arg Asn Thr Asn Arg Arg Pro
    5                   10                  15

cag gac gtt aag ttc ccg ggc ggt ggt cag atc gtc ggt gga gtt tac 333
Gln Asp Val Lys Phe Pro Gly Gly Gly Gln Ile Val Gly Gly Val Tyr
20                  25                  30                  35

ctg ttg ccg cgc agg ggc ccc agg ttg ggt gtg cgc gcg act agg cag 381
Leu Leu Pro Arg Arg Gly Pro Arg Leu Gly Val Arg Ala Thr Arg Gln
                40                  45                  50

act tcc gag cgg tcg cag cct cgt gga agg cga caa cct atc ccc aag 429
Thr Ser Glu Arg Ser Gln Pro Arg Gly Arg Arg Gln Pro Ile Pro Lys
            55                  60                  65

gtt cgc cgg ccc gag ggc aga acc tgg gct cag ccc ggg tat cct tgg 477
Val Arg Arg Pro Glu Gly Arg Thr Trp Ala Gln Pro Gly Tyr Pro Trp
        70                  75                  80

ccc ctc tat ggc aat gag ggc ttg ggg tgg gca gga tgg ctc ctg tca 525
Pro Leu Tyr Gly Asn Glu Gly Leu Gly Trp Ala Gly Trp Leu Leu Ser
    85                  90                  95

ccc cgt ggc tcc cgg cct agt tgg ggc ccc acg gac ccc cgg cgt agg 573
Pro Arg Gly Ser Arg Pro Ser Trp Gly Pro Thr Asp Pro Arg Arg Arg
100                 105                 110                 115

tcg cgt aat ttg ggt aag gtc atc gat acc ctc aca tgc ggc ttc gcc 621
Ser Arg Asn Leu Gly Lys Val Ile Asp Thr Leu Thr Cys Gly Phe Ala
                120                 125                 130

gac ctc atg ggg tat att ccg ctt gtc ggc gcc cct tta gga ggc gct 669
Asp Leu Met Gly Tyr Ile Pro Leu Val Gly Ala Pro Leu Gly Gly Ala
            135                 140                 145

gcc agg gcc ctg gca cat ggt gtc cgg gtt ctg gag gac ggc gtg aat     717
Ala Arg Ala Leu Ala His Gly Val Arg Val Leu Glu Asp Gly Val Asn
        150                 155                 160

tct gca aca ggg aat ttg cct ggt tgc tct ttc tct atc ttc ctc ttg     765
Ser Ala Thr Gly Asn Leu Pro Gly Cys Ser Phe Ser Ile Phe Leu Leu
    165                 170                 175

gct ctg ctg tcc tgt ttg acc atc cca gct tcc gct tat gaa gtg cgc     813
Ala Leu Leu Ser Cys Leu Thr Ile Pro Ala Ser Ala Tyr Glu Val Arg
180                 185                 190                 195

acg tgc gcg gtc cat cca gag cca atc ttt gac atc acc aac ctc ctg     861
Thr Cys Ala Val His Pro Glu Pro Ile Phe Asp Ile Thr Asn Leu Leu
                200                 205                 210

ctc gcc ata ctc ggc ccg ctc atg gtg ctc cag gct ggc ata act aga     909
Leu Ala Ile Leu Gly Pro Leu Met Val Leu Gln Ala Gly Ile Thr Arg
            215                 220                 225

gtg ccg tac ttc gta cgc gct caa ggg ctc att cgt gca tgc atg tta     957
Val Pro Tyr Phe Val Arg Ala Gln Gly Leu Ile Arg Ala Cys Met Leu
        230                 235                 240

gtg cgg aaa acg cct ggg ggt cat tat gtc caa atg gcc ctc atg agg     1005
Val Arg Lys Thr Pro Gly Gly His Tyr Val Gln Met Ala Leu Met Arg
    245                 250                 255

ctg gcc gca ctg a                                                   1018
Leu Ala Ala Leu
260

<210> 48
<211> 263

<212>  PRT
<213>  Hepatitis C virus

<400>  48

Met Ser Thr Asn Pro Lys Pro Gln Arg Lys Thr Asn Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Gln Thr Ser Glu Arg Ser Gln Pro Arg Gly Arg Arg Gln Pro
    50                  55                  60

Ile Pro Lys Val Arg Arg Pro Glu Gly Arg Thr Trp Ala Gln Pro Gly
65                  70                  75                  80

Tyr Pro Trp Pro Leu Tyr Gly Asn Glu Gly Leu Gly Trp Ala Gly Trp
                85                  90                  95

Leu Leu Ser Pro Arg Gly Ser Arg Pro Ser Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Ala Ala Arg Ala Leu Ala His Gly Val Arg Val Leu Glu Asp
145                 150                 155                 160

Gly Val Asn Ser Ala Thr Gly Asn Leu Pro Gly Cys Ser Phe Ser Ile
                165                 170                 175

Phe Leu Leu Ala Leu Leu Ser Cys Leu Thr Ile Pro Ala Ser Ala Tyr
            180                 185                 190

Glu Val Arg Thr Cys Ala Val His Pro Glu Pro Ile Phe Asp Ile Thr
        195                 200                 205

Asn Leu Leu Leu Ala Ile Leu Gly Pro Leu Met Val Leu Gln Ala Gly
    210                 215                 220

Ile Thr Arg Val Pro Tyr Phe Val Arg Ala Gln Gly Leu Ile Arg Ala
225                 230                 235                 240

Cys Met Leu Val Arg Lys Thr Pro Gly Gly His Tyr Val Gln Met Ala
                245                 250                 255

Leu Met Arg Leu Ala Ala Leu
            260

<210> 49
<211> 32
<212> DNA
<213> synthetic

<400> 49
cgcggatcct tagtcctcca gaacccggac ac 32

<210> 50
<211> 19
<212> DNA
<213> synthetic

<400> 50
tgcacggtct acgagacct 19

<210> 51
<211> 20
<212> DNA
<213> synthetic

<400> 51
tagtggtctg cggaaccggt 20

<210> 52
<211> 25
<212> DNA
<213> synthetic

<400> 52
gccgcatgta agggtatcga tgacc 25

<210> 53
<211> 25
<212> DNA
<213> synthetic

<400> 53
ggtcatcgat acccttacat gcggc 25

<210> 54
<211> 32
<212> DNA
<213> synthetic

<400> 54
gcgaattctt atcagaagaa ctcgtcaaga ag 32

<210> 55
<211> 26
<212> DNA
<213> synthetic

<400> 55
gcctattggc ctggagtgtt tagctc 26

<210> 56
<211> 29
<212> DNA
<213> synthetic

<400> 56
atggcgttag tatgagtgtc gtgcagcct 29

<210> 57
<211> 25
<212> DNA
<213> synthetic

<400> 57
agccgcatgt aagggtatcg atgac 25

<210> 58
<211> 23
<212> DNA
<213> synthetic

<400> 58
tggttcggct gyacatggat gaa 23

<210> 59
<211> 26
<212> DNA
<213> synthetic

<400> 59
ggrtagtgcc aragcctgta tgggta 26

<210> 60
<211> 31
<212> DNA
<213> synthetic

<400> 60
tcgggcacga gacaggctgt gatatatgtc t 31

<210> 61
<211> 30
<212> DNA
<213> synthetic

<400> 61
atcgtcttca cgcagaaagc gtctagccat 30

<210> 62
<211> 31
<212> DNA
<213> synthetic

<400> 62
gccagccccc tgatgggggc gacactccac c 31

<210> 63
<211> 30
<212> DNA
<213> synthetic

<400> 63
aatcatatgt ctttgaggtt taggatttgt 30

<210> 64
<211> 29
<212> DNA
<213> synthetic

<400> 64
gacatatgat tgaacaagat ggattgcac 29

<210> 65
<211> 33
<212> DNA
<213> synthetic

<400> 65
gtcctgcagg ccagccccct gatgggggcg aca 33

<210> 66
<211> 34
<212> DNA
<213> synthetic

<400> 66
gacctgcagg ttatcagaag aactcgtcaa gaag 34

<210> 67
<211> 50
<212> DNA
<213> synthetic

<400> 67
gccttaatta atacgactca ctataggcca gccccctgat gggggcgaca 50

<210> 68
<211> 76
<212> DNA
<213> Synthetic

<400> 68
tctagtcgac ggccagtgaa ttgtaatacg actcactata gggcggccag ccccctgatg 60

ggggcgacac tccacc 76

<210> 69
<211> 28
<212> DNA
<213> synthetic DNA

<400> 69
aggcctgtga agacgctctc ccagaact 28

<210> 70
<211> 21
<212> DNA
<213> synthetic DNA

<400> 70
ggtgatgacc ttggtctcca t 21

<210> 71
<211> 32
<212> DNA
<213> synthetic DNA

<400> 71
gcttagaggc tagtgatgat gcaaccaagt ac 32

<210> 72
<211> 24
<212> DNA
<213> synthetic DNA

<400> 72

ggcgaccgca tagtagtttc cata 24

<210> 73
<211> 32
<212> DNA
<213> synthetic DNA

<400> 73
ctggaggacg gcgtgaacta tgcaacaggg aa 32

<210> 74
<211> 32
<212> DNA
<213> synthetic DNA

<400> 74
ggaacttgcc cggttgctct ttctctatct tc 32

<210> 75
<211> 21
<212> DNA
<213> synthetic DNA

<400> 75
tggggcaaga tggttataaa c 21

<210> 76
<211> 31
<212> DNA
<213> synthetic DNA

<400> 76

ggggtaagat ggttataaac gtacgtacct g 31

<210> 77
<211> 31
<212> DNA
<213> synthetic DNA

<400> 77
ataatgaccc ccggcgactt tccgcactaa c 31

<210> 78
<211> 24
<212> DNA
<213> synthetic DNA

<400> 78
tgacatcagc atgtctcgtg acca 24

<210> 79
<211> 26
<212> DNA
<213> synthetic DNA

<400> 79
cttgaaaaag ccctggattg tcagat 26

<210> 80
<211> 20
<212> DNA
<213> synthetic DNA

<400> 80

acatgatctg cagagaggcc 20

<210> 81
<211> 26
<212> DNA
<213> synthetic DNA

<400> 81
ctacggggcc tgttactcca ttgaac 26

<210> 82
<211> 35
<212> DNA
<213> synthetic DNA

<400> 82
acatgatctg cagagaggcc agtatcagca ctctc 35

<210> 83
<211> 73
<212> DNA
<213> synthetic
<400> 83
tctagtcgac ggccagtgaa ttgtaatacg actcactcta gggcggcggg gtcgggcwcg
ngacabgctg tga 73

<210> 84
<211> 31
<212> DNA
<213> synthetic DNA

<400> 84

tctccattgg gctgaacacc acaggctcca c 31

<210> 85
<211> 31
<212> DNA
<213> synthetic DNA

<400> 85
ggggagaggt ggtcatagat gtaagtgccg g 31

<210> 86
<211> 30
<212> DNA
<213> synthetic DNA

<400> 86
catagatgta agtgccggtc cacctgccta 30

<210> 87
<211> 30
<212> DNA
<213> synthetic DNA

<400> 87
ctcctgcgag gtgtctcacc agggtacaca 30

<210> 88
<211> 29
<212> DNA
<213> synthetic DNA

<400> 88

agcagagcgt gagctctgac gaagtatgg 29

<210> 89
<211> 32
<212> DNA
<213> synthetic DNA

<400> 89
ggaatctacc cggttgctct ttttctatct tc 32

<210> 90
<211> 32
<212> DNA
<213> synthetic DNA

<400> 90
ctggaagacg ggataaatta tgcaacaggg aa 32

<210> 91
<211> 24
<212> DNA
<213> synthetic DNA

<400> 91
ctcgcaagca ccctatcagc cagt 24

<210> 92
<211> 20
<212> DNA
<213> synthetic DNA

<400> 92
aggcattgag cgggtttatc 20

<210> 93
<211> 38
<212> DNA
<213> synthetic DNA

<400> 93
ctagactcga gtcgacatcg tttttttttt tttttttt 38

<210> 94
<211> 20
<212> DNA
<213> synthetic DNA

<400> 94
atcttagccc tagtcacggc 20

<210> 95
<211> 20
<212> DNA
<213> synthetic DNA

<400> 95
ctagactcga gtcgacatcg 20

<210> 96
<211> 30
<212> DNA
<213> synthetic DNA

<400> 96
ctagctgtga aaggtccgtg agccgcatga 30